# EUROPEAN PATENT APPLICATION

(11) **EP 2 287 340 A2**
(43) Date of publication of application: **23.02.2011**
(21) Application number: 10075441.5
(22) Date of filing: 11.05.2006
(51) Int. Cl.: C12Q 1/68

(54) **Method for diagnosis and treatment of a mental disease**

(30) Priority: 11.05.2005 DK 200500680
(62) Divisional of application: 06742417.6
(71) Applicant: Aarhus Universitet, 8000 Århus C (DK); Region Midtjylland, 8800 Viborg (DK)
(72) Inventor: Ewald, Henrik, Deceased (DK); Børglum, Anders, 6270 Hoejbjerg (DK); Severinsen, Jacob, 8210 Århus C (DK); Mors, Ole, 8240 Risskov (DK); Muir, Walter, Peebles EH45 8NA (GB); Blackwood, Douglas, Edinburgh EH10 4RY (GB)
(74) Representative: Didmon, Mark

(57) **Abstract**

The present invention relates to association of one or more polymorphisms in the human NHP2L1, PACSIN2, SERHL, PIPPIN, BRD1, EP300, FAM19A5 and/or GPR24 genes to the occurrence of schizophrenia and/or bipolar disorder. The invention relates both to methods for diagnosing a predisposition to said diseases and for treating subjects having said diseases.

## Description

### Field of invention

The present invention relates to association of one or more polymorphisms located in the human NHP2L1, PACSIN2, SERHL, PIPPIN, BRD1, EP300, FAM19A5 and/or GPR24 genes to the occurrence of schizophrenia and/or bipolar disorder. The invention relates both to methods for diagnosing a predisposition to said diseases and for treating subjects having said diseases.

### Background of invention

### Polymorphisms

DNA polymorphisms provide an efficient way to study the association of genes and diseases by analysis of linkage and linkage disequilibrium. With the sequencing of the human genome a myriad of hitherto unknown genetic polymorphisms have been detected. Most common among these are the single nucleotide polymorphisms, also called SNPs, of which are counted now to several millions. Other examples of genetic polymorphisms are variable number of tandem repeat polymorphisms, insertions, deletions and block modifications. Tandem repeats often have multiple different alleles (variants), whereas the other groups of polymorphisms usually just have two alleles. Some of these genetic polymorphisms probably play a direct role in the biology of the individuals, including the risk of developing disease, but the virtue of the majority is that they can serve as markers for the surrounding DNA.

The association of an allele of one sequence polymorphism with particular alleles of other sequence polymorphisms in the surrounding DNA has two origins, known in the genetic field as linkage and linkage disequilibrium, respectively. Linkage arises because large parts of chromosomes are passed unchanged from parents to offspring, so that minor regions of a chromosome tend to flow unchanged from one generation to the next and also to be similar in different branches of the same family. Linkage is gradually eroded by recombination occurring in the cells of the germline, but typically operates over multiple generations and distances of a number of million bases in the DNA.

Linkage disequilibrium deals with whole populations and has its origin in the (distant) forefather in whose DNA a new sequence polymorphism arose. The immediate surroundings in the DNA of the forefather will tend to stay with the new allele for many generations. Recombination and changes in the composition of the population will again erode the association, but the new allele and the alleles of any other polymorphism nearby will often be partly associated among unrelated humans even today. A crude estimate suggests that alleles of sequence polymorphisms with distances less then 10000 bases in the DNA will have tended to stay together since modern man arose. Linkage disequilibrium in limited populations, for instance Europeans, often extends over longer distances, e.g. over more than 1,000,000 bases. This can be the result of newer mutations, but can also be a consequence of one or more "bottlenecks" with small population sizes and considerable inbreeding in the history of the current population. Two obvious possibilities for "bottlenecks" in Europeans are the exodus from Africa and the repopulation of Europe after the last ice age.

### Schizophrenia and bipolar disorder

The importance of genetics in the etiology of bipolar disorder (BPD) and schizophrenia (SCH) are by now an accepted fact and confirmed through family, twin and adoption studies (McGuffin et al., 1995; Potash and DePaulo, 2000). Overlapping between these diseases has long been noted clinically and a shared genetic heritability is probable according to genetic epidemiology studies and linkage studies (Berrettini, 2000; Potash et al., 2003a) Linkage studies have identified several shared susceptibility loci (on 18p11; 13q31; 10p14; and 22q11-13) which makes it plausible that there are partial shared genetic risks, and susceptibility genes. On chromosome 22q12-13 implication in schizophrenia has been suggested by weak linkage (Gill et al., 1996; Pulver et al., 1994) and a resent meta-analysis including 20 genome scans in schizophrenics has confirmed the locus 22q12-13 as a susceptibility locus. In addition significant linkage to BPD on 22q12-13 was evidenced by Kelso et al (Kelsoe et al., 2001). In a meta-analysis including 11 BPD and 18 schizophrenic genome scans 22q12-13 were one of two regions that showed strong evidence for harboring a susceptibility loci for both BPD and SCH (Badner and Gershon, 2002; Potash et al., 2003b). However, there were obtained no evidence that polymorphism of DNA in this locus is associated with the diseases.

### Genes

Several genes with known or unknown biological function have been mapped to chromosome 22q13.

### 1. BRD1

The gene was originally described in connection with study of the genes involved in oncogenic transformation in human acute leukemias and mapped to 22q13 by fluorescence in situ hybridization (McCullagh et al. (1999)).

In human acute leukemias the MLL gene is reciprocally translocated with one of a number of different partner genes. The precise mechanism of oncogenic transformation is unclear since most of the partner genes encode unrelated proteins. However, 2 partner genes, AF10 and AF17, are related through the presence of a cysteine-rich region and a leucine zipper. McCullagh et al. (1999) suggested that the identification of other proteins having these structures might aid understanding of their role in normal and leukemic cells. They reported the cloning of a novel human gene that encodes a 1,058-amino acid protein containing a cysteine-rich region related to that of AF10 and AF17. Overall, the protein was most closely related to the BR140 protein, and was therefore designated BRL (BR140-like gene). Northern blot analysis revealed that a 4.6-kb BRL transcript is expressed at high levels in testis and in several cell lines. A monoclonal antibody raised to a BRL peptide sequence confirmed its widespread expression as a 120-kD protein and demonstrated localization to the nucleus of spermatocytes.

The gene encodes a protein of unknown function. The protein contains a bromodomain, a sequence motif often found in transcriptional co-activators, and localizes to the nucleus in testis and several other cell types.

### 2. NHP2L1

In the course of sequencing cDNA clones from fetal brain cDNA libraries, Saito et al. (1996) isolated a gene that encodes a protein highly homologous to NHP2 from Saccharomyces cerevisiae (Kolodrubetz and Burgum, 1991). NHP2 is a high-mobility group (HMG)-like protein which is located in the nucleus, although it shows weak homology to some ribosomal proteins. The cDNA cloned by Saito et al. (1996), symbolized NHP2L1, was expressed in all human tissues examined and was localized to 12q24.3 by fluorescence in situ hybridization.

Originally named because of its sequence similarity to the Saccharomyces cerevisiae NHP2 (non-histone protein 2), this protein appears to be a highly conserved nuclear protein that is a component of the [U4/U6.U5] tri-snRNP. It binds to the 5' stem-loop of U4 snRNA. It is suggested that the protein may play a role in the late stage of spliceosome assembly. The protein is related to the L7AE family of ribosomal proteins. The protein has ubiquitous tissue expression. Two transcript variants encoding the same protein have been found for human NHP2L1 gene.

### 3. PACSIN2

Human PACSIN2 gene was mapped to 22q13 (Sumoy et al. (2001)).

PACSIN family members, such as mouse Pacsin1 and chicken FAP52, are cytoplasmic adapter proteins with a common arrangement of domains and conserved regions, including a CDC15 N-terminal domain, which contains a RAEYL motif and a coiled-coil region, and a C-terminal SH3 domain. By searching an EST database for novel members of the PACSIN family, Ritter et al. (1999) identified ESTs encoding human PACSIN2 and mouse Pacsin2. The complete human PACSIN2 coding sequence, obtained from 2 overlapping retina ESTs, encodes a deduced 486-amino acid protein that shares 93.6% sequence identity with mouse Pacsin2. The PACSIN2 proteins contain a CDC15 N-terminal domain, a C-terminal SH3 domain, 3 conserved regions specific to the PACSIN family, and 3 asn-pro-phe (NPF) motifs, which potentially bind to EH domains. The PACSIN2 proteins share high sequence similarity with chicken FAP52 and mouse Pacsin1. However, compared to these proteins, PACSIN2 proteins have a 41-amino acid insertion, which contains 1 NPF motif. In contrast to the restricted neural expression of Pacsin1 protein (Plomann et al., 1998), Northern blot analysis detected Pacsin2 transcript expression in all tissues examined, with the highest levels in brain, heart, skeletal muscle, and ovary. Immunofluorescence microscopy revealed a broad, vesicle-like cytoplasmic distribution of recombinant Pacsin2 expressed in fibroblasts that appeared to partially overlap with the distributions of both the actin filament and microtubule networks. Ritter et al. (1999) suggested that PACSIN2 protein may participate in the organization of the actin cytoskeleton and the regulation of vesicular traffic.

Human PACSIN2 can homo- and hetero-aggregate with other PACSINs, bind dynamin 1, synaptojanin, synapsin 1 and the neural Wiskott-Aldrich syndrome protein (N-WASP), is phosphorylated by casein kinase 2 (CK2) and protein kinase C (PKC). Vesicle-like cytoplasmic distribution of the protein suggests its possible role in vesicle formation and transport.

### 4. SERHL

Sadusky et al. (2001) cloned Serhl by subtractive hybridization of transcripts upregulated in passively stretched mouse skeletal muscle. The deduced 311-amino acid protein contains a putative serine hydrolase active center, prenylation and N-myristoylation sites, several putative phosphorylation sites, an N-glycosylation site, and a C-terminal peroxisomal targeting sequence. Northern blot analysis detected transcripts of 1.4 and 2.4 kb expressed in all mouse tissues examined. Highest levels of both transcripts were detected in kidney, and lowest levels were detected in skeletal muscle and small intestine. Brain predominantly expressed the 2.4-kb form. Western blot analysis detected a Serhl protein with an apparent molecular mass of about 35 kD in mouse skeletal and cardiac muscle, brain, and kidney. Immunolocalization detected Serhl expressed in small perinuclear vesicles in mononucleated mouse myoblasts and recently fused multinucleated myotubes. By genomic sequence analysis Sadusky et al. (2001) mapped the SERHL gene to 22q 13.2.

### 5. PIPPIN

The gene encodes an RNA-binding protein, which is highly enriched in the brain, contains two putative double stranded RNA-binding domains, a cold-shock domain, binds with high specificity to the transcripts that encode H1 and H3.3. histone variants (Castiglia et al. 1996, Nastasi et al., 1999; Nastasi et al, 2000). It has been shown that PIPPIN inhibits translation of H1(0) and H3.3 mRNA in cell-free system. Based on this finding it has been suggested that PIPPIN down-regulates histone variant expression in the developing ret brain. The PIPPIN gene is mapped to 22q13.2

### 6.EP300

Eckner et al. (1994) mapped the p300 gene, symbolized EP300, to 22q13.2.

EP300 encodes the adenovirus E1A-associated cellular p300 transcriptional co-activator protein. The growth-controlling functions of the adenovirus E1A oncoprotein depend on its ability to interact with a set of cellular proteins. Among these are the retinoblastoma protein, p107, p130, and p300. Eckner et al. (1994) isolated a cDNA encoding full-length human p300. p300 contains 3 cysteine- and histidine-rich regions of which the most carboxy-terminal region interacts specifically with E1A. In its center, p300 contains a bromodomain, a hallmark of certain transcriptional coactivators. p300 and CREB-binding protein (CREBBP, or CBP) are highly related in primary structure (Arany et al., 1994). Several protein motifs such as a bromodomain, a KIX domain, and 3 regions rich in cys/his residues are well conserved between these 2 proteins. Lin et al. (2001) identified a compactly folded 46-residue domain in CBP and p300, the IRF3-binding domain (IBID), and determined its structure by nuclear magnetic resonance spectroscopy. IBID has a helical framework containing an apparently flexible polyglutamine loop that participates in ligand binding. Spectroscopic data indicated that induced folding accompanies association of IBID with its partners, which exhibit no evident sequence similarities. IBID is an important contributor to signal integration by CBP and p300.

EP300 (p300) is a multifunctional protein:
- like CPB it can stimulate transcription through activation of CREB. This EP300 activity is specifically inhibited by the adenovirus oncoprotein E1A;
- EP300 has also been identified as a co-activator of HIF1A (hypoxia-inducible factor 1 alpha), and thus plays a role in the stimulation of hypoxia-induced genes such as VEGF;
- EP300/CREBBP and IRF3 are components of DRAF1 (double-stranded RNA-activated factor-1), a positive regulator of interferon-stimulated gene transcription that functions as a direct response to viral infection (Weaver et al. 1998);
- the formation of a complex between transcription factors STAT3 and SMAD1, bridged by p300, is involved in the cooperative signaling of cytokines LIF and BMP2 and the subsequent induction of astrocytes from neuronal progenitors (Nakashima et al. 1999);
- it play a role in DNA repair synthesis and other DNA metabolic events through its interaction with proliferating cell nuclear antigen (PCNA) and with flap endonuclease-1 (FEN1) (Hasan et al. 2001);
- it has a regulatory role for protein acetylation in base mismatch repair and maintaining genomic stability (Tini et al. 2002);
- has a role in the mechanism for circadian phase control (Etchegaray et al. 2003);
- generation of the polyubiquitinated forms of p53 that are targeted for proteasome degradation requires the intrinsic ubiquitin ligase activities of MDM2 and p300 (Grossman et al. 2003);

The EP300 protein is a histone acetyltransferase that regulates transcription via chromatin remodeling and is important in the processes of cell proliferation and differentiation. A role for EP300 in cancer had been implied by the fact that it is targeted by viral oncoproteins (Arany et al., 1995), it is fused to MLL in leukemia (Ida et al., 1997), and 2 missense sequence alterations in EP300 were identified in epithelial malignancies (Muraoka et al., 1996). Gayther et al. (2000) described EP300 mutations that predicted a truncated protein in 6 (3%) of 193 epithelial cancers analyzed. Of these 6 mutations, 2 were in primary tumors (a colorectal cancer and a breast cancer) and 4 were in cancer cell lines (colorectal, breast, and pancreatic). In addition, they identified a somatic in-frame insertion in a primary breast cancer and missense alterations in a primary colorectal cancer and 2 cell lines (breast and pancreatic). Inactivation of the second allele was demonstrated in 5 of the 6 cases with truncating mutations and in 2 other cases. The data showed that EP300 is mutated in epithelial cancers and provided the first evidence that it behaves as a classic tumor suppressor gene.

### 7. GPR24

The protein encoded by GPR24 gene is a member of the G protein-coupled receptor family 1, an integral plasma membrane protein which binds melanin-concentrating hormone. The encoded protein can inhibit cAMP accumulation and stimulate intracellular calcium flux, and is probably involved in the neuronal regulation of food consumption. Although structurally similar to somatostatin receptors, the protein does not seem to bind somatostatin. The protein encoded by the GPR24 gene is also termed in scientific literature as SLC1, MCH1R, MCHR1.

The somatostatin receptors (SSTRs) are a family of G protein-coupled receptors which bind to somatostatin peptides. Kolakowski et al. (1996) identified an EST sequence with significant homology to the SSTRs that does not bind somatostatin. They cloned the gene corresponding to this EST from a human genomic library. Sequencing of a genomic clone, which they termed SLC1, revealed a full-length and intronless open reading frame encoding a 402-amino acid protein. Its transmembrane regions are approximately 40% identical to other members of the SSTR family, including several residues considered to form the ligand-binding pocket of SSTRs. Northern blot analysis detected a single 2.4-kb transcript with greatest abundance in the human brain, particularly in the frontal cortex and hypothalamus. These regions are associated with emotion, memory, and sensory perception. Kolakowski et al. (1996) expressed the SLC1 receptor in COS-7 cells and found that it does not bind to somatostatin peptides. They identified a polymorphic CA repeat in the 5-prime untranslated region of this gene, and they used fluorescence in situ hybridization to map the gene to 22q13.3.

Chambers et al. (1999) used a reverse pharmacology approach to identify the natural cognate ligand for SLC1. They expressed the receptor in HEK293 cells and screened against a large library of known bioactive substances, including over 500 naturally occurring or putative neuropeptides. In this screen, melanin-concentrating hormone (MCH) was the only substance to produce a robust, dose-dependent (EC-50 = 3.72 nM), transient elevation of intracellular calcium in HEK293 cells transiently transfected with SLC1.

MCH can act as a functional antagonist of alpha-melanocyte-stimulating hormone (alpha-MSH) in a diverse range of animal species and physiologic roles. In mammals, MCH is orexigenic and alpha-MSH is anorexigenic. Chambers et al. (1999) tested alpha-MSH at up to 10-mM concentration and no agonistic or antagonistic interaction with SLC1 was observed. Together with the observation that MCH could not displace alpha-MSH from melanocortin receptors, these results supported the idea that the functional, mutually antagonistic effects of alpha-MSH and MCH are mediated by their interaction at separate receptors. Using in situ hybridization, Chambers et al. (1999) demonstrated that SLC1 is widely and strongly expressed in the rat brain. There were clear mRNA signals in the olfactory tubercle, cerebral cortex, substantia nigra, basal forebrain, CA1, CA2, and CA3 fields of the hippocampus, amygdala, and various other nuclei in the hypothalamus, thalamus, midbrain, and hindbrain. There were also strong signals in the ventromedial and dorsomedial nuclei of the hypothalamus, areas widely recognized as being involved in feeding behavior.

Saito et al. (1999) identified SLC1 as an MCH hormone receptor using a different technique and found similar EC-50 and brain distribution for MCHR1 (SLC1).

Borowsky et al. (2002) showed that the selective high-affinity MCH1R (MCHR1) receptor antagonist SNAP-7941 inhibited food intake stimulated by central administration of MHC, reduced consumption of palatable food, and after chronic administration to rats with diet-induced obesity, resulted in a marked, sustained decrease in body weight. Borowsky et al. (2002) also showed that SNAP-7941 produced effects similar to clinically used antidepressants and anxiolytics in 3 animal models of depression/anxiety: the rat forced-swim test, rat social interaction, and guinea pig maternal-separation vocalization tests. Given these observations, the authors concluded that an MCH1R antagonist may be useful not only in the management of obesity but also as a treatment for depression and/or anxiety.

### Genetic markers of mental diseases

A number of studies have been performed in the attempt to identify the genes responsible for development of mental diseases such as SCH and BPD. Among these quite a few studies focused on the finding a correlation of the polymorphism of genomic sequences with development and inheritance of mental disorders such as SCH and BPD, which would be useful in disease diagnosis and prognosis (Skibinska et al. 2004;; Xi et al. 2004; Sinibaldi et al. 2004; Li et al,.2004). However, there were so far identified no reliable genetic markers in association with the diseases so far.

### Summary of invention

The authors of the present invention for the first time herein
1) described a strong correlation between polymorphism(s) of selected genes mapped to chromosome 22q13 such as the NHP2L1, PACSIN2, SERHL, PIPPIN, BRD1, EP300, FAM19A5 and/or GPR24 genes, and a predisposition to SCH and/or BPD, proposed to use said polymorphism(s) for diagnosis/prognosis of a predisposition of an individual to SCH and/or BPD, and suggested said polymorphisms, said genes, their gene products and related pathways/interacting genes and gene products as targets for medical treatment of SCH and/or BDP and in the search for new drug candidates for medical treatment of SCH and/or BPD;
2) described the association of specific haplotypes of the identified polymorphisms with a predisposition to SCH and/or BPD and proposed to use haplotype analysis as a mean in diagnosis/prognosis of a predisposition to SCH and/or BPD and the SNPs of said haplotypes as targets for medical treatment of SCH and/or BPD and in the search for new drug candidates for medical treatment of SCH and/or BPD;
3) described single nucleotide polymorphisms (SNPs) mapped to the genes of chromosome 22q13 which are in linkage disequilibrium with the polymorphisms of the BRD1, NHP2L1, PACSIN2, SERHL, PIPPIN, EP300, FAM19A5 and GPR24 genes associated with SCH and/or BPD, and proposed to use the latter SNPs as diagnostic/prognostic markers of a predisposition to SCH and/or BPD and targets for medical treatment SCH and/or BPD and in the search for new drug candidates for medical treatment of SCH and/or BPD;
4) described new etiologic factors, such as transcription and translation products of the above genes containing polymorphism(s), associated with SCH and/or BDP, and provided methods for diagnosis, prognosis and treatment of SCH and/or BPD comprising a step of identification such factor in a sample from a patient and/or a step of modulating biological activity of such factor a compound described herein;
5) provided a method of determining a predisposition/no predisposition to/protection against SCH and/or BPD and a method for diagnosis of said diseases, said methods comprising determining a polymorphism of the BRD1, NHP2L1, PACSIN2, SERHL, PIPPIN, EP300, FAM19A5 and GPR24 genes and/or a polymorphism of another gene of chromosome 22q13, said polymorphisms being in linkage disequilibrium;
6) provided a method for gene therapy treatment of SCH and/or BPD, said method comprising providing a gene therapy vector comprising a DNA sequence of the invention, e.g. a DNA sequence including the protective allele of a polymorphism being associated with SCH and/or BPD;
7) provided a method for identification of new drug candidate compounds for the treatment of SCH and/or BPD, said method comprising screening compounds for a capability of inhibiting/modulating biological activity of the BRD1, NHP2L1, PACSIN2, SERHL, PIPPIN, EP300, FAM19A5 and GPR24 genes and/or products of said genes, said activity being associated with manifestation/predisposition of/to SCH and/or BDP;
8) provided diagnostic/prognostic kits for diagnosis/prognosis of SCH and/or BPD;
9) provided a method for estimating the likelihood of developing SCH and/or BPD in an individual comprising a step of determining the allele of an SNP of the BRD1, NHP2L1, PACSIN2, SERHL, PIPPIN, EP300, FAM19A5 and GPR24 genes, said SNP being associated with a predisposition of an individual to SCH and/or BPD.

Accordingly, in the first aspect the invention relates to a method for determining a predisposition/no predisposition to a mental disease in a subject comprising determining in a biological sample isolated from said subject one or more polymorphisms in the chromosome regions containing the NHP2L1, PACSIN2, SERHL, PIPPIN, BRD1, EP300, FAM19A5 and/or GPR24 genes, or in a translational or transcriptional product from said regions, said polymorphism being indicative of said predisposition/no predisposition.

The present inventors have discovered that polymorphisms, such as SNPs, identified in the coding and/or non-coding regions of the NHP2L1, PACSIN2, SERHL, PIPPIN, BRD1, EP300, FAM19A5 and/or GPR24 genes are strongly associated with the presence or absence of some mental diseases such as SCH and BPD. Thus, detecting the presence or absence of one or more alleles of the SNPs of the present invention amounts to determining a predisposition to having or not having a mental disease. It thus follows that determining the presence of a specific allele amounts to determining a predisposition to having/not having a mental disease. According to the invention the strength of the association between the presence/absence of a specific SNP in the above genes and the diseases is very strong.

Diagnosis of individuals for genetic predisposition to mental diseases, such as SCH and/or BPD is very important so that they can be given the best treatment and adapt their lifestyle according to their genetic predisposition.

The authors of the present invention performed haplotype analysis of the identified SNPs and found out that the coincidence of some haplotypes in association with a particular disease is higher then the coincidence of another haplotype and the disease. Thus, the invention also relates to specific haplotypes of the identified SNPs. Moreover, it is expected that with the information made available by the inventors, more polymorphisms in the NHP2L1, PACSIN2, SERHL, PIPPIN, BRD1, EP300, FAM19A5 and/or GPR24 genes will be found predisposing to mental diseases, such as SCH and/or BPD. Therefore, all polymorphisms being in linkage disequilibrium with the identified in the present invention SNPs in the chromosome regions adjusted to the genes of the invention are included in the scope of the protection as diagnostic markers of the predisposition to a mental disease, in particular SCH and/or BPD. Moreover, some particular SNPs found in other genes than the genes of the invention and described herein as a part of specific haplotypes associated with a predisposition to SCH and/or BPD are also concerned by the invention as genetic markers of said predisposition.

The inventors have defined the alleles of the described SNPs, which when expressed in an individual have either promotional or no effect on developing a mental disease (the susceptibility or protective allele correspondingly) The application thus also provides a method for estimating the likelihood for development of SCH and/or BPD by analysing the allele variance of the SNPs associated with SCH and/or BPD present in an individual.

In a further aspect the invention relates to isolated oligonucleotide sequences comprising at least 10 contiguous nucleotides being 100% identical to a subsequence of the NHP2L1, PACSIN2, SERHL, PIPPIN, BRD1, EP300, FAM19A5 and/or GPR24 genes comprising or adjacent to a polymorphism of the invention, said polymorphism or mutation being associated to a mental disease.

As the present inventors have determined that the NHP2L1, PACSIN2, SERHL, PIPPIN, BRD1, EP300 and GPR24 genes are etiological factors in mental diseases it is important to be able to detect and correct or suppress any polymorphism in the genes which is correlated to these diseases. The isolated oligonucleotides may be used as probes for detection of the polymorphisms and/or as primer pairs for amplification of a target nucleotide sequence and/or as part of a gene therapy vector for administration to a patient suffering from mental diseases, such as SCH and/or BPD.

In a further aspect the invention relates to a kit for predicting an increased risk of a subject of developing mental diseases, such as SCH and/or BPD or for other diagnostic and classification purposes of SCH and/or BPD comprising at least one probe comprising at least two nucleic acid sequences as defined above.

These kits which may further comprise buffers and primers and reagents can be used for diagnosing the polymorphisms and mutations which correlate to mental diseases of the invention.

The invention also relates to variant proteins comprising variants which correspond to the identified in the application polymorphisms of the corresponding genes. These variant proteins may also be used for diagnosis of the described herein mental diseases.

According to a further aspect the invention relates to antibodies capable of selectively binding to the variant proteins as defined above with a different (such as lower or higher) binding affinity than when binding to the polypeptide having the amino acid sequence of wild type protein.

These antibodies may be used in diagnosing individuals with the polymorphisms. It is also envisaged that such specific antibodies may be used for treating patients carrying the variant protein.

In further aspects the present invention relates to methods of treating patients suffering from mental disorders, in particular SCH and/or BPD. Among the therapeutic methods, one method relates to a method of treating SCH and/or BPD in a subject being diagnosed as having a predisposition according to the invention, comprising administering to said subject a therapeutically effective amount of a gene therapy vector. The invention also relates to a gene therapy vector itself, said vector being capable of altering the polymorphism in cells of a subject being diagnosed as having a predisposition according to the invention, or being capable of correcting, suppressing, supporting or changing the expression of the NHP2L1, PACSIN2, SERHL, PIPPIN, BRD1, EP300, FAM19A5 and/or GPR24 genes in cells of a subject suffering from said diseases.

With the advent of gene therapy it has become possible to suppress and/or to eliminate the effects of a polymorphism by administering to a subject a gene therapy vector which either alters the polymorphism or suppresses the transcription and/or translation from the gene. Such gene therapy vectors have the advantage of being highly specific. Therapeutic methods of treatment of patients suffering from SCH and/or BPD of the invention also include methods of treatment comprising a step of modulating the activity of products of the genes comprising a polymorphism associated with SCH and/or BPD.

### Definitions

### Gene / gene sequence:

A compilation of
   - the genomic sequences which are transcribed into a transcriptional entity
   - the genomic sequences in between
   - the genomic sequences involved in regulation of expression and splicing of the gene comprising at least 2000 bp upstream and downstream from the transcribed entity.
The present invention relates to the genes identified in the NCBI database (http://www.ncbi.nlm.nih.gov) as
GeneID: 4809 (NHP2L1)
GeneID: 11252 (PACSIN2)
GeneID: 94009 (SERHL)
GeneID: 27254 (PIPPIN)
GeneID: 23774 (BRD1)
GeneID: 2033 (EP300)
GeneID: 2847 (GPR24)
GeneID: 25817 (FAM19A5)
Genomic sequences of the above genes (http://genome.ucsc.edu/) are identified in the present invention as

| | |
|---|---|
| NHP2L1 gene | SEQ ID NO: 1 |
| PACSIN2 gene | SEQ ID NO: 2 |
| SERHL gene | SEQ ID NO: 3 |
| PIPPIN gene | SEQ ID NO: 4 |
| BRD1 gene | SEQ ID NO: 5 |
| EP300 gene | SEQ ID NO: 6 |
| GPR24 gene | SEQ ID NO: 7 |
| FAM19A5 | SEQ ID NO: 94 |

The term "chromosome region containing a gene" means herein a part of a human chromosome containing a gene of the invention and nucleotide sequences of 2 to 2000 base pairs adjacent to both ends of the defined gene sequence (SEQ ID NO: 1-7 and 94), wherein one end of the gene corresponds to the first nucleotide of the gene sequence, and another end corresponds to the last nucleotide of the gene sequence.

The term "adjacent" is used in connection with
(i) a gene sequence to indicate a nucleotide sequence/chromosome region that is sufficiently closely located to said gene sequence in a chromosome, such as for instance less then 1 000 000 for example within 900 000-200 000 nucleotide positions, such as 100 000, e. g. less then 90 000, such as less then 80 000, e. g. less then 70 000, such as less then 60 000, e. g. from 10 000 to 50 000, e. g. 20 000 or 10 000 nucleotide positions, or from 1 to 10 000 nucleotide positions. It is preferred that the adjacent region is in linkage disequilibrium with said gene sequence;
(ii) an oligonucleotide sequence to indicate that said oligonucleotide sequence recognises a sequence that is sufficiently closely located to a specific nucleotide of interest for the oligonucleotide sequence to be suitable for the desired detection technique, such as for instance as a primer for amplification of a target nucleotide sequence. Preferably, adjacent means less than 500, such as less than 400, e.g. less than 300, such as less than 200, e.g. less than 100, such as less than 50 nucleotide positions away from the nucleotide or nucleotide sequence of interest.

As used herein, the term "coding sequence" refers to that portion of a gene that encodes the amino acid sequence of a protein. Exons contain the coding sequence of the gene.

Coding sequences of the above genes (cDNA) are identified herein as

| | |
|---|---|
| NHP2L1 cDNA | SEQ ID NO: 8 |
| PACSIN2 cDNA | SEQ ID NO: 9 |
| SERHL cDNA | SEQ ID NO: 10 |
| PIPPIN cDNA | SEQ ID NO: 11 |
| BRD1 cDNA | SEQ ID NO: 12 |
| EP300 cDNA | SEQ ID NO: 13 |
| GPR24 cDNA | SEQ ID NO: 14.. |
| FAM19A5 | SEQ ID NO: 95 |

The promoter, UTR and intron regions referred herein as the "non-coding region(s)/sequence(s)" of the given genes. As used herein, "intron" refers to a DNA sequence present in a given gene that is spliced out during mRNA maturation. The term "promoter region" refers to the portion of DNA of a gene that controls transcription of the DNA to which it is operatively linked. The promoter region includes specific sequences of DNA that are sufficient for RNA polymerase recognition, binding and transcription initiation. This portion of the promoter region is referred to as the promoter. In addition, the promoter region includes sequences that modulate the recognition, binding and transcription initiation activity of the RNA polymerase. The UTR regions of the gene corresponds to the 5' and 3' sequences that are transcribed into (mature) mRNA but are not translated into protein.

The term "fragment" when used in connection with nucleotide sequences means any fragment of the nucleotide sequence consisting of at least 20 consecutive nucleotides of that sequence.

As used herein, the term "polymorphism" refers to the coexistence of more than one form of a gene or portion thereof. A portion of a gene of which there are at least two different forms, i. e., two different nucleotide sequences, is referred to as a "polymorphic region of a gene". A polymorphic region can be a single nucleotide, the identity of which differs in different alleles. Such polymorphism is referred herein as "single nucleotide polymorphism" or SNP. A polymorphic region also can be several nucleotides in length. A gene having at least one polymorphic region is referred to as a "polymorphic gene".

SNPs, which are known in the art, are identified herein with the numbers corresponding to the refSNP ID NOs (rs numbers) of the NCBI SNP database (hftp://www.ncbi.nlm.nih.gov/SNP/) and UCSC Genome SNP database (http://www.genome.ucsc.edu/), for example such as
rs11561, rs5758405 rs8779, rs132806,
rs2068943, rs2267487
rs881542, rs926333,rs1060387
rs1006407
rs4468, rs138855, rs2239848, rs138880, rs138881
rs20551, rs2294976, rs2076578, rs1046088
rs133068, rs133069
rs 133070, rs 1330739.

By the term "SNP type" is meant the promoter, UTR, intron, synonymous or non-synonymous SNP.

By "promoter SNP" is meant an SNP located in the promoter region of a gene. This type of SNP may affect expression of the gene.

By the term "UTR SNP" is meant a SNP located in part of the genome that is transcribed into mRNA, but this part of the mRNA is not translated into protein. SNPs in this part of the genome may for example affect splicing, regulation of transcription, the fate of the mRNA in the cell changing the stability or the location where the mRNA is transported.

By the term "Intron SNP" is meant a SNP located in an intronic region of the gene. Introns are spliced out of the mature mRNA before it leaves the cell nucleus. This type SNP may affect splicing of the primary transcript into the mature mRNA and thereby influence what is translated into protein (e.g. exon-skipping leading to defect protein, or change of reading-frame leading to truncated protein, or nonsensemediated-decay). Intron SNPs may also have a regulatory potential.

By the term "synonymous SNP" (syn) is meant a SNP which is associated with the change of a nucleotide in the (coding) exon sequence that doesn't lead to the change of an amino acid in the protein encoded by the gene. A syn-SNPs may have an impact on the regulation of the gene and on splicing e.g. by introduction of cryptic splice sites

The term "non-synonymous SNP" (non-syn) designates a SNP which is associated with a nucleotide change in the coding DNA sequence that leads to the change of an amino acid in the sequence of the encoded protein. This type of SNP may lead to a change in the function and/or structure of the protein.

As used herein, "allele", which is used interchangeably herein with "allelic variant" refers to alternative forms of a gene or portions thereof. Alleles occupy the same locus or position on homologous chromosomes. When an individual has two identical alleles of a gene, the individual is said to be homozygous for the gene or allele. When an individual has two different alleles of a gene, the individual is said to be heterozygous for the gene or alleles. Alleles of a specific gene can differ from each other in a single nucleotide, or several nucleotides, and can include substitutions, deletions, and insertions of nucleotides. An allele of a gene also can be a form of a gene containing a mutation.

By the term "susceptibility/risky allele" is in the present content meant the allele of a polymorphism, e. g. SNP, which is associated with a predisposition of an individual carrying this allele for development of a disease, e. g. SCH and/or BPD. By the term "protective allele" is meant the allele of a polymorphism, e. g. SNP, said polymorphism being associated with a predisposition of an individual for development of a disease, the presence of which in the genetic material from an individual, e. g. a DNA sample, indicates no predisposition of an individual carrying this allele for development of a disease, e. g. SCH and/or BPD.

As used herein, "predisposition" means that an individual having a particular genotype and/or haplotype has a higher likelihood than one not having such a genotype and/or haplotype for a particular condition/disease as one of the described herein.

As used herein, the term "haplotype" refers to a set of closely positioned alleles present on one chromosome which tend to be inherited together (not easily separable by recombination).

As used herein, the term "genetic marker" refers to an identifiable physical location on a chromosome (e.g., single nucleotide polymorphism (SNP), restriction enzyme cutting site) whose inheritance can be monitored. Markers can be expressed regions of DNA (genes) or some segment of DNA with no known coding function but whose pattern of inheritance can be determined.

As used herein, the term "linkage" refers to an association in inheritance between genetic markers such that the parental genetic marker combinations appear among the progeny more often than the non-parental.

As used herein, the term "linkage disequilibrium" (LD) means that the observed frequencies of haplotypes in a population does not agree with haplotype frequencies predicted by multiplying the frequencies of individual genetic marker alleles in each haplotype; LD means that there exist correlations among neighbouring alleles, reflecting 'haplotypes' descended from single, ancestral chromosomes.

Target nucleic acid: a nucleic acid isolated from an individual and comprising at least one polymorphism identified in the present invention as well as further nucleotides upstream or downstream. The target nucleic acid can be used for hybridisation, for sequencing or other analytical purposes.

By the term "gene products" is meant herein products of gene transcription, such as an mRNA transcript and said mRNA transcript splicing products, and products of gene translation, such as polypeptide(s) translated from any of the gene mRNA transcripts and various products of post-translational processing of said polypeptides, such as the products of post-translational proteolytic processing of the polypeptide(s) or products of various post-translational modifications of said polypeptide(s).

Alignment. When reference is made to alignment of protein sequences alignment is carried out using the MultAlin algorithm with default settings ("Multiple sequence alignment with hierarchical clustering", F Corpet, 1988, Nucl. Acids Res., 16 (22), 10881-10890), which is available at the internet address: http:/prodes.toulouse.inra.fr/multalin/multalin.html.

### Conservative amino acid substitutions:

Substitutions within the groups of amino acids are considered conservative amino acid substitutions. Substitutions among the groups of amino acids are considered non-conservative amino acid substitutions.
P, A, G, S, T (neutral, weakly hydrophobic)
Q, N, E, D, B, Z (hydrophilic, acid amine)
H, K, R (hydrophilic, basic)
F, Y, W (hydrophobic, aromatic)
L, I, V, M (hydrophobic)
C (cross-link forming)

SCHIZOPHRENIA (SCH) A mental disorder or heterogeneous group of mental disorders (the schizophrenias or schizophrenic disorders) comprising most major psychotic disorders and characterized by disturbances in form and content of thought (loosening of associations, delusions and hallucinations) mood (blunted, flattened or inappropriate affect), sense of self and relationship to the external world (loss of ego boundaries, dereistic thinking and autistic withdrawal) and behavior (bizarre, apparently purposeless and stereotyped activity or inactivity).

BIPOLAR AFFECTIVE DISORDER (BPD) is one of the most common, severe, and persistent mental diseases. It is characterized by periods of deep, prolonged, and profound depression that alternate with periods of excessively elevated and/or irritable mood known as mania. The symptoms of mania include a decreased need for sleep, pressured speech, increased libido, reckless behaviour without regard for consequences, grandiosity, and severe thought disturbances, which may or may not include psychosis.

### Description of drawings

**Figure 1** presents the results of statistical evaluation of the coincidence of the presence of specific haplotypes comprising the SNPs of the BRD1 gene and occurrence of the SCH, BPD or combined SCH and BPD phenotype.
**Figure 2** presents the results of statistical evaluation of the coincidence of the presence of specific haplotypes comprising the SNPs of the GPR24 gene and selected SNPs positioned on chromosome 22q and occurrence of the SCH, BPD or combined SCH and BPD phenotype.
**Figure 3** illustrates one way to connect the cases from the Faroe Islands to a common ancestor. The sex of the individuals in the pedigree is not distinguished.
**Figure 4** shows the statistical analysis of the observed distribution of the overall 1-5 marker haplotypes in the sample from the Faeroe Islands calculated using the program CLUMP. Significant associations with p-values less than 0.05 and less than 0.01 are shaded in light gray and dark grey, respectively.
**Figure 5** presents the degree of pair-wise Linkage Disequilibrium (LD) between the SNPs genotyped in the Scottish case-control sample calculated using LDmax from the GOLD software package..

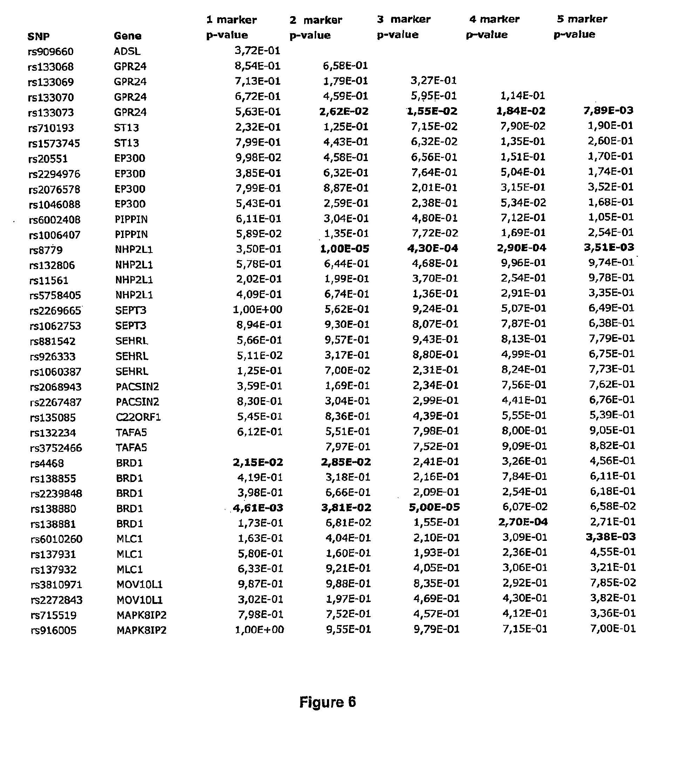

**Figure 6** demonstrates the results of association analysis of haplotypes comprising 1-5 SNPs (BPD and SCH vs. controls)

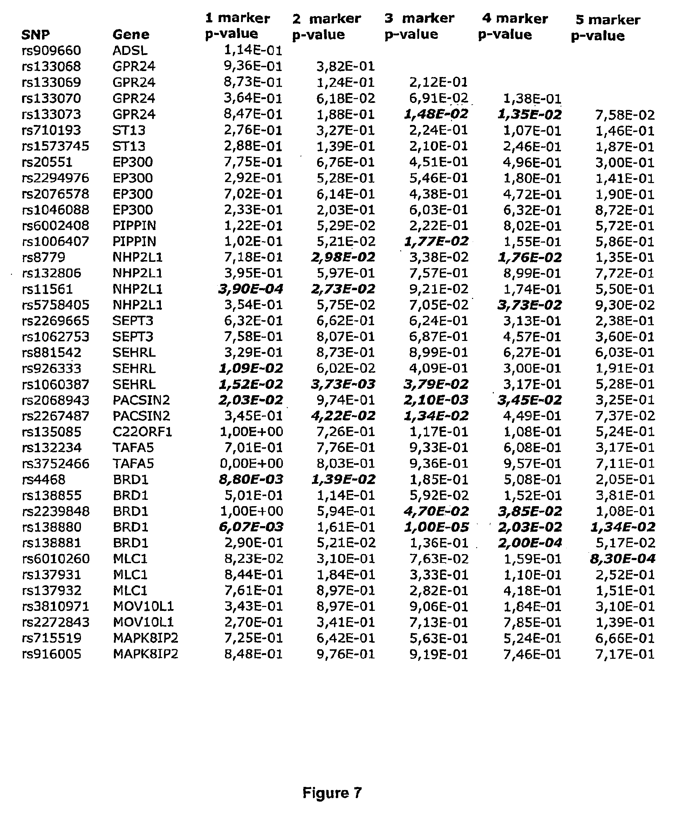

**Figure 7** demonstrates the results of association analysis of haplotypes comprising 1-5 SNPs (SCH vs. controls)

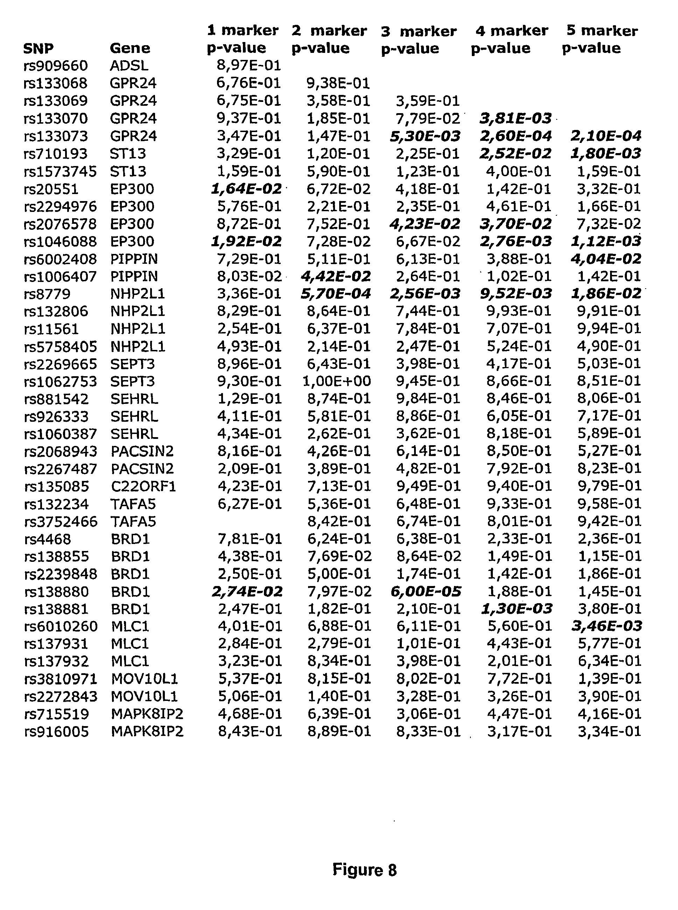

**Figure 8** demonstrates the results of association analysis of haplotypes comprising 1-5 SNPs (BPD vs. controls)

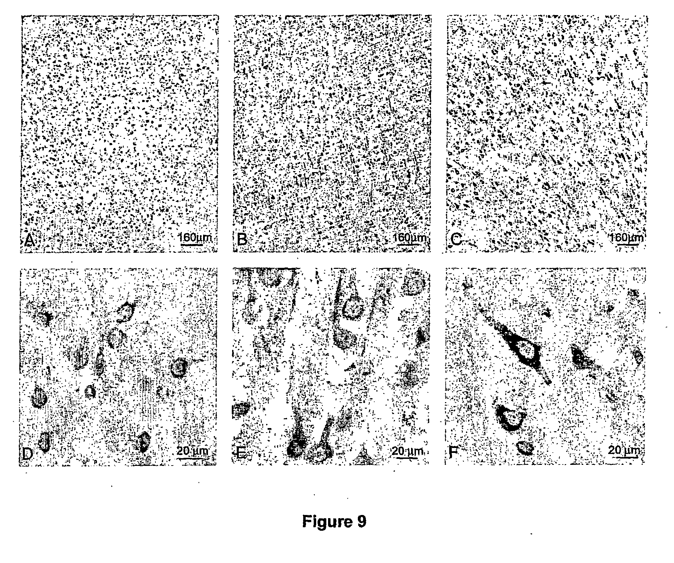

**Fig. 9****:** BRD1 immunostaining of adult rat, rabbit and human cerebral cortex. The 3 species exhibit a similar BRD1 staining pattern. (A) Rat cerebral cortex layer I-VI, (B) Rabbit cerebral cortex layer II-VI, (C) Human cerebral cortex layer II-V, (D) Rat layer II neurons, (E) Rabbit layer VI neurons, (F) Human layer V neurons.

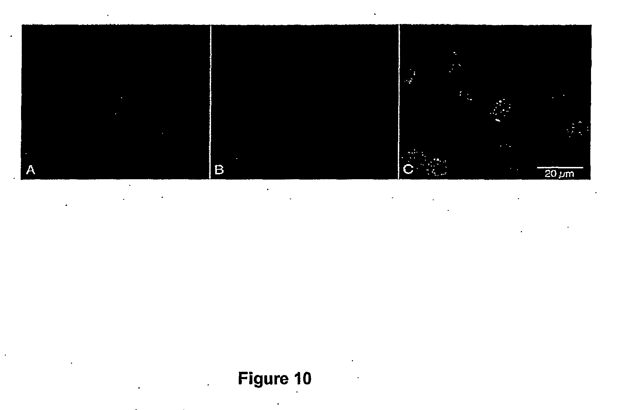

**Fig. 10****:** Confocal microscopic images of BRD1 immunoflouresence stained rat cerebral cortex layer II-III neurons (A), TO-PRO-3 stained cell nuclei in the same area (B), BRD1 and TO-PRO-3 double-stained section (C). The BRD1-immunoreactivity is located both in the cytosol and the nucleus of the neurons whereas surrounding glial cells with small TO-PRO-3 positive nuclei seem BRD1-negative.

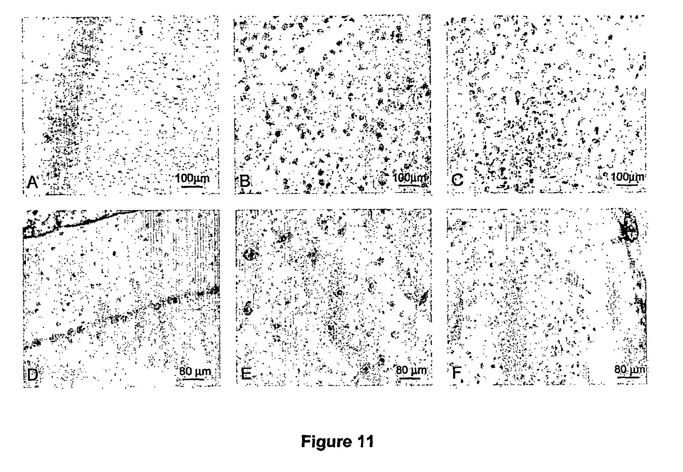

**Fig. 11****:** BRD1-positive neurons in different adult rat and rabbit CNS areas. (A) BRD1-positive pyramidal cells in CA1 of the rabbit hippocampus. Note the prominent staining of the proximal pyramidal dendrites in stratum radiatum. (B) Rabbit striatum. (C) Rat ventromedial hypothalamic nucleus. (D) Rat cerebellar cortex, note BRD1-staining of granule cells, purkinje cells, and molecular layer interneurons. (E) Rat brainstem trigeminal motor nucleus. (F) Caudal part of the trigeminal spinal nucleus in the rat cervical spinal cord.

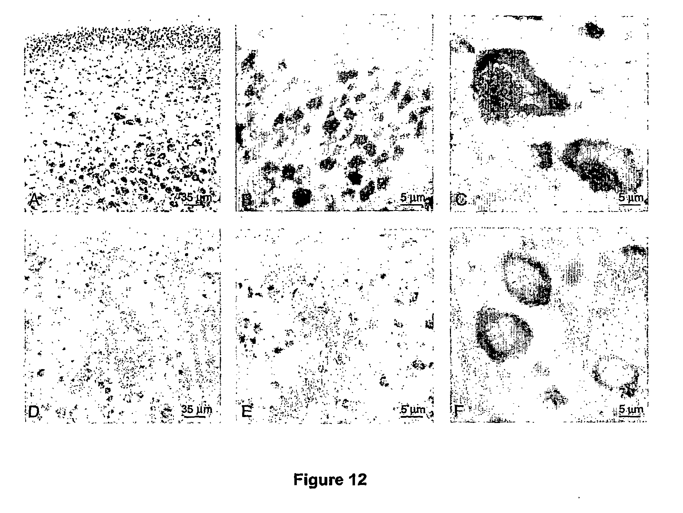

**Fig. 12****:** BRD1 immunostaining of subventricular myelencephalon from fetal pig of embryonic day 60 (A-C) and day of birth/embryonic day 115 (D-F). (A) Next to the lumen of the 4^{th} ventricle, a cell rich neuroepithelial layer is noted wherefrom maturing neuroblasts migrate into the surrounding part of the myelencephalon. (B) Close view of the neuroepithelial layer seen in A. The neuroepithelial cells display prominent nuclear BRD1 immunopositivity. (C) Close view of maturing neuroblasts seen in A. Prominent BRD1-staining is seen both in the nucleus and the surrounding cytosol. (D) Subventricular medulla at the day of birth corresponding to the area depicted in A, displays less prominent BRD1 immunoreactivity. (E) Close view of the subventricular layer seen in D. The subventricular cells display a weaker nuclear BRD1-immunoreactivity than seen in B. (F) Close view of the medullar neurons seen in D. Note the pronounced decrease in nuclear BRD1 immunoreactivity.

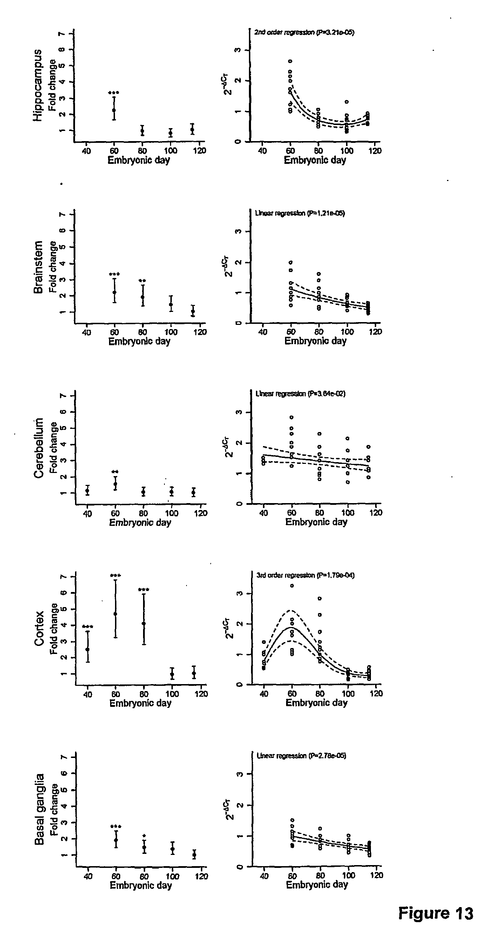

**Fig. 13****:** Relative quantification of *BRD1* mRNA expression in terms of fold changes (2^{-ΔΔC_{T}} and 2^{-ΔC_{T}}) found by analysis of variance (left panel) contrasting to embryonic day 115 the mean threshold difference from each of the other days. The significance of the contrasts is indicated (*: p<0.05, **: p<0.01 and ***: p<0.001) with p-values adjusted for the number of contrasts tested using the Sidák method. A polynomial regression model up to third order (right panel) was determined using a forward inclusion stepwise procedure. The significance of the highest order term is indicated.

### Detailed description of the invention

### Gene Polymorphism

In the first aspect the invention relates to a method for determining a predisposition to a mental disease in a subject comprising determining in a biological sample isolated from said subject one or polymorphisms in the chromosome regions containing the NHP2L1, PACSIN2, SERHL, PIPPIN, BRD1, EP300, FAM19A5 and/or GPR24 genes, or in a translational or transcriptional product from said regions, said polymorphism being indicative of said predisposition.

The invention relates to a mental disease being a disease characterised by at least two symptoms, preferably three or more symptoms, which are related to schizophrenia (SCH) and/or bipolar disorder (BPD). SCH and BPD are preferred mental diseases of the invention. In some embodiments a preferred mental disease is characterised by symptoms of both SCH and BPD.

### Position of polymorphisms

In one embodiment the present invention relates to polymorphisms of the above identified genes, wherein the polymorphisms are located in the non-coding regions of the genes, such as the regions controlling expression of the genes, for example promoter and UTR regions, and the regions controlling the splicing of the gene transcript, such as introns or exon/intron boundaries. Such polymorphisms according to the invention may influence expression of the gene or affect the splicing or maturation of the gene transcript, mRNA.

In another embodiment the invention relates to polymorphisms locates in the coding regions of the gene, such as exons. Such polymorphisms according to the invention may lead to the production of variant proteins. Variant proteins are the proteins, amino acid sequence of which contains an amino acid change, such as substitution(s), insertion(s) and/or deletion(s), corresponding to the polymorphism(s) of the gene. A variant protein may have functional activity altered due to the latter gene polymorphism.

In one aspect the present invention relates to a method comprising the determination of one or more polymorphisms in the chromosome regions, said regions containing the NHP2L1, PACSIN2, SERHL, PIPPIN, BRD1, EP300, FAM19A5 and/or GPR24, and relating said one or more polymorphisms to the predisposition to SCH and BPD. The polymorphisms may be located either/both in the coding region and/or non-coding region of said genes. Another aspect of the invention relates to a method for determining one or more polymorphisms in the chromosome regions, which are in linkage disequilibrium with the polymorphisms of the above genes, and relating said one or more polymorphisms to a predisposition to SCH and/or BPD. The polymorphisms may be located in one individual gene as well as in two or more different individual genes. The polymorphism(s) may be located either or both in the coding and/or non-coding regions of these genes.

In further embodiments a predisposition to a mental disease comprises determining more than one polymorphism in any identified herein genes, or may be determined specifically for a selected disease by determining two or more selected polymorphisms of the invention, wherein said polymorphisms have stronger correlation with said selected disease then other polymorphisms identified in the present application. Thus, the invention relates to determining specific haplotypes comprising two or more identified herein polymorphisms which are associated with a specific mental disease, in particular SCH or BPD. The specific haplotypes of the invention may further comprise one or more polymorphisms of the DNA sequences adjusted to the genes of the invention.

Preferably, the invention relates to polymorphism of DNA being an SNP.

Preferred SNPs according to the invention are the SNPs having refSNP Nos.
rs11561, rs5758405 rs8779, rs132806,
rs2068943, rs2267487
rs881542, rs926333,rs1060387
rs1006407, rs6002408
rs4468, rs138855, rs2239848, rs138880, rs138881
rs20551, rs2294976, rs2076578, rs1046088
rs133068, rs133069, rs133070, rs133073
The above identified group of SNPs consists of the SNPs identified in the genomic sequences of the NHP2L1, PACSIN2, SERHL, PIPPIN, BRD1, EP300, FAM19A5 and/or GPR24 (SEQ ID NO: 1-7 and 94).

Positions of the SNPs within the genomic sequences of the genes (SEQ ID NOS: 1-7 and 94) are identified in Table 1.

**Table 1**

| **Gene** | **SEQ ID NO** | **Chromo some ID** | **SNP ID** | **Nucleotide No** **(position of SNP*)** | **SNP** | **Type of SNP** |
|---|---|---|---|---|---|---|
| **NHP2L1** | 1 | 22q13.2 | rs8779 | 40313315 | C/T | UTR |
| **NHP2L1** | 1 | 22q13.2 | rs132806 | 40313832 | C/T | UTR |
| **NHP2L1** | 1 | 22q13.2 | rs11561 | 40314236 | T/C | Non-syn |
| **NHP2L1** | 1 | 22q13.2 | rs5758405 | 40319420 | C/A | Intron |
| | | | | | | |
| **PACSIN2** | 2 | 22q13.2 | rs2068943 | 41587252 | C/T | Promoter |
| **PACSIN2** | 2 | 22q13.2 | rs2267487 | 41642330 | C/T | Promoter |
| | | | | | | |
| **SERHL** | 3 | 22q13.2 | rs881542 | 41126941 | C/G | Prom |
| **SERHL** | 3 | 22q13.2 | rs926333 | 41128686 | A/G | Non-syn |
| **SERHL** | 3 | 22q13.2 | rs1060387 | 41136001 | C/T | Non-syn |
| | | | | | | |
| **PIPPIN** | 4 | 22q13.2 | rs6002408 | 40210909 | C/T | UTR |
| **PIPPIN** | 4 | 22q13.2 | rs1006407 | 40215071 | C/T | UTR |
| | | | | | | |
| **BRD1** | 5 | 22q13.3 | rs4468 | 48386988 | T/C | UTR |
| **BRD1** | 5 | 22q13.3 | rs138855 | 48417818 | G/C | Intron |
| **BRD1** | 5 | 22q13.3 | rs2239848 | 48436090 | G/A | Syn |
| **BRD1** | 5 | 22q13.3 | rs138880 | 48437947 | A/C | Promoter |
| **BRD1** | 5 | 22q13.3 | rs138881 | 48439818 | G/A | Promoter |
| | | | | | | |
| **EP300** | 6 | 22q13.2 | rs20551 | 39781047 | A/G | Non-syn |
| **EP300** | 6 | 22q13.2 | rs2294976 | 39807747 | C/A | Intron |
| **EP300** | 6 | 22q13.2 | rs2076578 | 39812648 | C/T | Intron |
| **EP300** | 6 | 22q13.2 | rs1046088 | 39817422 | A/C | Non-syn |
| | | | | | | |
| **GPR24** | 7 | 22q13.2 | rs133068 | 39317446 | C/G | Promoter |
| **GPR24** | 7 | 22q13.2 | rs133069 | 39317501 | A/C | Promoter |
| **GPR24** | 7 | 22q13.2 | rs133070 | 39317812 | A/G | Promoter |
| **GPR24** | 7 | 22q13.2 | rs133073 | 39318734 | C/T | Syn |
| | | | | | | |
| **FAM19A5** | 84 | 22q13.3 | Rs132234 | 47424523 | C/T | Intron |
| **FAM19A5** | 84 | 22q13.3 | Rs3752466 | 47466709 | C/T | 3'UTR |
| | | | | | | |

According to the invention the above SNPs are genetic markers of a mental disease of the invention. * Nucleotide position in a genomic sequence is given according to the sequences of the UCSC database, July 2003 assembly (http://www.genome.ucsc.edu/).

The invention describes several haplotypes of the above SNPs in association with SCH and/or BPD.

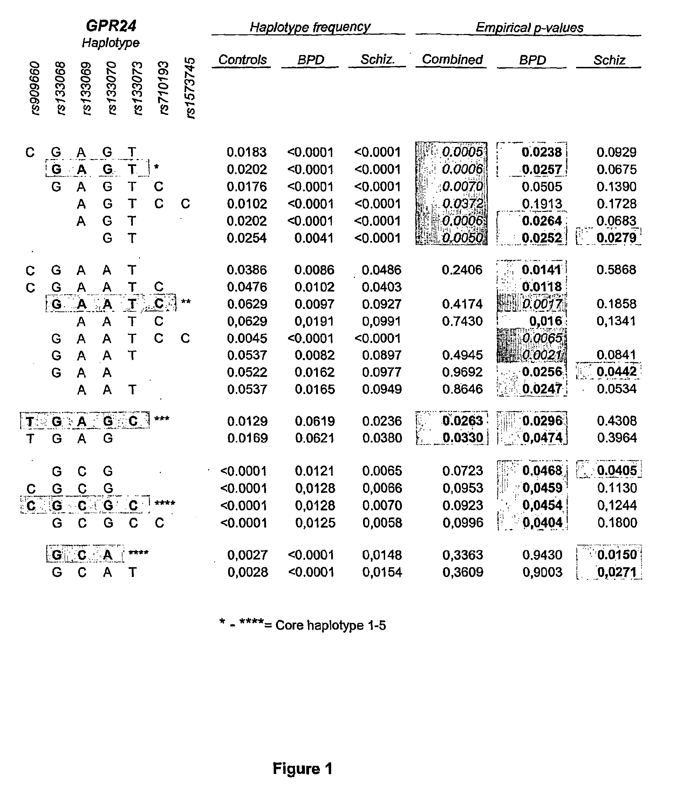

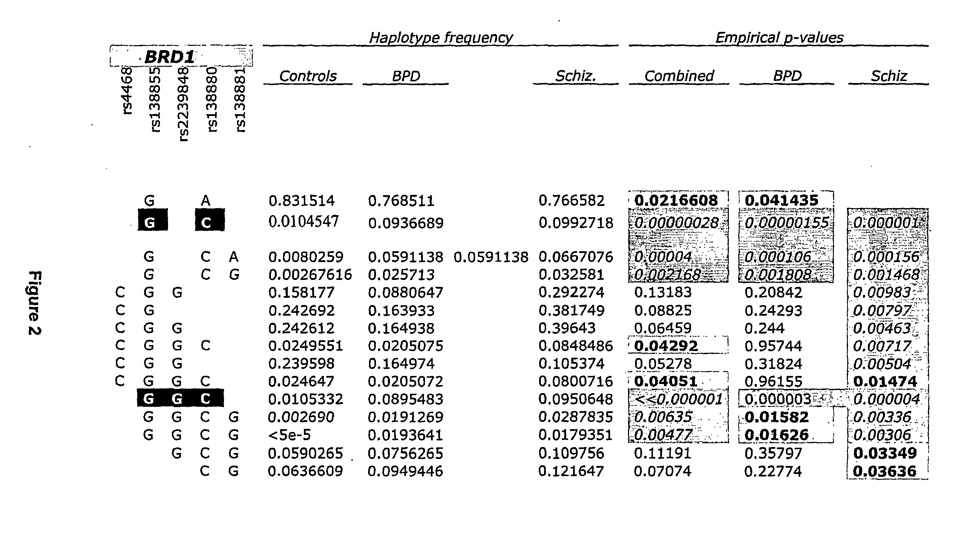

The following is non-limited examples of specific haplotypes of the invention:
A three SNP haplotype consisting of the SNPs rs133069, rs133070, rs133073 of the GRP24 gene is according to invention indicative of BPD,
A four SNP haplotype consisting of rs133069, rs133070, rs133073 rs133068 of the GRP24 gene is according to invention indicative of SCH.
More examples of the SNPs and haplotypes of the invention are shown in Figures 1 and 2 of the present application and in Table 2 below.

**Table 2.**

| *Gene* | *SNP ID NO* | *SNP* | |
|---|---|---|---|
| BRD1 | rs4468 | T/C | C over-represented in SCH/Part of haplotype associated with SCH and combined BPD&SCH |
| BRD1 | rs138855 | G/C | Part of haplotype associated with SCH and BPD |
| BRD1 | rs2239848 | G/A | Part of haplotype associated with SCH, BPD and combined BPD&SCH |
| BRD1 | rs138880 | A/C | C over-represented in BPD and SCH /Part of haplotype iassociated with SCH, BPD and combined BPD&SCH |
| BRD1 | rs138881 | G/A | Part of haplotype associated with SCH, BPD and combined BPD&SCH |
| | | | |
| EP300 | rs20551 | A/G | A over-represented in BPD/part of haplotype associated with BPD |
| EP300 | rs2294976 | C/A | part of haplotype associated with BPD |
| EP300 | rs2076578 | C/T | part of haplotype associated with BPD |
| EP300 | rs1046088 | A/C | A over-represented in BPD/part of haplotype associated with BPD |
| | | | |
| GPR24 | rs133068 | C/G | Part of haplotype associated with SCH, BPD and Combined BPD&SCH |
| GPR24 | rs133069 | A/C | Part of haplotype associated with SCH, BPD and Combined BPD&SCH |
| GPR24 | rs133070 | A/G | Part of haplotype associated with SCH, BPD and Combined BPD&SCH |
| GPR24 | rs133073 | C/T | Part of haplotype associated with SCH, BPD and Combined BPD&SCH |
| NHP2L1 | rs8779 | C/T | Part of haplotype associated with SCH, BPD and combined BPD&SCH |
| NHP2L1 | rs132806 | C/T | Part of haplotype associated with SCH |
| NHP2L1 | rs11561 | T/C | C over-represented in SCH /Part of haplotype associated with SCH |
| NHP2L1 | rs5758405 | C/A | Part of haplotype associated with SCH |
| | | | |
| PACSIN2 | rs2068943 | C/T | C over-represented in SCH /part of haplotype associated with SCH in SCH /part of haplotype associated with |
| PACSIN2 | rs2267487 | C/T | part of haplotype associated with SCH |
| | | | |
| SERHL | rs881542 | C/G | C over-represented in SCH |
| SERHL | rs926333 | A/G | A over-represented in SCH/Part of haplotype associated with SCH |
| SERHL | rs1060387 | C/T | Part of haplotype associated with SCH |
| | | | |
| PIPPIN | rs6002408 | C/T | Part of haplotype associated with SCH, BPD and Combined BPD&SCH |
| PIPPIN | rs1006407 | C/T | Part of haplotype associated with SCH, BPD and Combined BPD&SCH |

The invention features haplotypes of the above SNPs that are present on one chromosome, e. g. within the sequence of one particular gene, or located within the sequences of two different juxtaposed genes, which tend to be inherited together. The latter group of SNPs may for example consist of the SNPs having refSNP ID NO: rs909660, rs710193, rs1573745, rs132234, rs3752466, rs6010260, rs137931, rs137932, rs3810971, rs2272843, rs1063900, rs715519, rs916005. This group of SNPs includes the SNPs which are identified in the present invention as parts of different haplotypes, said haplotypes being associated with SCH and/or BPD (as for example shown in Figure 1, 2 and 4 of the present application.

In another aspect the invention relates to polymorphisms located in the chromosome regions, which do not contain the above identified genes, said polymorphisms being in linkage disequilibrium with at least one of the above identified SNPs. In particular, the invention relates to polymorphisms in the human chromosome 22q being in linkage disequilibrium with one or more polymorphisms in the NHP2L1, PACSIN2, SERHL, PIPPIN, BRD1, EP300, FAM19A5 and/or GPR24 genes, such as an SNP selected from rs11561, rs5758405 rs8779, rs132806, rs2068943, rs2267487, rs881542, rs926333, rs1060387, rs1006407, rs4468, rs138855, rs2239848, rs138880, rs138881, rs20551, rs2294976, rs2076578, rs1046088, rs133068, rs133069, rs133070, rs133073, rs6002408 as well as the microsatelites D22S922 and D22S1169

Likewise, any polymorphism of the genes being adjacent to the genes of the invention, in particular a polymorphism(s) being located within the sequence of 1 to 1 000 000 nucleotides adjacent to said genes, and is in linkage disequilibrium with any of the SNPs identified above, is in the scope of the invention. The SNPs located within 50-100 0000 nucleotides sequence adjusted to the nucleotide corresponding to the first or the last nucleotide of a sequence selected from the sequences identified as SEQ ID NO: 1-7 and 94 are preferred.

### Gene Products

The invention relates to a method for determining a predisposition to a mental disease comprising determining at least one polymorphism in any of the above genes or in transcriptional or translational products of the genes.

As used herein, the term "transcriptional product of the gene" refers to a pre-messenger RNA molecule, pre-mRNA, that contains the same sequence information (albeit that U nucleotides replace T nucleotides) as the gene, or mature messenger RNA molecule, mRNA, which was produced due to splicing of the pre-mRNA, and is a template for translation of genetic information of the gene into a protein.

As used herein, the term "translational product of the gene" refers to a protein, which is encoded by the gene.

Thus, the invention includes in the scope of protection nucleic acids comprising the coding nucleotide sequences of the above genes comprising a polymorphism and proteins comprising a polymorphism corresponding to the polymorphism of the encoding nucleic acid sequence.

In particular, the invention relates to transcriptional products of the above genes being
(i) nucleic acid sequences identified in the invention as SEQ ID NO: 8-14, or fragments thereof,
(ii) nucleic acid sequences having at least 90% identity with SEQ ID NO: 8-14, or fragments thereof,
(iii) nucleic acid sequences being complementary to any of the sequences of (i) or (ii),
said nucleic acid sequences comprising the polymorphisms of the genomic sequences described above.

Translational products of the genes of the invention are identified herein as
(i) variant proteins corresponding to the proteins identified in the NCBI database under Ass. Nos.: NP_001003796/004999 (NHP2L1) (SEQ ID NO: 87), NP_009160 (PACSIN2) (SEQ ID NO: 88), NP_733795 (SERHL) (SEQ ID NO: 89), NP_055275 (PIPPIN) (SEQ ID NO: 90), NP_055392 (BRD1) (SEQ ID NO: 91), NP_001420 (EP300) (SEQ ID NO: 92), NP_005288 (GPR24) (SEQ ID NO: 93), or variants, or fragments thereof,
(ii) polypeptide sequences having at least 90% identity with the variant proteins, or fragments thereof, of (i),
said variant proteins, fragments thereof and said polypeptide sequences are comprising polymorphism corresponding to the polymorphism of the corresponding genomic sequences or transcriptional products of said genomic sequences.

Selected, but non-limited examples of protein polymorphism of the invention are given in Table 3 below:

**Table 3**

| **Gene** | **SNP ID.** | **Alleles** | **Protein polymorphism** |
|---|---|---|---|
| EP300 | rs1046088 | A/C | Gln2223Pro |
| EP300 | rs20551 | A/G | Ile997Val |
| | | | |
| SERHL | rs926333 | A/G | Ser46Ala Ala2Val |
| SERHL | rs1060387 | C/T | |
| | | | |
| NHP2L1 | rs11561 | T/C | (Thr43Ala |

Thus, it is an embodiment of the invention to use the above identified variant proteins for the purpose of
i) diagnosis of SCH and/or BPD in a neuropsychiatric patient, and/or
ii) prognosis of likelihood of development of SCH and/or BPD by an individual, and/or
iii) development new drug candidates for the treatment of SCH and/or BPD, and/or
iv) therapeutic treatment of SCH and/or BPD.

### Methods of determining polymorphisms

### 1 SNP

Many methods (see Table 4 below) are known in the prior art for determining the presence of particular nucleotide sequences or for determining particular proteins having particular amino acid sequences. All of these methods may be adapted for determining the polymorphisms according to the present invention.

**Table 4.**

| **Method** | **Result** |
|---|---|
| Restriction fragment length polymorphism | Cleavage or non-cleavage based on SNP results in difference in length |
| Amplified fragment length polymorphism | Cleavage or non-cleavage based on SNP results in difference in length |
| Mass spectrometry | Difference in molecular weight of hybrids between a probe and the different alleles |
| Single strand conformation polymorphism (SSCP). SSCP heteroduplex. | Different separation in gel based on different conformation caused by single nucleotide polymorphism. |
| Single nucleotide extension | Difference in signal through incorporation of differently labelled nucleotide or labelled/non-labelled nucleotide |
| Sequencing | Difference in sequence |
| Hybridisation | Hybridisation or non-hybridisation at high stringency. Often detected by using differently labelled probes. |
| Determination of Tₘ profile | Difference in Tₘ profile between target and homologous vs. non-homologous probe. |
| Cleavage of single-stranded DNA | |
| Denaturing HPLC | DHPLC is based on resolving heteroduplex from homoduplex DNA fragments produced by PCR amplification using temperature-modulated heteroduplex analysis. |
| TAQMAN | PCR based technique. |

One common method for detecting SNPs comprises the use of a probe bound to a detectable label. By carrying out hybridisation under conditions of high stringency it is ensured that the probe only hybridises to a sequence which is 100% complementary to the probe. According to the present invention this method comprises hybridising a probe to a target nucleic acid sequence comprising at least one of the SNPs at the positions identified in Table 1 (see above). For other polymorphisms or mutations within the defined region, similar probes can be designed by the skilled practitioner and used for hybridisation to a target nucleic acid sequence. The design and optimisation of probes and hybridisation conditions lies within the capabilities of the skilled practitioner.

In the scope of the present invention the term "hybridisation" signifies hybridisation under conventional hybridising conditions, preferably under stringent conditions, as described for example in Sambrook et al., Molecular Cloning, A Laboratory Manual, 2nd Edition (1989) Cold Spring Harbor Laboratory Press, Cold Spring Harbor, N.Y.). The term "stringent" when used in conjunction with hybridisation conditions is as defined in the art, i.e. 15-20°C under the melting point Tₘ, cf. Sambrook et al, 1989, pages 11.45-11.49. Preferably, the conditions are "highly stringent", i.e. 5-10°C under the melting point Tₘ. Under highly stringent conditions hybridisation only occurs if the identity between the oligonucleotide sequence and the locus of interest is 100 %, while no hybridisation occurs if there is just one mismatch between oligonucleotide and DNA locus. Such optimised hybridisation results are reached by adjusting the temperature and/or the ionic strength of the hybridisation buffer as described in the art. However equally high specificity may be obtained using high-affinity DNA analogues. One such high-affinity DNA analogues has been termed "locked nucleic acid" (LNA). LNA is a novel class of bicyclic nucleic acid analogues in which the furanose ring conformation is restricted in by a methylene linker that connects the 2'-O position to the 4'-C position. Common to all of these LNA variants is an affinity toward complementary nucleic acids, which is by far the highest reported for a DNA analogue (Ørum et al. (1999) Clinical Chemistry 45, 1898-1905; WO 99/14226 EXIQON). LNA probes are commercially available from Proligo LLC, Boulder, Colorado, USA. Another high-affinity DNA analogue is the so-called protein nucleic acid (PNA). In PNA compounds, the sugar backbone of an oligonucleotide is replaced with an amide containing backbone, in particular an aminoethylglycine backbone. The nucleobases are retained and are bound directly or indirectly to aza nitrogen atoms of the amide portion of the backbone *(*Science (1991) 254: 1497-1500).

Various different labels can be coupled to the probe. Among these fluorescent reporter groups are preferred because they result in a high signal/noise ratio.

Suitable examples of the fluorescent group include fluorescein, Cy2, Cy3, Cy3.5, Cy5, Cy5.5, Cy7, acridin, Hoechst 33258, Rhodamine, Rhodamine Green, Tetramethylrhodamine, Texas Red, Cascade Blue, Oregon Green, Alexa Fluor, europium and samarium.

Another type of labels are enzyme tags. After hybridisation to the target nucleic acid sequence a substrate for the enzyme is added and the formation of a coloured product is measured. Examples of enzyme tags include a beta-Galactosidase, a peroxidase, horseradish peroxidase, a urease, a glycosidase, alkaline phosphatase, chloramphenicol acetyltransferase and a luciferase.

A further group of labels include chemiluminescent group, such as hydrazides such as luminol and oxalate esters.

A still further possibility is to use a radioisotope and detect the hybrid using scintillation counting. The radioisotope may be selected from the group consisting of ³²P, ³³P, ³⁵S, ¹²⁵I, ⁴⁵Ca, ¹⁴C and ³H.

One particularly preferred embodiment of the probe based detection comprises the use of a capture probe for capturing a target nucleic acid sequence. The capture probe is bound to a solid surface such as a bead, a well or a stick. The captured target nucleic acid sequence can then be contacted with the detection probe under conditions of high stringency and the allele be detected.

One embodiment of the probe based technique based on TAQMAN technique. This is a method for measuring PCR product accumulation using a dual-labeled flourogenic oligonucleotide probe called a TAQMAN® probe. This probe is composed of a short (ca. 20-25 bases) oligodeoxynucleotide that is labeled with two different flourescent dyes. On the 5' terminus is a reporter dye and on the 3' terminus is a quenching dye. This oligonucleotide probe sequence is homologous to an internal target sequence present in the PCR amplicon. When the probe is intact, energy transfer occurs between the two flourophors and emission from the reporter is quenched by the quencher. During the extension phase of PCR, the probe is cleaved by 5' nuclease activity of Taq polymerase thereby releasing the reporter from the oligonucleotide-quencher and producing an increase in reporter emission intensity.

Other suitable methods include using mass spectrometry, single base extension, determining the Tm profile of a hybrid between a probe and a target nucleic acid sequence, using single strand conformation polymorphism, using single strand conformation polymorphism heteroduplex, using RFLP or RAPD, using HPLC, using sequencing of a target nucleic acid sequence from said biological sample.

Denaturing high-performance liquid chromatography (DHPLC) has been proven useful in human and animal genetic studies for detecting single nucleotide polymorphisms (SNPs). In contrary to most SNP detection methods that are currently in used, SNP detection by DHPLC is not based on a re-sequencing strategy that is expensive to implement, nor does it require gel-based genotyping procedures. Instead, SNP detection by DHPLC is based on resolving heteroduplex from homoduplex DNA fragments produced by PCR amplification using temperature-modulated heteroduplex analysis.

In connection with several of these methods there is a need for amplifying the amount of target nucleic acid in the biological sample isolated from the subject. Amplification may be performed by any known method including methods selected from the group consisting of polymerase chain reaction (PCR), Ligase Chain Reaction (LCR), Nucleic Acid Sequence-Based Amplification (NASBA), strand displacement amplification, rolling circle amplification, and T7-polymerase amplification.

More particularly, PCR-based amplification can be carried out using for example a primer pair comprising appropriate sequences selected from the sequences identified in Table 5 below.

**Table 5**

| **Gene** | **SNP Rs ID No.** | | **Primer** | **SEQ ID** |
|---|---|---|---|---|
| **NHP2L1** | rs8779 | F | AGCAGCATACCAAAGAGATG | 15 |
| | | R | ACA CAT GAA CTA GCA CTC TC | 16 |
| | | snp | AAT GCT GCA GGA CTC CGG AGG C | 17 |
| | rs132806 | F | TTC AAC GGT GCC ACC ACC | 18 |
| | | R | ATC CAT TCA GCA GTC CAT TG | 19 |
| | | snp | AAC TGA CTA AAC TAG GTG CCA CGT CGT GAA ACC TGT AAA ACA GAA CAA AAA | 20 |
| | rs11561 | F | TGT AGG GCT TGA GAA AAT GC | 21 |
| | | R | CAG GTG CAG AAT GAT CTC C | 22 |
| | | snp | AAC TGA CTA AAC TAG GTG CCA CGC CCC TGT TGA GGG TTT TGG | 23 |
| | rs5758405 | F | TTA CAT GAC TGC TGA ACG AG | 24 |
| | | R | GTC CAT TGT TAA CTA CCT GG | 25 |
| | | snp | CTC AGT CTA TGG GGG GGA | 26 |
| **PIPPIN** | rs6002408 | F | CGA ACC ATA TTC TAG ACC ATC | 27 |
| | | R | TCG ACT CTG AAG TCA TGG TG | 28 |
| | | snp | GAG CCC GTG GCT GGT GAG GC | 29 |
| | rs1006407 | F | CCA AGA TGT GTG TGC ACC C | 30 |
| | | R | CGG CCC CAC TTC TCC CC | 31 |
| | | snp | CCA GGA AGG AGG GCC CTG TC | 32 |
| **EP300** | Rs20551 | F | ATC CTC CAT CTA CTA GTA GC | 33 |
| | | R | GAA GTA CTT GGC TGG TCT TC | 34 |
| | | snp | AAC TGA CTA AAC TAG GTG CCA CGT CGT GAA CAG ATA CGC AGC CGG AGG AT | 35 |
| | Rs2294976 | F | ATG GTT ACT ATT GGT GAT TCC | 36 |
| | | R | TCG GTA TGG AAA GGA TTC TG | 37 |
| | | snp | AAC TGA CTA AAC TAG GTG CCA CGT CGT GAA AGT CTG ACA AGT TAT GTT GTG GTT CCC CCA | 38 |
| | Rs2076578 | F | GGA GAT ATT CTG TGC TAT TCC | 39 |
| | | R | TGC TGT CTC CCT TGG TCA C | 40 |
| | | snp | ACT GAC TAA ACT AGG TGC AAA CCT TAT TTT CTT GTC TC | 41 |
| | Rs1046088 | F | CAA CCA TAA CCA GTT CCA GC | 42 |
| | | R | CAT ATT TCC TTG TTG CAT CTG | 43 |
| | | snp | AAC TGA CTA AAC TAG AGT TGG CTA CCC ACC ACA GC | 44 |
| **PACSIN2** | rs2068943 | F | CAG CAG TCA GTG GGA CAG | 45 |
| | | R | TCT TAG GAG ACA GTC GTG C | 46 |
| | | snp | AAC TGA CTA AAC TAG GTG CCA CGT CGT GAA AGT CTG ACT TGC ACA GGA TAA TCT TAC A | 47 |
| | rs2267487 | F | TGA GTA GAG AGG CGG CTC | 48 |
| | | R | GAT TAC CTT CCC TGA ACT TC | 49 |
| | | snp | AAC TGA CTA AAC TAG GTG GCG AGC GGC TTT GGG AGA GT | 50 |
| **BRD1** | rs138881 | F | ATC CGC CGA TTC CAC TAA C | 51 |
| | | R | ACG AGT GAC ACG ATT GAG G | 52 |
| | | snp | AAG GAG TTC TGA GAA TCC GTA G | 53 |
| | rs138880 | F | TAC CTG GTC CTA TCG GAT G | 54 |
| | | R | TTT CCA TGG TGG TCA CTG C | 55 |
| | | snp | AAC TGA CAC AGG CCC TCC ACA CGC ACA | 56 |
| | rs4468 | F | TCT TTC CAC AAA GTA CCA TTC | 57 |
| | | R | TCC AAG TTT TCT AGG AAC CC | 58 |
| | | snp | AAC TGA CTA AAC TAG GTG CGT GAC GCC AGC AGC ACG GCC | 59 |
| | rs138855 | F | GAG GTA CAT GCG TGT AGT C | 60 |
| | | R | GCT GAC ACA GAT GTG GTT G | 61 |
| | | snp | AAC TGA CTA AAC TAG GGC ACT CCA GCC TGG GTG ACA | 62 |
| | rs2239848 | F | ACA CGG TCG GCA GGA CC | 63 |
| | | R | AAA GAC CGC TTA CTG TGA TG | 64 |
| | | snp | AAC TGA CAG ACG CCA TTT TTC ATT TC | 65 |
| **SERHL** | rs1060387 | F | AAC AGG ACA TGG GCC TGC | 66 |
| | | R | CCT TGT GAC GAG AAT TCA CC | 67 |
| | | snp | AAC TGA CTA AAC TAG GTG CCA CGT CGT GAA AAC AGC ATT GAC TTC GTC AG | 68 |
| | rs881542 | F | TTT AGC CAT TTT GAC CAG GC | 69 |
| | | R | CTG GTC TCG AAC TCT CAA C | 70 |
| | | snp | AAC TGA CTA AAC TAG GTG CCA CGT CGT GAA AGT CTG ACA ATC CCA GCA TTT TGG GAG G | 71 |
| | rs881542 | F | TGT GTC TTT CAG GTC TGA TC | 72 |
| | | R | GAA GAG GGA TGA GTC TGT C | 73 |
| | | snp | AAC TGA CTA AAC TAG GTG CCA CGT CGT CGG CTG GCT GGA CAA TGC CA | 74 |
| **GPR24** | rs133068 | F | AAG CAT GGT TCC TCA CTT CC | 75 |
| | | R | AGA TCC CTA CAT GTG TCC TC | 76 |
| | | snp | AAC TGA CTA AAG AGG GGT TTT CTG TCT CCC C | 77 |
| | rs133069 | F | AAG CAT GGT TCC TCA CTT CC | 78 |
| | | R | AGA TCC CTA CAT GTG TCC TC | 79 |
| | | snp | ATC CCC CAC CCC ACC CCC | 80 |
| | rs133070 | F | CCT GAG TCT GGC AGT GGG | 81 |
| | | R | CAC CCT CGG CCC CTT CC | 82 |
| | | snp | AAC TGA CTA AAC TAG TCA GAT ATT TGT CTC AAA GA | 83 |
| | rs133073 | F | GGA GCT CAG CTC GGT TGT G | 84 |
| | | R | GCT CTG CTC CTG CCC GTC | 85 |
| | | snp | AAC TGA CTA AAC TAG GTG CCA CGT CGT CTG CTG CCC ACT GGT CCC AA | 86 |

| | | | | |
|---|---|---|---|---|
| F - forward PCR primer R - reverse PCR primer snp - primers for the single base extension detection method | | | | |

One of the primers may comprise a moiety for subsequent immobilisation of the amplified fragments.

It is understood that the primers identified above may also be used as probes for determining the polymorphisms of the invention in a nucleic acid sequence using any of the methods known in the art and featured above.

To the extent that the polymorphisms as defined in the present invention are present in DNA sequences transcribed as mRNA transcripts these transcripts constitute a suitable target sequence for detection of the polymorphisms. Commercial protocols are available for isolation of total mRNA. Through the use of suitable primers the target mRNA can be amplified and the presence or absence of polymorphisms be detected with any of the techniques described above for detection of polymorphisms in a DNA sequence.

### 2. Proteins

As discussed above, genetic polymorphism of the invention can also be detected as a polymorphism of a protein product of the gene, or a change in a biological response mediated by the protein.

The polymorphism located for example in the NHP2L1, SERHL or EP300 genes may also be detected by isolating the protein from a biological sample and determining the presence or absence of the mutated residue (according to Table 3 above) by sequencing said protein, or determining the presence or absence of another polymorphic amino acid located in a mutant gene. The polymorphism of any of the variant proteins of the invention may be detected likewise.

Isolated/identified variant proteins expressed by any of the other polymorphic genes of the invention are used as alternative diagnostic markers of the genetic polymorphism associated with a predisposition to a mental disease of the invention.

The invention also concerns using variant proteins described herein, or fragments or variants thereof, said fragments and variants comprising a polymorphism corresponding to the polymorphism of the variant protein, for the manufacture of an antibody, which may be used both in methods for diagnosis and methods of treatment of SCH and/or BPD.

### Isolated oligonucleotides

In one aspect the invention relates to an isolated oligonucleotide comprising at least 10 contiguous nucleotides being 100% identical to a subsequence of the genes of the invention comprising or adjacent to a polymorphism or mutation being correlated to an mental disease, or being 100% identical to a subsequence of the human genome which is in linkage disequilibrium with any of the genes of the invention comprising or adjacent to a polymorphism or mutation being correlated to a mental disease. As explained in the summary, such probes may be used for detecting the presence of a polymorphism of interest and/or they may constitute part of a primer pair and/or they may form part of a gene therapy vector used for treating the mental diseases.

Preferably the isolated oligonucleotide comprises at least 10 contiguous bases of a sequence identified as SEQ ID NOs: 8-14 or the corresponding complementary strand, or a strand sharing at least 90% sequence identity more preferably at least 95% sequence identity with SEQ ID NOs: 8-14 or a complementary strand thereof, said isolated oligonucleotide comprising a polymorphism of the invention.

Further preferred isolated oligonucleotide may comprise at least 10 contiguous bases of any of the sequences identified as SEQ ID NOS: 1-7 and 94 or the corresponding complementary strand thereof, or a strand sharing at least 90% sequence identity more preferably at least 95% sequence identity with any of the SEQ ID NOS: 1-7 and 94 or a complementary strand thereof, said isolated oligonucleotide comprising a polymorphism of the invention.

These particular oligonucleotides may be used as probes for assessing the polymorphisms in the human BRD1, NHP2L1, PACSIN2, SERHL, PIPPIN, EP300, FAM19A5 and GPR24 genes which are strongly correlated with mental diseases of the invention, such as SCH and/or BPD.

The length of the isolated oligonucleotide depends on the purpose. When being used for amplification from a sample of genomic DNA, the length of the primers should be at least 15 and more preferably even longer to ensure specific amplification of the desired target nucleotide sequence. When being used for amplification from mRNA the length of the primers can be shorter while still ensuring specific amplification. In one particular embodiment one of the pair of primers may be an allele specific primer in which case amplification only occurs if the specific allele is present in the sample. When the isolated oligonucleotides are used as hybridisation probes for detection, the length is preferably in the range of 10-15 nucleotides. This is enough to ensure specific hybridisation in a sample with an amplified target nucleic acid sequence. When using nucleotides which bind stronger than DNA (e.g. LNA and/or PNA), the length of the probe can be somewhat shorter, e.g. down to 7-8 bases.

The length may be at least 15 contiguous nucleotides, such as at least 20 nucleotides. An upper limit preferably determines the maximum length of the isolated oligonucleotide. Accordingly, the isolated oligonucleotide may be less than 1000 nucleotides, more preferably less than 500 nucleotides, more preferably less than 100 nucleotides, such as less than 75 nucleotides, for example less than 50 nucleotides, such as less than 40 nucleotides, for example less than 30 nucleotides, such as less than 20 nucleotides.

The isolated oligonucleotide may comprise from 10 to 50 nucleotides, such as from 10 to 15, from 15 to 20, from 20 to 25, or comprising from 20 to 30 nucleotides, or from 15 to 25 nucleotides.

Depending on the use the polymorphism may be located in the centre of the nucleic acid sequence, in the 5' end of the nucleic acid sequence, or in the 3' end of the nucleic acid sequence.

For detection based on single base extension the sequence of the oligonucleotide is adjacent to the mutation/polymorphism, either in the 3' or 5' direction.

The isolated oligonucleotide sequence may be complementary to a sub-sequence of the coding strand of a target nucleotide sequence or to a sub-sequence to the non-coding strand of a target nucleotide sequence as the polymorphism may be assessed with similar efficiency in the coding and the non-coding strand.

The isolated oligonucleotide sequence may be made from RNA, DNA, LNA, PNA monomers or from chemically modified nucleotides capable of hybridising to a target nucleic acid sequence. The oligonucleotides may also be made from mixtures of said monomers.

### Antibody

Antibodies directed against unimpaired (wild type) or mutant polypeptide products of the BRD1, NHP2L1, PACSIN2, SERHL, PIPPIN, EP300, FAM19A5 and/or GPR24 genes or conserved variants or peptide fragments thereof, which are discussed, above, may also be used as compounds in the methods for diagnosis/prognosis of a BRD1, NHP2L1, PACSIN2, SERHL, PIPPIN, EP300, FAM19A5 and/or GPR24 gene associated disorder or a neuropsychiatric disorder, in particular such as SCH and BPD.

Such methods may be used to detect abnormalities in the level of the BRD1, NHP2L1, PACSIN2, SERHL, PIPPIN, EP300, FAM19A5 and/or GPR24 gene encoded polypeptide synthesis or expression, or abnormalities in the structure, temporal expression, and/or physical location of BRD1, NHP2L1, PACSIN2, SERHL, PIPPIN, EP300, FAM19A5 and GPR24 proteins. The antibodies and immunoassay methods described below have, for example, important in vitro applications in assessing the efficacy of treatments for the BRD1, NHP2L1, PACSIN2, SERHL, PIPPIN, EP300, FAM19A5 and GPR24 gene associated disorders , such as neuropsychiatric disorders, for example SCH and/or BDP. Antibodies, or fragments of antibodies, such as those described below, may be used to screen potentially therapeutic compounds in vitro to determine their effects on BRD1, NHP2L1, PACSIN2, SERHL, PIPPIN, EP300, FAM19A5 and/or GPR24 gene expression and BRD1, NHP2L1, PACSIN2, SERHL, PIPPIN, EP300, FAM19A5 and/or GPR24 protein production. The compounds that have beneficial effects on a BRD1, NHP2L1, PACSIN2, SERHL, PIPPIN, EP300, FAM19A5 and/or GPR24 gene disorder, such as SCH and BPD, can be identified, and a therapeutically effective dose determined.

In vitro immunoassays may also be used, for example, to assess the efficacy of cell-based gene therapy for a BRD1, NHP2L1, PACSIN2, SERHL, PIPPIN, EP300, FAM19A5 and/or GPR24 gene associated disorder, such as SCH and/or BPD. Antibodies directed against BRD1, NHP2L1, PACSIN2, SERHL, PIPPIN, EP300, FAM19A5 and/or GPR24 polypeptides may be used in vitro to determine, for example, the level of BRD1, NHP2L1, PACSIN2, SERHL, PIPPIN, EP300, FAM19A5 and/or GPR24 gene expression achieved in cells genetically engineered to produce BRD1, NHP2L1, PACSIN2, SERHL, PIPPIN, EP300, FAM19A5 and/or GPR24 polypeptides. In the case of intracellular BRD1, NHP2L1, PACSIN2, SERHL, PIPPIN, EP300, FAM19A5 and/or GPR24 gene products, including both transcriptional and translational products, such an assessment is done, preferably, using cell lysates or extracts. Such analysis will allow for a determination of the number of transformed cells necessary to achieve therapeutic efficacy in vivo, as well as optimization of the gene replacement protocol.

For the purpose of below discussion all molecules that produced due to activity of the BRD1, NHP2L1, PACSIN2, SERHL, PIPPIN, EP300, FAM19A5 and/or GPR24 genes, such as transcriptional and translational products of the genes, are termed herein "gene products", if not specified otherwise.

The tissue or cell type to be analyzed will generally include those that are known, or suspected, to express the BRD1, NHP2L1, PACSIN2, SERHL, PIPPIN, EP300, FAM19A5 and/or GPR24 genes. The protein isolation methods employed herein may, for example, be such as those described in Harlow and Lane (1988, "Antibodies: A Laboratory Manual", Cold Spring Harbor Laboratory Press, Cold Spring Harbor, New York). The isolated cells can be derived from cell culture or from a patient. The analysis of cells taken from culture may be a necessary step in the assessment of cells to be used as part of a cell-based gene therapy technique or, alternatively, to test the effect of compounds on expression of the BRD1, NHP2L1, PACSIN2, SERHL, PIPPIN, EP300, FAM19A5 and/or GPR24 genes.

Preferred diagnostic methods for the detection of BRD1, NHP2L1, PACSIN2, SERHL, PIPPIN, EP300, FAM19A5 and/or GPR24 gene products, such as BRD1, NHP2L1, PACSIN2, SERHL, PIPPIN, EP300, FAM19A5 and/or GPR24 proteins or conserved variants or peptide fragments thereof, may involve, for example, immunoassays wherein these products are detected by their interaction with an anti-BRD1, NHP2L1, PACSIN2, SERHL, PIPPIN, EP300, FAM19A5 and/or GPR24-specific antibody.

For example, antibodies, or fragments of antibodies, useful in the present invention may be used to quantitatively or qualitatively detect the presence of BRD1, NHP2L1, PACSIN2, SERHL, PIPPIN, EP300, FAM19A5 and/or GPR24 proteins or conserved variants or peptide fragments thereof. This can be accomplished, for example, by immunofluorescence techniques employing a fluorescently labeled antibody coupled with light microscopic, flow cytometric, or fluorimetric detection. Such techniques are especially preferred for the proteins that are expressed on the cell surface.

The antibodies, or fragments thereof, useful in the present invention may, additionally, be employed histologically, as in immunofluorescence or immunoelectron microscopy, for in situ detection of the BRD1, NHP2L1, PACSIN2, SERHL, PIPPIN, EP300, FAM19A5 and/or GPR24 gene derived polypeptide products or conserved variants or peptide fragments thereof. In situ detection may be accomplished by removing a histological specimen from a patient, and applying thereto a labelled antibody of the present invention. The antibody (or fragment) is preferably applied by overlaying the labeled antibody (or fragment) onto a biological sample. Through the use of such a procedure, it is possible to determine not only the presence of BRD1, NHP2L1, PACSIN2, SERHL, PIPPIN, EP300, FAM19A5 and/or GPR24 protein or conserved variants or peptide fragments, but also its distribution in the examined tissue. Using the present invention, those of ordinary skill will readily perceive that any of a wide variety of histological methods (such as staining procedures) can be modified in order to achieve such in situ detection.

Immunoassays for BRD1, NHP2L1, PACSIN2, SERHL, PIPPIN, EP300, FAM19A5 and/or GPR24 proteins or conserved variants or peptide fragments thereof will typically comprise incubating a sample, such as a biological fluid, a tissue extract, freshly harvested cells, or lysates of cells, that have been incubated in cell culture, in the presence of a detectably labeled antibody capable of identifying BRD1, NHP2L1, PACSIN2, SERHL, PIPPIN, EP300, FAM19A5 and/or GPR24 proteins or conserved variants or peptide fragments thereof, and detecting the bound antibody by any of a number of techniques well-known in the art.

The biological sample may be brought in contact with and immobilized onto a solid phase support or carrier such as nitrocellulose, or other solid support that is capable of immobilizing cells, cell particles or soluble proteins. The support may then be washed with suitable buffers followed by treatment with the detectably labeled BRD1, NHP2L1, PACSIN2, SERHL, PIPPIN, EP300, FAM19A5 and/or GPR24 protein specific antibody. The solid phase support may then be washed with the buffer a second time to remove unbound antibody. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of an anti- BRD1, NHP2L1, PACSIN2, SERHL, PIPPIN, EP300, FAM19A5 and/or GPR24 antibody may be determined according to well known methods. Those skilled in the art will be able to determine operative and optimal assay conditions for each determination by employing routine experimentation.

One of the ways in which an BRD1, NHP2L1, PACSIN2, SERHL, PIPPIN, EP300, FAM19A5 and/or GPR24-specific antibody can be detectably labeled is by linking the same to an enzyme and use in an enzyme immunoassay (EIA) (Voller, A., "The Enzyme Linked Immunosorbent Assay (ELISA)", 1978, Diagnostic Horizons 2, 1-7 and 94, Microbiological Associates Quarterly Publication, Walkersville, Md.); Voller, A. et al., 1978, J. Clin. Pathol. 31, 507-520; Butler, J. E., 1981, Meth. Enzymol. 73, 482-523; Maggio, E. (ed.), 1980, Enzyme Immunoassay, CRC Press, Boca Raton, Fla.,; Ishikawa, E. et al., (eds.), 1981, Enzyme Immunoassay, Kgaku Shoin, Tokyo). The enzyme which is bound to the antibody will react with an appropriate substrate, preferably a chromogenic substrate, in such a manner as to produce a chemical moiety that can be detected, for example, by spectrophotometric, fluorimetric or by visual means. Enzymes that can be used to detectably label the antibody include, but are not limited to, malate dehydrogenase, staphylococcal nuclease, delta-5-steroid isomerase, yeast alcohol dehydrogenase, .alpha.-glycerophosphate, dehydrogenase, triose phosphate isomerase, horseradish peroxidase, alkaline phosphatase, asparaginase, glucose oxidase, .beta.-galactosidase, ribonuclease, urease, catalase, glucose-6-phosphate dehydrogenase, glucoamylase and acetylcholinesterase. The detection can be accomplished by colorimetric methods that employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labelling the antibodies or antibody fragments, it is possible to detect the BRD1, NHP2L1, PACSIN2, SERHL, PIPPIN, EP300, FAM19A5 and/or GPR24 polypeptides through the use of a radioimmunoassay (RIA) (see, for example, Weintraub, B., Principles of Radioimmunoassays, Seventh Training Course on Radioligand Assay Techniques, The Endocrine Society, March, 1986). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as .sup.152 Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase, green fluorescent protein and aequorin.

### Biological sample

The biological sample used in the present invention may be any suitable biological sample comprising genetic material and/or proteins. In a preferred embodiment the sample is a blood sample, a tissue sample, a secretion sample, semen, ovum, hairs, nails, tears, and urine. The most convenient sample type is a blood sample.

### Kits

In one aspect there is provided a kit for predicting the risk of a subject for developing mental diseases, such as SCH and/or BPD or for other diagnostic and classification purposes of mental diseases such as SCH and/or BPD comprising at least one probe comprising a nucleic acid sequence as defined in the previous section.

In one embodiment the probe is linked to a detectable label.

In another embodiment based on single nucleotide extension the kit further comprises at least one nucleotide monomer labelled with a detectable label, a polymerase and suitable buffers and reagents.

The kit preferably also comprises set of primers for amplifying a region comprising at least one of the identified above polymorphisms in any of the genes selected from the BRD1, NHP2L1, PACSIN2, SERHL, PIPPIN, EP300, FAM19A5 and GPR24 genes or transcriptional products of said genes, or the corresponding complementary strands. The primers preferably are at least 15 bases long and may be coupled to an entity suitable for subsequent immobilisation.

In another embodiment a diagnostic kit of the invention may comprise an antibody as described above.

### Diseases

According to the invention the association of an SNP with a particular disease means the association a particular allele at the position of said SNP with a predisposition to said disease or with a protection against said disease. Non-limited examples of the protective/susceptibility alleles of the SNP having the positions as of Table 1 are given in Table 6 below.

**Table 6**

| **Gene** | **SEQ ID NO** | **SNP** | **SNP ID** | **Allele** | |
|---|---|---|---|---|---|
| | | | | **protective** | **susceptib ility** |
| **NHP2L1** | 1 | A/G | rs11561 | A | G in SCH |
| **PACSIN2** | 2 | C/T | rs2068943 | T | C in SCH |
| **SERHL** | 3 | C/G | rs881542 | G | C in SCH |
| | 3 | A/G | rs926333 | G | A in SCH |
| **BRD1** | 5 | T/C | rs4468 | T | C in SCH |
| | 5 | A/C | rs138880 | A | C in SCH |
| **EP300** | 6 | A/G | rs20551 | G | A in BPD |
| | 6 | A/C | rs1046088 | C | A in BPD |

According to the invention individuals carrying the protective alleles at positions identified herein are less likely to develop a mental disease of the invention. In contrary, the presence of the susceptibility allele is indicative of a predisposition to a mental disease of the invention.

Thus, in one embodiment the invention relates to a method for determining a predisposition of an individual to a mental disease, in particular SCH and/BDP,, said method comprising determining the presence in a biological sample from said individual the susceptibility allele of at least one SNP selected from the SNPs identified above as rs11561, rs5758405 rs8779, rs132806, rs2068943, rs2267487, rs881542, rs926333, rs1060387, rs1006407, rs4468, rs138855, rs2239848, rs138880, rs138881, rs20551, rs2294976, rs2076578, rs1046088, rs133068, rs133069, rs133070, rs133073, rs6002408.

In another embodiment the invention relates to a method for determining a predisposition of an individual to a mental disease, in particular SCH and/BDP, said method comprising determining the presence in a biological sample from said individual the susceptibility allele of two or more of the SNPs identified above as rs11561, rs5758405 rs8779, rs132806, rs2068943, rs2267487, rs881542, rs926333, rs1060387, rs1006407, rs4468, rs138855, rs2239848, rs138880, rs138881, rs20551, rs2294976, rs2076578, rs1046088, rs133068, rs133069, rs133070, rs133073, rs6002408.

In still another embodiment, the invention relates to a method for determining a predisposition of an individual to a mental disease, in particular SCH and/BDP, said method comprising determining the presence in a biological sample from said individual the susceptibility allele of one or more SNPs of the identified above as rs11561, rs5758405 rs8779, rs132806, rs2068943, rs2267487, rs881542, rs926333, rs1060387, rs1006407, rs4468, rs138855, rs2239848, rs138880, rs138881, rs20551, rs2294976, rs2076578, rs1046088, rs133068, rs133069, rs133070, rs133073, rs6002408 and the presence at least one of the SNPs of the identified above as rs909660, rs710193, rs1573745, rs132234, rs3752466, rs6010260, rs137931, rs137932, rs3810971, rs2272843, rs1063900, rs715519, rs916005.

In yet another embodiment, the invention relates to a method for determining a predisposition of an individual to a mental disease, in particular SCH and/BDP, said method comprising determining the presence in a biological sample from said individual one or more SNPs selected from the SNPs identified above rs909660, rs710193, rs1573745, rs132234, rs3752466, rs6010260, rs137931, rs137932, rs3810971, rs2272843, rs1063900, rs715519, rs916005.

In yet another embodiment, the invention relates to a method for determining a predisposition of an individual to not having a mental disease, in particular SCH and/BDP, said method comprising determining the presence in a biological sample from said individual the protective allele of one or more SNPs selected from the SNPs identified above as rs11561, rs5758405 rs8779, rs132806, rs2068943, rs2267487, rs881542, rs926333, rs1060387, rs1006407, rs4468, rs138855, rs2239848, rs138880, rs138881, rs20551, rs2294976, rs2076578, rs1046088, rs133068, rs133069, rs133070, rs133073, rs6002408.

In some embodiments the method for determining a predisposition/no predisposition to a mental disease of the invention, in particular SCH and/or BDP, may concern determining two or more of the above described SNPs

**Screening for new candidate drugs for therapeutic treatment SCH and/or BPD** Screening methods for compounds with are capable of modulating the BRD1, NHP2L1, PACSIN2, SERHL, PIPPIN, EP300, FAM19A5 and/or GPR24 protein-protein interactions are within the scope of the invention.

For the purpose of below discussion molecules that produced in the cells due to activity of the BRD1, NHP2L1, PACSIN2, SERHL, PIPPIN, EP300, FAM19A5 and/or GPR24 genes, such as transcriptional and translational products of the genes, are termed herein "gene products", if not specified otherwise.

Any method suitable for detecting protein-protein interactions may be employed for identifying the BRD1, NHP2L1, PACSIN2, SERHL, PIPPIN, EP300, FAM19A5 and/or GPR24 protein-protein interactions.

Among the traditional methods that may be employed are co-immunoprecipitation, cross-linking and co-purification through gradients or chromatographic columns. Utilizing procedures such as these allows for the identification of proteins, including intracellular proteins, which interact with BRD1, NHP2L1, PACSIN2, SERHL, PIPPIN, EP300, FAM19A5 and/or GPR24 proteins. Once isolated, such a protein can be identified and can be used in conjunction with standard techniques, to identify proteins it interacts with. For example, at least a portion of the amino acid sequence of a protein that interacts with BRD1, NHP2L1, PACSIN2, SERHL, PIPPIN, EP300, FAM19A5 and/or GPR24 protein can be ascertained using techniques well known to those of skill in the art, such as via the Edman degradation technique (see, e.g., Creighton, 1983, "Proteins: Structures and Molecular Principles," W.H. Freeman & Co., N.Y., pp.34-49). The amino acid sequence obtained may be used as a guide for the generation of oligonucleotide mixtures that can be used to screen for gene sequences encoding such proteins. Screening made be accomplished, for example, by standard hybridization or PCR techniques. Techniques for the generation of oligonucleotide mixtures and the screening are well-known. (See, e.g., Ausubel, supra, and 1990, "PCR Protocols: A Guide to Methods and Applications," Innis, et al., eds. Academic Press, Inc., New York).

Additionally, methods may be employed that result in the simultaneous identification of genes that encode a protein which interacts with BRD1, NHP2L1, PACSIN2, SERHL, PIPPIN, EP300, FAM19A5 and/or GPR24 protein. These methods include, for example, probing expression libraries with labelled BRD1, NHP2L1, PACSIN2, SERHL, PIPPIN, EP300, FAM19A5 and/or GPR24 polypeptides, using BRD1, NHP2L1, PACSIN2, SERHL, PIPPIN, EP300, FAM19A5 and/or GPR24 proteins in a manner similar to the well known technique of antibody probing of lambda.gtll and lambda.gt10 libraries.

One method that detects protein interactions in vivo, the two-hybrid system, is described in detail for illustration only and not by way of limitation. One version of this system has been described (Chien, et al., 1991, Proc. Natl. Acad. Sci. USA, 88, 9578-9582) and is commercially available from Clontech (Palo Alto, Calif.).

Briefly, utilizing such a system, plasmids are constructed that encode two hybrid proteins: one consists of the DNA-binding domain of a transcription activator protein fused to the BRD1, NHP2L1, PACSIN2, SERHL, PIPPIN, EP300, FAM19A5 and/or GPR24 gene peptide product and the other consists of the transcription activator protein's activation domain fused to an unknown protein that is encoded by a cDNA that has been recombined into this plasmid as part of a cDNA library. The DNA-binding domain fusion plasmid and the cDNA library are transformed into a strain of the yeast Saccharomyces cerevisiae that contains a reporter gene (e.g., HBS or lacZ) whose regulatory region contains the transcription activator's binding site. Either hybrid protein alone cannot activate transcription of the reporter gene: the DNA-binding domain hybrid cannot because it does not provide activation function and the activation domain hybrid cannot because it cannot localize to the activator's binding sites. Interaction of the two hybrid proteins reconstitutes the functional activator protein and results in expression of the reporter gene, which is detected by an assay for the reporter gene product.

The two-hybrid system or related methodology may be used to screen activation domain libraries for proteins that interact with the "bait" gene product. By way of example, and not by way of limitation, BRD1, NHP2L1, PACSIN2, SERHL, PIPPIN, EP300, FAM19A5 and/or GPR24 gene derived peptide products may be used as the bait gene product. Total genomic or cDNA sequences are fused to the DNA encoding an activation domain. This library and a plasmid encoding a hybrid of a bait BRD1, NHP2L1, PACSIN2, SERHL, PIPPIN, EP300, FAM19A5 and/or GPR24 protein, or a fragment thereof, fused to the DNA-binding domain are co-transformed into a yeast reporter strain, and the resulting transformants are screened for those that express the reporter gene. For example, and not by way of limitation, a bait BRD1, NHP2L1, PACSIN2, SERHL, PIPPIN, EP300, FAM19A5 and/or GPR24 gene sequence, such as the open reading frame of the BRD1, NHP2L1, PACSIN2, SERHL, PIPPIN, EP300, FAM19A5 and/or GPR24 gene, can be cloned into a vector such that it is translationally fused to the DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait BRD1, NHP2L1, PACSIN2, SERHL, PIPPIN, EP300, FAM19A5 and/ GPR24 gene product are to be detected can be made using methods routinely practiced in the art. According to the particular system described herein, for example, the cDNA fragments can be inserted into a vector such that they are translationally fused to the transcriptional activation domain of GAL4. This library can be co-transformed along with the bait BRD1, NHP2L1, PACSIN2, SERHL, PIPPIN, EP300, FAM19A5 and/or GPR24 gene sequence-GAL4 fusion plasmid into a yeast strain that contains a lacZ gene driven by a promoter that contains GAL4 activation sequence. A cDNA encoded protein, fused to GAL4 transcriptional activation domain, that interacts with bait BRD1, NHP2L1, PACSIN2, SERHL, PIPPIN, EP300, FAM19A5 and/or GPR24 gene product will reconstitute an active GAL4 protein and thereby drive expression of the HIS3 gene. Colonies that express HIS3 can be detected by their growth on petri dishes containing semi-solid agar based media lacking histidine. The cDNA can then be purified from these strains, and used to produce and isolate the bait BRD1, NHP2L1, PACSIN2, SERHL, PIPPIN, EP300, FAM19A5 and/or GPR24 protein-interacting protein using techniques routinely practiced in the art.

The invention also related to screening assays for compounds that interfere with the BRD1, NHP2L1, PACSIN2, SERHL, PIPPIN, EP300, FAM19A5 and/or GPR24 gene products macromolecule interaction.

The BRD1, NHP2L1, PACSIN2, SERHL, PIPPIN, EP300, FAM19A5 and/or GPR24 gene products of the invention may, in vivo, interact with one or more macromolecules, including intracellular macromolecules, such as proteins. Such macromolecules may include, but are not limited to, nucleic acid molecules and those proteins identified via methods such as those described above. For purposes of this discussion, the macromolecules are referred to herein as "binding partners". Compounds that are able to disrupt the BRD1, NHP2L1, PACSIN2, SERHL, PIPPIN, EP300, FAM19A5 and GPR24 gene products binding in this way may be useful in regulating the activity of products of the BRD1, NHP2L1, PACSIN2, SERHL, PIPPIN, EP300, FAM19A5 and GPR24 genes, especially variant BRD1, NHP2L1, PACSIN2, SERHL, PIPPIN, EP300, FAM19A5 and GPR24 proteins and thereof derived peptide products. Such compounds may include, but are not limited to molecules such as peptides, and the like, which would be capable of gaining access to a BRD1, NHP2L1, PACSIN2, SERHL, PIPPIN, EP300, FAM19A5 and/or GPR24 gene product.

The basic principle of the assay systems used to identify compounds that interfere with the interaction between BRD1, NHP2L1, PACSIN2, SERHL, PIPPIN, EP300, FAM19A5 and GPR24 gene products and their binding partner or partners involves preparing a reaction mixture containing the BRD1, NHP2L1, PACSIN2, SERHL, PIPPIN, EP300, FAM19A5 and/or GPR24 gene product, and the binding partner under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of BRD1, NHP2L1, PACSIN2, SERHL, PIPPIN, EP300, FAM19A5 and/or GPR24 gene product and its binding partner. Control reaction mixtures are incubated without the test compound or with a placebo. The formation of any complexes between the BRD1, NHP2L1, PACSIN2, SERHL, PIPPIN, EP300, FAM19A5 and/or GPR24 gene product and the binding partner is then detected. The formation of a complex in the control reaction, but not in the reaction mixture containing the test compound, indicates that the compound interferes with the interaction of the BRD1, NHP2L1, PACSIN2, SERHL, PIPPIN, EP300, FAM19A5 and/or GPR24 gene product and the interactive binding partner. Additionally, complex formation within reaction mixtures containing the test compound and for example normal (wild type) BRD1, NHP2L1, PACSlN2, SERHL, PIPPIN, EP300, FAM19A5 and/or GPR24 protein may also be compared to complex formation within reaction mixtures containing the test compound and a variant BRD1, NHP2L1, PACSIN2, SERHL, PIPPIN, EP300, FAM19A5 and/or GPR24 protein. This comparison may be important in those cases wherein it is desirable to identify compounds that disrupt interactions of mutant but not wild type BRD1, NHP2L1, PACSIN2, SERHL, PIPPIN, EP300, FAM19A5 and/or GPR24 protein.

The assay for compounds that interfere with the interaction of BRD1, NHP2L1, PACSIN2, SERHL, PIPPIN, EP300, FAM19A5 and/ GPR24 gene products and their binding partners can be conducted in a heterogeneous or homogeneous format. Heterogeneous assays involve anchoring either the BRD1, NHP2L1, PACSIN2, SERHL, PIPPIN, EP300, FAM19A5 and/or GPR24 gene product or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the BRD1, NHP2L1, PACSlN2, SERHL, PIPPIN, EP300, FAM19A5 and/or GPR24 gene products and the binding partners, e.g., by competition, can be identified by conducting the reaction in the presence of the test substance; i.e., by adding the test substance to the reaction mixture prior to or simultaneously with the BRD1, NHP2L1, PACSlN2, SERHL, PIPPIN, EP300, FAM19A5 and/or GPR24 gene protein and interactive intracellular binding partner. Alternatively, test compounds that disrupt preformed complexes, e.g., compounds with higher binding constants that displace one of the components from the complex, can be tested by adding the test compound to the reaction mixture after complexes have been formed. The various formats are described briefly below.

In a heterogeneous assay system, either the BRD1, NHP2L1, PACSIN2, SERHL, PIPPIN, EP300, FAM19A5 and/or GPR24 gene product or the interactive binding partner, is anchored onto a solid surface, while the non-anchored species is labeled, either directly or indirectly. In practice, microtiter plates are conveniently utilized. The anchored species may be immobilized by non-covalent or covalent attachments. Non-covalent attachment may be accomplished simply by coating the solid surface with a solution of the BRD1, NHP2L1, PACSIN2, SERHL, PIPPIN, EP300, FAM19A5 and/or GPR24 gene product or binding partner and drying. Alternatively, an immobilized antibody specific for the species to be anchored may be used to anchor the species to the solid surface. The surfaces may be prepared in advance and stored.

In order to conduct the assay, the partner of the immobilized species is exposed to the coated surface with or without the test compound. After the reaction is complete, unreacted components are removed (e.g., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labelled, an indirect label can be used to detect complexes anchored on the surface; e.g., using a labeled antibody specific for the initially non-immobilized species (the antibody, in turn, may be directly labeled or indirectly labeled with a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. In this approach, a preformed complex of a BRD1, NHP2L1, PACSIN2, SERHL, PIPPIN, EP300, FAM19A5 and/or GPR24 gene product and the interactive binding partner is prepared in which either the BRD1, NHP2L1, PACSIN2, SERHL, PIPPIN, EP300, FAM19A5 and/or GPR24 gene product or its binding partners is labeled, but the signal generated by the label is quenched due to complex formation (see, e.g., U.S. Pat. No.4,109,496 by Rubenstein which utilizes this approach for immunoassays). The addition of a test substance that competes with and displaces one of the species from the preformed complex will result in the generation of a signal above background. In this way, test substances that disrupt the BRD1, NHP2L1, PACSlN2, SERHL, PIPPIN, EP300, FAM19A5 and GPR24 gene product/binding partner interaction can be identified.

In another embodiment, the BRD1, NHP2L1, PACSIN2, SERHL, PIPPIN, EP300, FAM19A5 and/or GPR24 gene product can be prepared for immobilization using recombinant DNA techniques. For example, the BRD1, NHP2L1, PACSIN2, SERHL, PIPPIN, EP300, FAM19A5 and/or GPR24 gene coding region can be fused to the glutathione-S-transferase (GST) gene using a fusion vector, such as pGEX-5X-1, in such a manner that its binding activity is maintained in the resulting fusion protein. The interactive binding partner can be purified and used to raise an antibody, using methods routinely practiced in the art. The antibody can then be labeled with a radioactive isotope such as .sup.125 l, for example, by methods routinely practiced in the art. In a heterogeneous assay, e.g., the GST- BRD1, NHP2L1, PACSIN2, SERHL, PIPPIN, EP300, FAM19A5 and/or GPR24 fusion protein can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between the BRD1, NHP2L1, PACSIN2, SERHL, PIPPIN, EP300, FAM19A5 and/or GPR24 gene product and the interactive binding partner can be detected by measuring the amount of radioactivity that remains associated with the glutathione-agarose beads. A successful inhibition of the interaction by the test compound will result in a decrease in measured radioactivity.

Alternatively, the GST-BRD1, NHP2L1, PACSIN2, SERHL, PIPPIN, EP300, FAM19A5 and/or GPR24 fusion protein and the interactive binding partner can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the BRD1, NHP2L1, PACSIN2, SERHL, PIPPIN, EP300, FAM19A5 and/or GPR24 gene product/binding partner interaction can be detected by adding the labelled antibody and measuring the radioactivity associated with the beads.

In still another embodiment of the invention, these same techniques can be employed using peptide fragments that correspond to the binding domains of BRD1, NHP2L1, PACSIN2, SERHL, PIPPIN, EP300, FAM19A5 and/or GPR24 proteins and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensating mutations in the gene encoding the second species in the complex can then be selected. Sequence analysis of the genes encoding the respective proteins will reveal the mutations that correspond to the region of the protein involved in interactive binding. Alternatively, one protein can be anchored to a solid surface using methods described in this Section above, and allowed to interact with and bind to its labeled binding partner, which has been treated with a proteolytic enzyme, such as trypsin. After washing, a short, labelled peptide comprising the binding domain may remain associated with the solid material, which can be isolated and identified by amino acid sequencing. Also, once the gene coding for the segments can be engineered to express peptide fragments of the protein, which can then be tested for binding activity and purified or synthesized.

For example, and not by way of limitation, a BRD1, NHP2L1, PACSIN2, SERHL, PIPPIN, EP300, FAM19A5 and/or GPR24 gene product can be anchored to a solid material as described above by making a GST- BRD1, NHP2L1, PACSIN2, SERHL, PIPPIN, EP300, FAM19A5 and/or GPR24 fusion protein and allowing it to bind to glutathione agarose beads. The interactive binding partner obtained can be labeled with a radioactive isotope, such as³⁵ S, and cleaved with a proteolytic enzyme such as trypsin. Cleavage products can then be added to the anchored GST-BRD1, NHP2L1, PACSIN2, SERHL, PIPPIN, EP300, FAM19A5 and/or GPR24 fusion protein and allowed to bind. After washing away unbound peptides, labelled bound material, representing the binding partner binding domain, can be eluted, purified, and analyzed for amino acid sequence by well-known methods. Peptides so identified can be produced synthetically or fused to appropriate facilitative proteins using recombinant DNA technology.

The invention also provides assays for identification of compounds that ameliorate the BRD1, NHP2L1, PACSIN2, SERHL, PIPPIN, EP300, FAM19A5 and GPR24 gene associated disorders, such as SCH and/or BPD.

Compounds, including but not limited to binding compounds identified via assay techniques such as those described above can be tested for the ability to ameliorate symptoms of a BRD1, NHP2L1, PACSIN2, SERHL, PIPPIN, EP300, FAM19A5 and/or GPR24 gene associated disorder including a disorder of thought and/or mood, neuropsychiatric disorders including bipolar (BPD), genetically related unipolar affective disorders, delusional disorders, paraphrenia, paranoid psychosis, SCH, schizotypal disorder, schizoaffective disorder, schizoaffective bipolar and genetically related unipolar affective disorders, psychogenic psychosis, catatonia, periodic bipolar and genetically related unipolar affective disorders, cycloid psychosis, schizoid personality disorder, paranoid personality disorder, bipolar and genetically related unipolar affective disorders related affective disorders and subtypes of unipolar affective disorder.

It should be noted that the assays described herein can identify compounds that affect the BRD1, NHP2L1, PACSlN2, SERHL, PIPPIN, EP300, FAM19A5 and/or GPR24 gene activity by either affecting BRD1, NHP2L1, PACSIN2, SERHL, PIPPIN, EP300, FAM19A5 and GPR24 gene expression or by affecting the level of BRD1, NHP2L1, PACSIN2, SERHL, PIPPIN, EP300, FAM19A5 and GPR24 gene product activity. For example, compounds may be identified that are involved in another step in the pathway in which the BRD1, NHP2L1, PACSlN2, SERHL, PIPPIN, EP300, FAM19A5 and/or GPR24 gene and/or the BRD1, NHP2L1, PACSIN2, SERHL, PIPPIN, EP300, FAM19A5 and/or GPR24 gene product is involved and, by affecting this same pathway may modulate the effect of the BRD1, NHP2L1, PACSIN2, SERHL, PIPPIN, EP300, FAM19A5 and/or GPR24 gene on the development of a neuropsychiatric disorder such as SCH and/or BPD. Such compounds can be used as part of a therapeutic method for the treatment of the disorder.

Described below are cell-based and animal model-based assays for the identification of compounds exhibiting such an ability to ameliorate symptoms of the BRD1, NHP2L1, PACSIN2, SERHL, PIPPIN, EP300, FAM19A5 and GPR24 gene activity associated with a neuropsychiatric disorder, such SCH and/or BPD.

First, cell-based systems can be used to identify compounds that may act to ameliorate symptoms of a BRD1, NHP2L1, PACSIN2, SERHL, PIPPIN, EP300, FAM19A5 and/or GPR24 gene associated disorder, such as BPD and/or SCH. Such cell systems can include, for example, recombinant or non-recombinant cell, such as cell lines, that express the BRD1, NHP2L1, PACSIN2, SERHL, PIPPIN, EP300, FAM19A5 and/or GPR24 gene.

In utilizing such cell systems, cells that express the BRD1, NHP2L1, PACSIN2, SERHL, PIPPIN, EP300, FAM19A5 and/or GPR24 gene may be exposed to a compound suspected of exhibiting an ability to ameliorate symptoms of a BRD1, NHP2L1, PACSIN2, SERHL, PIPPIN, EP300, FAM19A5 and/or GPR24 gene disorder, such as SCH and/or BPD, at a sufficient concentration and for a sufficient time to elicit such an amelioration of such symptoms in the exposed cells. After exposure, the cells can be assayed to measure alterations in the expression of the BRD1, NHP2L1, PACSIN2, SERHL, PIPPIN, EP300, FAM19A5 and/or GPR24 gene, e.g., by assaying cell lysates for the presence of BRD1, NHP2L1, PACSIN2, SERHL, PIPPIN, EP300, FAM19A5 and/or GPR24 gene transcripts (e.g., by Northern analysis) or for the BRD1, NHP2L1, PACSIN2, SERHL, PIPPIN, EP300, FAM19A5 and GPR24 gene translation products expressed by the cell. Compounds that modulate expression of the BRD1, NHP2L1, PACSIN2, SERHL, PIPPIN, EP300, FAM19A5 and/or GPR24 gene are considered to be good candidates as therapeutics.

Alternatively, the cells are examined to determine whether one or more cellular phenotypes associated with a BRD1, NHP2L1, PACSIN2, SERHL, PIPPIN, EP300, FAM19A5 and/or GPR24 gene disorder, such as SCH and/or BPD, has been altered to resemble a more normal or unimpaired, unaffected phenotype, or a phenotype more likely to produce a lower incidence or severity of disorder symptoms.

In addition, animal-based systems or models for a BRD1, NHP2L1, PACSIN2, SERHL, PIPPIN, EP300, FAM19A5 and/or GPR24 gene associated disorder, such as SCH and/or BPD, which may include, for example mice, may be used to identify compounds capable of ameliorating symptoms of the disorder. Such animal models may be used as test substrates for the identification of drugs, pharmaceuticals, therapies and interventions that may be effective in treating such disorders. For example, animal models may be exposed to a compound suspected of exhibiting an ability to ameliorate symptoms, at a sufficient concentration and for a sufficient time to elicit such an amelioration of symptoms of a BRD1, NHP2L1, PACSIN2, SERHL, PIPPIN, EP300, FAM19A5 and/or GPR24 gene associated disorder, such as SCH and/or BPD, in the exposed animals. The response of the animals to the exposure may be monitored by assessing the reversal of such symptoms.

With regard to intervention, any treatments that reverse any aspect of symptoms of a BRD1, NHP2L1, PACSIN2, SERHL, PIPPIN, EP300, FAM19A5 and/or GPR24 gene associated disorder, such as SCH and/or BPD, should be considered as candidates for human therapeutic intervention in such a disorder.

### Therapeutic treatment

The present invention relates to a BRD1, NHP2L1, PACSIN2, SERHL, PIPPIN, EP300, FAM19A5 and/or GPR24 gene associated disorder including a disorder of thought and/or mood, neuropsychiatric disorders including bipolar (BPD), genetically related unipolar affective disorders, delusional disorders, paraphrenia, paranoid psychosis, SCH, schizotypal disorder, schizoaffective disorder, schizoaffective bipolar and genetically related unipolar affective disorders, psychogenic psychosis, catatonia, periodic bipolar and genetically related unipolar affective disorders, cycloid psychosis, schizoid personality disorder, paranoid personality disorder, bipolar and genetically related unipolar affective disorders related affective disorders and subtypes of unipolar affective disorder.

Having identified a group of subjects having a polymorphism as described in the present invention, the invention also relates to the use of compounds directed to decreasing or modulating the effect of the polymorphism for the preparation of a medicament for the treatment of SCH and/or BPD in said subjects.

The compounds that bind to a BRD1, NHP2L1, PACSIN2, SERHL, PIPPIN, EP300, FAM19A5 and/or GPR24 gene product, intracellular proteins or portions of proteins that interact with a BRD1, NHP2L1, PACSIN2, SERHL, PIPPIN, EP300, FAM19A5 and/or GPR24 gene product, compounds that interfere with the interaction of a BRD1, NHP2L1, PACSIN2, SERHL, PIPPIN, EP300, FAM19A5 and/or GPR24 gene product with intracellular proteins and compounds that modulate the activity of the BRD1, NHP2L1, PACSIN2, SERHL, PIPPIN, EP300, FAM19A5 and GPR24 genes (i.e., modulate the level of the BRD1, NHP2L1, PACSlN2, SERHL, PIPPIN, EP300, FAM19A5 and GPR24 gene expression and/or modulate the level of the BRD1, NHP2L1, PACSIN2, SERHL, PIPPIN, EP300, FAM19A5 and GPR24 gene product activity) are considered to be good candidates for the manufacture of a medicament for treatment of a BRD1, NHP2L1, PACSIN2, SERHL, PIPPIN, EP300, FAM19A5 and/or GPR24 gene associated disorder. Assays may additionally be utilized that identify compounds that bind to the BRD1, NHP2L1, PACSIN2, SERHL, PIPPIN, EP300, FAM19A5 and/or GPR24 gene regulatory sequences (e.g., promoter sequences; see e.g., Platt, 1994, J. Biol. Chem. 269, 28558-28562), and that may modulate the level of BRD1, NHP2L1, PACSIN2, SERHL, PIPPIN, EP300, FAM19A5 and/or GPR24 gene expression. Compounds may include, but are not limited to, small organic molecules, such as ones that are able to cross the blood-brain barrier, gain entry into an appropriate cell and affect expression of the BRD1, NHP2L1, PACSlN2, SERHL, PIPPIN, EP300, FAM19A5 and/or GPR24 gene or some other gene involved in a BRD1, NHP2L1, PACSIN2, SERHL, PIPPIN, EP300, FAM19A5 and/or GPR24 gene dependent regulatory pathway, or intracellular proteins. Such intracellular proteins may for example be involved in the control and/or regulation of mood. Further, among these compounds are compounds that affect the level of BRD1, NHP2L1, PACSIN2, SERHL, PIPPIN, EP300, FAM19A5 and/or GPR24 gene expression and/or the BRD1, NHP2L1, PACSIN2, SERHL, PIPPIN, EP300, FAM19A5 and/or GPR24 gene product activity and that can be used as medicaments in the therapeutic treatment of the BRD1, NHP2L1, PACSIN2, SERHL, PIPPIN, EP300, FAM19A5 and/or GPR24 gene associated disorders, for example neuropsychiatric disorders such as SCH and/or BPD.

Compounds may include, but are not limited to, peptides such as, for example, soluble peptides, including but not limited to, lg-tailed fusion peptides, and members of random peptide libraries; (see, e.g., Lam, et al., 1991, Nature 354, 82-84; Houghten, et al., 1991, Nature 354, 84-86), and combinatorial chemistry-derived molecular library made of D- and/or L- configuration amino acids, phosphopeptides (including, but not limited to members of random or partially degenerate, directed phosphopeptide libraries; see, e.g., Songyang, et al., 1993, Cell 72, 767-778), antibodies (including, but not limited to, polyclonal, monoclonal, humanized, anti-idiotypic, chimeric or single chain antibodies, and FAb, F(ab').sub.2 and Fab expression library fragments, and epitope-binding fragments thereof), and small organic or inorganic molecules. Such compounds may further comprise compounds, in particular drugs or members of classes or families of drugs, known to ameliorate or exacerbate the symptoms of a neuropsychiatric disorder such as bipolar and genetically related unipolar affective disorders with the use of lithium salts, atypical antipsychotics such as ziprasadone, risperidone, clozapine, quetiapine, olanzapine, butyrophenone derivatives such as haloperidol and droperidol, phenothiazaine derivatives such as chlorpromazine, prochloperazine, promazine, trifluopromazine, thioxanthine derivatives such as flupenthixol, chlorprothixene and dibenzodiazepines and antipsychotic antiepileptic drugs such as carbamazepine, and valproic acid. Antidepressant drugs such imipramine, amitryptiline, nortryptiline, prothiaden, doxapine, other tricyclic antidepressants, tetracyclic antidepressants, serotonin reuptake inhibitor antidepressants such as fluoxetine, paroxetine, cipromil, venlafaxine, monoamine oxidase inhibitor antidepressants such as phenelzine, tranylcypromine, isocaboxazid, selegiline, and moclobamide. In addition psychotogenic drugs such as bromocriptine, apomorphine, amphetamine, methylphenidate, methylamphetaime, ketamine. Many of these drugs can be or have been used in combination.

*Compounds identified via assays such* as *those described herein may be useful, for example, in elaborating the biological function of the BRD1, NHP2L1, PACSIN2, SERHL, PIPPIN, EP300, FAM19A5 and GPR24 gene products, and for ameliorating the BRD1, NHP2L1, PACSIN2, SERHL, PIPPIN, EP300, FAM19A5 and GPR24 gene associated disorders or neuropsychiatric disorders, such* as *for example SCH and*/*or BPD.*

In one embodiment of treatment methods, the compounds administered do not comprise compounds, in particular drugs, reported to ameliorate or exacerbate the symptoms of a neuropsychiatric disorder, such as bipolar and genetically related unipolar affective disorders. Such compounds include antidepressants such as lithium salts, flupenthixol, risperidone, clozapine, quetiapine, olanzapine, haloperidol, droperidol, chlorpromazine, prochloperazine, phenothiazaine derivatives, promazine, trifluopromazine, butyrophenone derivatives, thioxanthine derivatives such as chlorprothixene and dibenzodiazepines and antipsychotic antiepileptic drugs such carbamazepine, and valproic acid, reserpine. Psychotogenic drugs such as LSD, bromocriptine, apomorphine, amphetamine, methylphenidate, methylamphetaime, ketamine, Many of these drugs are used in combination.

### 1. Inhibitory antisense, ribozyme and triple helix approaches

In another embodiment, symptoms of certain neuropsychiatric disorders, such as SCH and/or BPD, may be ameliorated by decreasing the level of BRD1, NHP2L1, PACSIN2, SERHL, PIPPIN, EP300, FAM19A5 and/or GPR24 gene expression and/or the BRD1, NHP2L1, PACSIN2, SERHL, PIPPIN, EP300, FAM19A5 and/or GPR24 gene product activity by using the BRD1, NHP2L1, PACSIN2, SERHL, PIPPIN, EP300, FAM19A5 and/or GPR24 gene derived nucleotide sequences in conjunction with well-known antisense, gene "knock-out," ribozyme and/or triple helix methods to decrease the level of BRD1, NHP2L1, PACSIN2, SERHL, PIPPIN, EP300, FAM19A5 and/or GPR24 gene expression. Among the compounds that may exhibit the ability to modulate the activity, expression of the BRD1, NHP2L1, PACSIN2, SERHL, PIPPIN, EP300, FAM19A5 and/or GPR24 gene and/or synthesis the gene products, including the ability to ameliorate the symptoms of a BRD1, NHP2L1, PACSIN2, SERHL, PIPPIN, EP300, FAM19A5 and/or GPR24 gene disorder, are antisense, ribozyme, and triple helix molecules. Such molecules may be designed to reduce or inhibit either unimpaired, or if appropriate, mutant target gene activity. Techniques for the production and use of such molecules are well known to those of skill in the art.

Antisense RNA and DNA molecules act to directly block the translation of mRNA by hybridizing to targetted mRNA and preventing protein translation. Antisense approaches involve the design of oligonucleotides that are complementary to a target gene mRNA. The antisense oligonucleotides will bind to the complementary target gene mRNA transcripts and prevent translation. Absolute complementarity, although preferred, is not required.

A sequence "complementary" to a portion of an RNA, as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

In one embodiment, oligonucleotides complementary to non-coding regions of the BRD1, NHP2L1, PACSIN2, SERHL, PIPPIN, EP300, FAM19A5 and/or GPR24 gene could be used in an antisense approach to inhibit translation of endogenous BRD1, NHP2L1, PACSlN2, SERHL, PIPPIN, EP300, FAM19A5 and/or GPR24 mRNA. Antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

Regardless of the choice of target sequence, it is preferred that in vitro studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors in vivo), or agents facilitating transport across the cell membrane (see, e.g., Letsinger, et al., 1989, Proc. Natl. Acad. Sci. U.S.A. 86,6553-6556; Lemaitre, et al., 1987, Proc. Natl. Acad. Sci. 84, 648-652; PCT Publication No. WO88/09810, published Dec. 15, 1988) or the blood-brain barrier (see, e.g., PCT Publication No. WO89/10134, published Apr. 25, 1988), hybridization-triggered cleavage agents (see, e.g., Krol et al., 1988, BioTechniques 6, 958-976) or intercalating agents (see, e.g., Zon, 1988, Pharm. Res. 5, 539-549). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyfuracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an .alpha.-anomeric oligonucleotide. An alpha.-anomeric oligonucleotide forms specific double-stranded hybrids with complementary RNA in which, contrary to the usual .beta.-units, the strands run parallel to each other (Gautier, et al., 1987, Nucl. Acids Res. 15, 6625-6641). The oligonucleotide is a 2'-O-methylribonucleotide (Inoue, et al., 1987, Nucl. Acids Res. 15, 6131-6148), or a chimeric RNA-DNA analogue (Inoue, et al., 1987, FEBS Lett. 215, 327-330).

Oligonucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein, et al. (1988, Nucl. Acids Res. 16, 3209), methylphosphonate oligonucleotides can be prepared by use of controlled pore glass polymer supports (Sarin, et al., 1988, Proc. Natl. Acad. Sci. U.S.A. 85, 7448-7451), etc.

While antisense nucleotides complementary to the target gene coding region sequence could be used, those complementary to the transcribed, untranslated region are most preferred. For example, antisense oligonucleotides having the following sequences can be utilized in accordance with the invention:
Antisense molecules should be delivered to cells that express the target gene in vivo. A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

However, it is often difficult to achieve intracellular concentrations of the antisense sufficient to suppress translation of endogenous mRNAs. Therefore a preferred approach utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the endogenous target gene transcripts and thereby prevent translation of the target gene mRNA. For example, a vector can be introduced e.g., such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, 1981, Nature 290, 304-310), the promoter contained in the 31 long terminal repeat of Rous sarcoma virus (Yamamoto, et al., 1980, Cell 22, 787-797), the herpes thymidine kinase promoter (Wagner, et al., 1981, Proc. Natl. Acad. Sci. U.S.A. 78, 1441-1445), the regulatory sequences of the metallothionein gene (Brinster, et al., 1982, Nature 296, 39-42), etc. Any type of plasmid, cosmid, YAC or viral vector can be used to prepare the recombinant DNA construct which can be introduced directly into the tissue site. Alternatively, viral vectors can be used that selectively infect the desired tissue, in which case administration may be accomplished by another route (e.g., systemically).

Ribozyme molecules designed to catalytically cleave target gene mRNA transcripts can also be used to prevent translation of target gene mRNA and, therefore, expression of target gene product. (See, e.g., PCT International Publication WO90/11364, published Oct. 4, 1990; Sarver, et al., 1990, Science 247, 1222-1225).

Ribozymes are enzymatic RNA molecules capable of catalyzing the specific cleavage of RNA. (For a review, see Rossi, 1994, Current Biology 4, 469-471). The mechanism of ribozyme action involves sequence specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage. For this sequence, see, e.g., U.S. Pat. No. 5,093,246, which is incorporated herein by reference in its entirety.

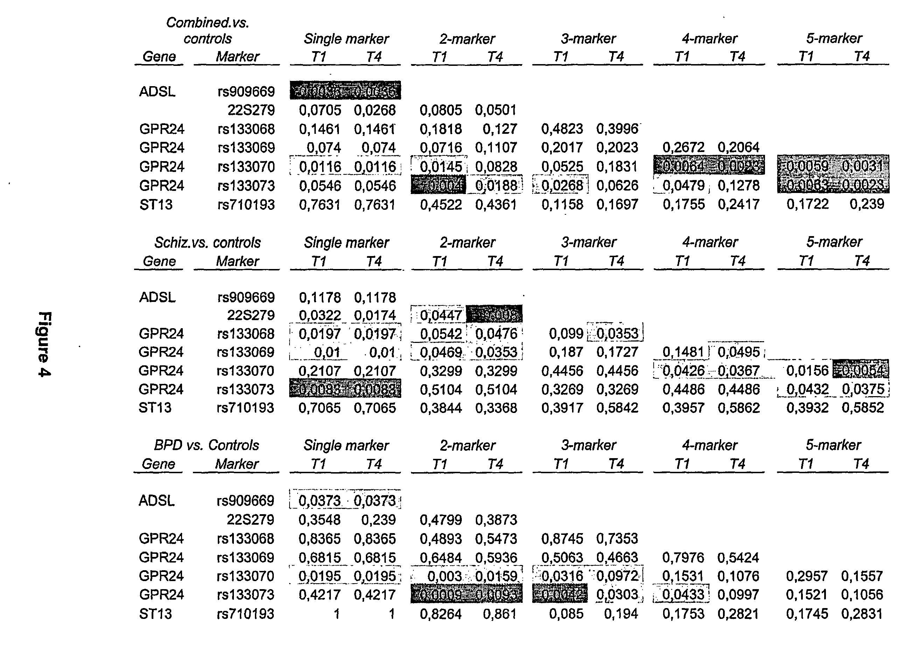

While ribozymes that cleave mRNA at site specific recognition sequences can be used to destroy target gene mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Myers, 1995, Molecular Biology and Biotechnology: A Comprehensive Desk Reference, VCH Publishers, New York, (see especially Figure. 4, page 833) and in Haseloff and Gerlach, 1988, Nature, 334, 585-591, which is incorporated herein by reference in its entirety.

Preferably the ribozyme is engineered so that the cleavage recognition site is located near the 5' end of the target gene mRNA, i.e., to increase efficiency and minimize the intracellular accumulation of non-functional mRNA transcripts. For example, hammerhead ribozymes having the following sequences can be utilized. The ribozymes of the present invention also include RNA endoribonucleases (hereinafter "Cech-type ribozymes") such as the one that occurs naturally in Tetrahymena thermophila (known as the IVS, or L-19 IVS RNA) and that has been extensively described by Thomas Cech and collaborators (Zaug, et al., 1984, Science, 224, 574-578; Zaug and Cech, 1986, Science, 231, 470-475; Zaug, et al., 1986, Nature, 324, 429-433; published International patent application No. WO 88/04300 by University Patents Inc.; Been and Cech, 1986, Cell, 47, 207-216). The Cech-type ribozymes have an eight base pair active site which hybridizes to a target RNA sequence where after cleavage of the target RNA takes place.

As in the antisense approach, the ribozymes can be composed of modified oligonucleotides (e.g., for improved stability, targeting, etc.) and should be delivered to cells that express the target gene in vivo. A preferred method of delivery involves using a DNA construct "encoding" the ribozyme under the control of a strong constitutive pol III or pol II promoter, so that transfected cells will produce sufficient quantities of the ribozyme to destroy endogenous target gene messages and inhibit translation. Because ribozymes unlike antisense molecules, are catalytic, a lower intracellular concentration is required for efficiency.

Endogenous target gene expression can also be reduced by inactivating or "knocking out" the target gene or its promoter using targeted homologous recombination (e.g., see Smithies, et al., 1985, Nature 317, 230-234; Thomas and Capecchi, 1987, Cell 51, 503-512; Thompson, et al., 1989, Cell 5, 313-321; each of which is incorporated by reference herein in its entirety). For example, a mutant, non-functional target gene (or a completely unrelated DNA sequence) flanked by DNA homologous to the endogenous target gene (either the coding regions or regulatory regions of the target gene) can be used, with or without a selectable marker and/or a negative selectable marker, to transfect cells that express the target gene in vivo. Insertion of the DNA construct, via targeted homologous recombination, results in inactivation of the target gene. Such approaches are particularly suited in the agricultural field where modifications to ES (embryonic stem) cells can be used to generate animal offspring with an inactive target gene (e.g., see Thomas and Capecchi, 1987 and Thompson, 1989, supra). However this approach can be adapted for use in humans provided the recombinant DNA constructs are directly administered or targeted to the required site in vivo using appropriate viral vectors.

Alternatively, endogenous target gene expression can be reduced by targeting deoxyribonucleotide sequences complementary to the regulatory region of the target gene (i.e., the target gene promoter and/or enhancers) to form triple helical structures that prevent transcription of the target gene in target cells in the body. (See generally, Helene, 1991, Anticancer Drug Des., 6(6), 569-584; Helene, et al., 1992, Ann. N.Y. Acad. Sci., 660, 27-36; and Maher, 1992, Bioassays 14(12), 807-815).

Nucleic acid molecules to be used in triplex helix formation for the inhibition of transcription should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC.sup.+ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

In instances wherein the antisense, ribozyme, and/or triple helix molecules described herein are utilized to inhibit mutant gene expression, it is possible that the technique may so efficiently reduce or inhibit the transcription (triple helix) and/or translation (antisense, ribozyme) of mRNA produced by normal target gene alleles that the possibility may arise wherein the concentration of normal target gene product present may be lower than is necessary for a normal phenotype. In such cases, to ensure that substantially normal levels of target gene activity are maintained, therefore, nucleic acid molecules that encode and express target gene polypeptides exhibiting normal target gene activity may, be introduced into cells via gene therapy methods such as those described, below, in Section 5.9.2 that do not contain sequences susceptible to whatever antisense, ribozyme, or triple helix treatments are being utilized. Alternatively, in instances whereby the target gene encodes an extracellular protein, it may be preferable to co-administer normal target gene protein in order to maintain the requisite level of target gene activity.

Anti-sense RNA and DNA, ribozyme, and triple helix molecules of the invention may be prepared by any method known in the art for the synthesis of DNA and RNA molecules, as discussed above. These include techniques for chemically synthesizing oligodeoxyribonucleotides and oligoribonucleotides well known in the art such as for example solid phase phosphoramidite chemical synthesis. Alternatively, RNA molecules may be generated by in vitro and in vivo transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

### 2 Gene therapy

Having identified polymorphism(s) as the cause of a disease it is also rendered possible with the present invention to provide a genetic therapy for subjects being diagnosed as having a predisposition according to the invention, said therapy comprising administering to said subject a therapeutically effective amount of a gene therapy vector.

Having discovered the NHP2L1, PACSIN2, SERHL, PIPPIN, BRD1, EP300, FAM19A5 and/or GPR24 genes as etiological factors in mental diseases, such as SCH and/or BPD, the inventors also provide methods for gene therapy and gene therapy vectors for use in subjects irrespective of whether they carry any of the susceptibility or protective alleles/haplotypes described in the present invention.

There are various different methods of gene therapy for the subjects defined in the present invention.

The first two are based on activation of the repair system of the cells by introducing into those cells a gene therapy vector which causes "correction" of the polymorphism by presenting the repair mechanism with a template for carrying out the correction. One such type includes the RNA/DNA chimeraplast, said chimeraplast being capable of correcting the polymorphism in cells of said subject. Examples of the design of such chimeraplasts can be found in e.g. US 5,760,012; US 5,888,983; US 5,731,181; US 6,010,970; US 6,211,351.

The second method is based on application of single stranded oligonucleotides, wherein the terminal nucleotides is protected from degradation by using 3' and 5' phosphorothioat-linkage of the monomers. This gene therapy vector is also capable of "correcting" the polymorphism by replacing one nucleotide with another.

These first two types of gene therapy vectors comprise a small sequence (less than 50 bases) which overlaps with the polymorphism in question. Suitable sequences for this purpose are genomic sequences located around the polymorphism.

Other types of gene therapy include the use of retrovirus (RNA-virus). Retrovirus can be used to target many cells and integrate stably into the genome. Adenovirus and adeno-associated virus can also be used. A suitable retrovirus or adenovirus for this purpose comprises an expression construct with the wildtype gene under the control of the wildtype promoter or a constitutive promoter or a regulatable promoter such as a repressible and/or inducible promoter or a promoter comprising both repressible and inducible elements.

A further group of gene therapy vectors includes vectors comprising interfering RNA (RNAi) for catalytic breakdown of mRNA carrying the polymorphism. RNAi can be used for lowering the expression of a given gene for a relatively short period of time. In particular these RNAi oligos may be used for therapy for both subjects carrying a susceptibility allele as described in the present invention as well as for subjects which do not carry such an allele.

Interfering RNA ("RNAi") is double stranded RNA that results in catalytic degradation of specific mRNAs, and can also be used to lower gene expression.

The gene therapy vectors carry the protective allele of the genes. The protective allele means in the present content that presence of this allele in an individual indicates protection against a mental disease of the invention.

Described below are methods and compositions whereby a BRD1, NHP2L1, PACSIN2, SERHL, PIPPIN, EP300, FAM19A5 and/or GPR24 gene disorder or a disorder of thought and/or mood, such as bipolar and genetically related unipolar affective disorders, may be treated.

With respect to an increase in the level of normal BRD1, NHP2L1, PACSIN2, SERHL, PIPPIN, EP300, FAM19A5 AND GPR24 gene expression and/or BRD1, NHP2L1, PACSIN2, SERHL, PIPPIN, EP300, FAM19A5 and GPR24 GENE product activity, the BRD1, NHP2L1, PACSIN2, SERHL, PIPPIN, EP300, FAM19A5 and GPR24 gene derived nucleotide sequences, for example, be utilized for the treatment of a BRD1, NHP2L1, PACSlN2, SERHL, PIPPIN, EP300, FAM19A5 and/or GPR24 gene associated disorder such as SCH and/or BPD. Such treatment can be performed, for example, in the form of gene replacement therapy. Specifically, one or more copies of a normal BRD1, NHP2L1, PACSlN2, SERHL, PIPPIN, EP300, FAM19A5 and/or GPR24 gene or a portion of said gene that directs the production of a gene product exhibiting normal BRD1, NHP2L1, PACSIN2, SERHL, PIPPIN, EP300, FAM19A5 and/or GPR24 gene function, may be inserted into the appropriate cells within a patient, using vectors that include, but are not limited to adenovirus, adeno-associated virus, and retrovirus vectors, in addition to other particles that introduce DNA into cells, such as liposomes.

Because the BRD1, NHP2L1, PACSIN2, SERHL, PIPPIN, EP300, FAM19A5 and GPR24 genes are expressed in the brain, such gene replacement therapy techniques should be capable delivering the BRD1, NHP2L1, PACSIN2, SERHL, PIPPIN, EP300, FAM19A5 and/or GPR24 gene sequences to these cell types within patients. Thus, in one embodiment, techniques that are well known to those of skill in the art (see, e.g., PCT Publication No. WO89/10134 published Apr. 25, 1988) can be used to enable the BRD1, NHP2L1, PACSIN2, SERHL, PIPPIN, EP300, FAM19A5 and/or GPR24 gene sequences to cross the blood-brain barrier readily and to deliver the sequences to cells in the brain. With respect to delivery that is capable of crossing the blood-brain barrier, viral vectors such as, for example, those described above, are preferable. Also included are methods using liposomes either in vivo ex vivo or in vitro. Wherein the BRD1, NHP2L1, PACSlN2, SERHL, PIPPIN, EP300, FAM19A5 and/or GPR24 gene sense or antisense DNA is delivered to the cytoplasm and nucleus of target cells. Liposomes can deliver the BRD1, NHP2L1, PACSIN2, SERHL, PIPPIN, EP300, FAM19A5 and GPR24 gene sense or nonsense RNA to humans and the human brain or in mammals through intrathecal delivery either as part of a viral vector or as DNA conjugated with nuclear localizing proteins or other proteins that increase take up into the cell nucleus.

In another embodiment, techniques for delivery involve direct administration of such BRD1, NHP2L1, PACSIN2, SERHL, PIPPIN, EP300, FAM19A5 and/or GPR24 gene sequences to the site of the cells in which the BRD1, NHP2L1, PACSlN2, SERHL, PIPPIN, EP300, FAM19A5 and/or GPR24 gene sequences are to be expressed. Additional methods that may be utilized to increase the overall level of the BRD1, NHP2L1, PACSIN2, SERHL, PIPPIN, EP300, FAM19A5 and/or GPR24 gene expression and/or the BRD1, NHP2L1, PACSIN2, SERHL, PIPPIN, EP300, FAM19A5 and/or GPR24 gene product activity include the introduction of appropriate BRD1, NHP2L1, PACSIN2, SERHL, PIPPIN, EP300, FAM19A5 and/or GPR24 gene-expressing cells, preferably autologous cells, into a patient at positions and in numbers that are sufficient to ameliorate the symptoms of a BRD1, NHP2L1, PACSlN2, SERHL, PIPPIN, EP300, FAM19A5 and/or GPR24 gene associated disorder, such as SCH and/or BPD. Such cells may be either recombinant or non-recombinant.

Among the cells that can be administered to increase the overall level of BRD1, NHP2L1, PACSIN2, SERHL, PIPPIN, EP300, FAM19A5 and/or GPR24 gene expression in a patient are normal cells, preferably brain cells and also choroid plexus cells within the CNS which are accessible through intrathecal injections. Alternatively, cells, preferably autologous cells, can be engineered to express BRD1, NHP2L1, PACSIN2, SERHL, PIPPIN, EP300, FAM19A5 and/or GPR24 gene sequences, and may then be introduced into a patient in positions appropriate for the amelioration of the symptoms of a BRD1, NHP2L1, PACSIN2, SERHL, PIPPIN, EP300, FAM19A5 and/or GPR24 gene asoociated disorder. Alternately, cells that express an unimpaired BRD1, NHP2L1, PACSIN2, SERHL, PIPPIN, EP300, FAM19A5 and/or GPR24 gene and that are from a MHC matched individual can be utilized, and may include, for example, brain cells. The expression of the BRD1, NHP2L1, PACSIN2, SERHL, PIPPIN, EP300, FAM19A5 and/or GPR24 gene derived sequences is controlled by the appropriate gene regulatory sequences to allow such expression in the necessary cell types. Such gene regulatory sequences are well known to the skilled artisan. Such cell-based gene therapy techniques are well known to those skilled in the art, see, e.g., Anderson, U.S. Pat. No. 5,399,349.

When the cells to be administered are non-autologous cells, they can be administered using well known techniques that prevent a host immune response against the introduced cells from developing. For example, the cells may be introduced in an encapsulated form which, while allowing for an exchange of components with the immediate extracellular environment, does not allow the introduced cells to be recognized by the host immune system.

Additionally, compounds, such as those identified via techniques such as those described above that are capable of modulating the BRD1, NHP2L1, PACSlN2, SERHL, PIPPIN, EP300, FAM19A5 and/or GPR24 gene product activity can be administered using standard techniques that are well known to those of skill in the art. In instances in which the compounds to be administered are to involve an interaction with brain cells, the administration techniques should include well known ones that allow for a crossing of the blood-brain barrier such as intrathecal injection and conjugation with compounds that allow transfer across the blood brain barrier.

Preferred embodiments of the invention concern the gene therapy vectors comprising
(i) a DNA sequence selected from the sequences identified as SEQ ID NO 1-7 and 94, or a fragment thereof, wherein said sequence or a said fragment comprises the protective allele of an SNP selected from the SNPs having refSNP IDs: rs11561, rs5758405 rs8779, rs132806, rs2068943, rs2267487, rs881542, rs926333, rs1060387, rs1006407, rs6002408, rs4468, rs138855, rs2239848, rs138880, rs138881, rs20551, rs2294976, rs2076578, rs1046088, rs133068, rs133069, rs133070, rs133073, rs6002408, or
(ii) a DNA sequence selected from the sequences identified as SEQ ID NOs: 8-14 and 95, or a fragment of said DNA sequence, wherein said DNA sequence or said fragment comprises the protective allele of an SNP selected from the SNPs having refSNP IDs: rs11561, rs5758405 rs8779, rs132806, rs2068943, rs2267487, rs881542, rs926333, rs1060387, rs1006407, rs6002408, rs4468, rs138855, rs2239848, rs138880, rs138881, rs20551, rs2294976, rs2076578, rs1046088, rs133068, rs133069, rs133070, rs133073, rs600240.

### Pharmaceutical compositions and methods of administration

The compounds that are determined to affect BRD1, NHP2L1, PACSlN2, SERHL, PIPPIN, EP300, FAM19A5 and/or GPR24 gene expression or gene product activity can be administered to a patient at therapeutically effective doses to treat or ameliorate a gene associated disorder, such as SCH and/or BPD. A therapeutically effective dose refers to that amount of the compound sufficient to result in amelioration of symptoms of such a disorder.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50 /ED50. Compounds that exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC.sub.50 (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

Pharmaceutical compositions for use in accordance with the present invention may be formulated in conventional manner using one or more physiologically acceptable carriers or excipients.

Thus, the compounds and their physiologically acceptable salts and solvates may be formulated for administration by inhalation or insufflation (either through the mouth or the nose) or intrathecal, oral, buccal, parenteral or rectal administration.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For buccal administration the compositions may take the form of tablets or lozenges formulated in conventional manner. For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

### Examples

In order to identify potential susceptibility variants in the NHP2L1, PACSIN2, SERHL, PIPPIN, BRD1, EP300, FAM19A5 and/or GPR24 genes, the genes were sequenced in a subset of patients with mental disorders. The genomic sequences containing upstream promoter sequences, intronic sequences close to the exon/intron boundaries and coding sequences were analysed. In addition, potential SNPs were identified by searching databases such as the dbSNP (hftr):/A,vww.ncbi.nim.nih.gov/proiects/SNP/). The identified variants were analysed in a case-control sample from Scotland described below.

### Example 1. Analysis of unrelated patients and ethnically matched unrelated controls from Scotland.

### Subjects

The case-control sample from Scotland consisted of 103 patients with SZ, 162 patients with BPD and 200 ethnically matched controls. Informed consent was obtained from all patients prior to inclusion, and the study was approved by the local research ethical committees where patients were recruited. Subjects were interviewed by an experienced psychiatrist and venous blood taken for subsequent DNA extraction using routine procedures. Diagnoses were made according to DSM-IV criteria after case-note review and personal interview using the Schedule for Affective Disorders and Schizophrenia - Lifetime version. Final diagnoses were reached by consensus between two experienced psychiatrists (DB and WM). Control subjects (prescreened to exclude those with serious chronic illness) were drawn from the same population in South East and South Central Scotland and recruited from Scottish National Blood Transfusion Service donors. We have reported these sample sets previously (17, 18).

### Genotyping

The genes selected were included on the basis of their location, expression profile and existing knowledge of their function. The selection criteria for the single nucleotide polymorphisms (SNPs) were also based on a functional approach evaluating the type of SNP (prioritizing non-synonymous SNPs) and location (preferably promoter, UTR, intron/exon boundaries, and conserved regions). Genotyping was performed using 40 ng of DNA per multiplex PCR. Exonuclease1 and Shrimp Alkaline Phosphatase were used for purification steps and the SNPs genotyped by multiplex single base extension technology using the ABI SNaP-shot kit and an ABI 310 Genetic Analyzer or a 3100 Avant Genetic Analyzer (Applied Biosystems, Foster City, CA) according to the manufacturer's recommendations. The data was analyzed using the GeneScan 3.1.2 program (Applied Biosystems, Foster City, CA). Standard PCR conditions were used. The microsatellite markers were analyzed using fluorescent primers, standard conditions for (duplex) PCR amplification and separation of allelic fragments on an ABI 310 Genetic Analyzer. All primer sequences are available on request. To minimize genotyping errors all polymorphisms were scored independently by two experienced investigators. Any discordances lead to re-analysis of the sample. In addition a number of SNPs (including *BRD1* rs4468 and rs138880) were analyzed twice in at least 85 individuals. No divergent genotypes were observed, indicating a very low error rate (for allele calls less than 0.006).

### Statistical analysis

Chi-square and Fisher's Exact test were used to assess allele and genotype distributions. Haplotype Trend Regression (HTR) was used to estimate the frequency and analyze the distribution of haplotypes (19). When comparing two groups, HTR produces an overall p-value for the observed distribution of all the haplotypes at an interval defined by a set of neighboring markers and also a haplotype-specific p-value describing the likelihood of the observed distribution of each of the individual haplotypes. The p-values from HTR presented in this study are empirical values based on up to 100,000,000 permutations. P-values less than 0.05 are referred to as significant. The p-values presented are not corrected for multiple testing. However, the highly significant haplotype associations remained significant even after a Bonferroni correction, which is overly conservative as the tests performed are not independent.

Tests of linkage disequilibrium were performed using the program Ldmax from the GOLD software package (http://www.sph.umich.edu/csg/abecasis/GOLD) which uses the Slatkin and Excoffier expectation-maximization based approach (20).

### In silico analysis

The impact of a promoter SNP on potential binding sites for transcription factors was analyzed using the program Matinspector (www.genomatix.de) (21, 22). This program utilizes a library of matrix descriptions for transcription factor binding sites to identify potential sites in a sequence analyzed and assign a quality rating of matches (core and matrix similarity) estimating the influence of a SNP on the binding of transcription factors.

The possible effect of an intragenic SNP on splicing was investigated using the programs ESEfinder release 2.0 (hftp://rulai.cshl.edu/tools/ESE) (21-23), RESCUE-ESE Web Server (http://genes.mit.edu/burgelab/rescue-ese) (24), FAS-ESS web server (http://genes.mit.edu/fas-ess/) (25), ExonScan Web Server (http://genes.mit.edu/exonscan/) (24-26) and NNSPLlCE (http://www.fruitfly.org/seq_tools/splice/) (27).

The effects of 3' UTR SNPs on microRNA binding sites were analyzed using the miRBase Targets Pre-release Version 1.0 (http://microrna.sanger.ac.uk/targets/v1/) which is a web resource provided by the Wellcome Trust Sanger Institute containing computationally predicted targets for microRNAs across a number of species.

### Northern blotting

Human Multiple Tissue Northern Blots II and V (Clontech Laboratories, CA, USA) were probed with a α-³²P-CTP-labelled probe against *BRD1.* Full length *BRD1* was cloned from human brain cDNA (Clontech, #639300), gel purified (Qiagen, #28704) and used as template for the probe synthesis. The probe was synthesized and labeled using 15,000 Ci/mmol α-³²P-CTP (EasyTides) and the Prime-It RmT Random Primer Labeling Kit (Stratagene, #300392) following the manufacturer's protocol. The specific activity of the probe was measured to 1.3 x 10⁹ dpm/µg. The blots were hybridized and rinsed as described previously (28) and exposed to X-ray film for 3 weeks.

### Preparation of rat, rabbit, human and fetal pig brain tissue

Seven male Wistar rats (250-300 g) and 5 male New Zealand white rabbits (2.5-3.5 kg) were deeply anesthetized before transcardial perfusion with 0.5 l (rats) or 1.5 I (rabbits) phosphate buffered 4% paraformaldehyde (pH 7.4) at 4°C as approved by the Danish Council for Animal Research Ethics. The brains were immersed in the same fixative for 24 hrs and divided into 2-3 minor coronal tissue blocks by histOmer embedding and sectioning on a HistOtech slicer (29) before vibratome sectioning into 50 µm sections.

Human brain tissue was obtained from 2 donors who had donated their remains for educational and scientific purposes at the Institute of Anatomy, University of Aarhus. The brains were removed 24-72 hrs postmortem and briefly stored in 10% formalin before smaller tissue blocks containing the frontal cortex were paraffin-embedded and microtome sectioned into 10 µm sections.

Fetal pig brain tissue was obtained from pregnant sows anesthetized by carbon dioxide and sacrificed by bleeding at embryonic day 40, 60, 80, 100 and 115, respectively. The removed fetal brain tissue was briefly immersed in 10% formalin before tissue blocks containing the forebrain (cortex cerebri) and the hindbrain (lower brainstem and cerebellum) were embedded in paraffin and microtome sectioned into 2 µm sections.

For quantitative analysis of the mRNA expression, the hippocampus, cortex, basal ganglia, cerebellum and brain stem were dissected from the fetal brains and immediately frozen in liquid nitrogen. After thawing on ice, total RNA was extracted with RNAlate^{™}-ICE (Ambion) according to the manufacturer's protocol.

### Immunohistochemistry

The anti-BRD1 monoclonal antibody used was provided by Bryan Young and was identical to that used in the initial BRD1 cloning study (30). It was raised against the 11 amino acid peptide RRPFSWEDVDR corresponding to amino acids 692-702 of BRD1. Vibratome and cryostat sections were initially blocked for endogenous biotin before preincubation with 1% Triton X-100 and 0.2% milk (Bidinger, Denmark) in TBS for 30 min followed by incubation at 4°C with the primary monoclonal antibody (mouse anti-BRD1) diluted 1:500-1:1000 in TBS containing 1% Triton X-100 and 0.2% milk for 72 hrs. After rinsing with TBS and 1% Triton X-100 for 3x15 min, the sections were incubated for 1 hr at room temperature with the secondary antibody (sheep anti-mouse lg biotin-labeled, Amersham, RPN 1001) diluted 1:200-1:400 in TBS containing 1% Triton X-100 and 0.2% milk. Endogenous peroxidase activity was blocked with a solution of 80 ml TBS with 10 ml H₂O₂ and 10 ml methanol for 10 min. Avidin-peroxidase (Sigma: A 3151) diluted 1:200-1:400 with TBS containing 1 % Triton X-100 and 0.2 % milk was then applied for 1 hr at room temperature. After rinsing for 3 x 15 min in TBS + 1 % Triton X-100, the formed avidin-peroxidase complexes were visualized by incubation for 10 min with diaminobenzidine (DAB) made by dissolving a 10 mg DAB-tablet (Kem-En-Tec Diagnostics A/S) in 10 ml water and immediately before use adding 10 µl of 35 % H₂O₂. After mounting and coverslipping with Depex, the sections were analyzed using a light microscope and compared with Nissl-stained sections, securing systematic analysis of consecutive coronal brain levels.

Paraffin embedded sections were stained according to the above mentioned protocol after initial target retrieval by microwave boiling of the sections for 10 min in a citrate buffer (pH=6.0) and usage of the primary monoclonal mouse anti-BRD1 antibody diluted 1:150.

Double immunofluorescence staining was performed on vibratome sections incubated for 72 hrs at 4°C with the primary monoclonal BRD1 antibody diluted 1:500, followed by a 1 hr incubation at room temperature with the secondary antibody (goat anti-mouse Ig FITC-labeled, Abcam, ab6785) diluted 1:600. The sections were then treated with 500 µg RNAseA (Roche, 109 142) for 20 min, before nuclear staining with TO-PRO-3 (Molecular Probes, T3605) diluted 1:1000 for 5 min, followed by mounting and coverslipping with Vecta-shield H1000 and subsequent confocal microscopic analysis.

### cDNA synthesis and real-time qPCR

cDNA was synthesized from 1 µg total RNA in 20 µl reactions using iScript^{™} cDNA synthesis Kit (Biorad). After synthesis the cDNA was diluted five times with double distilled water. The real-time RT-PCR reactions were made with DyNAmo^{™}SYBR® Green qPCR kit (Finnzymes) in a total volume of 20 µl, using 6 pmol primers specific for pig *BRD1* (forward: 5'-GGGCCAAGTGCAGCGGCTAC-3', reverse: 5'-CTCCATCATCTTCAGCTTGTC). Amplification was carried out on a Biorad iCycler using the following conditions: 95°C 15 min, (94°C 10 s, 58°C 20 s, 72°C 30 s) 40 repeats, 72°C 10 min, cooled to 20°C. PCR specificity was controlled by melting curve analysis. All reactions were also run using primers specific for pig-GAPDH as an internal standard
(forward: 5'-GGGGAATTCGCCACCATGGTGAAGGTCGGAGTGAAC-3', reverse:
5'-GGGGAATTCGATGACAAGCTTCCCATTCTC-3').

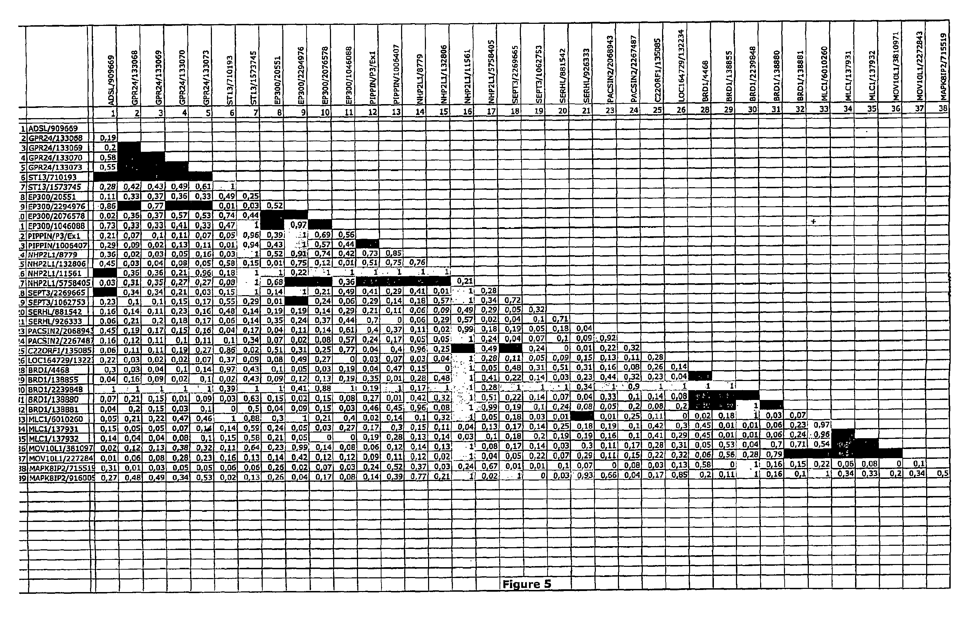

The relative standard curve for the real-time qPCR reactions were made from RT-PCR of cDNA from the 115-day-old fetus hippocampus, amplified with the above mentioned conditions. The product was run on a 1% agarose gel, cut out and purified using QlAquick Gel Extraction Kit Protocol (Qiagen). The DNA was diluted and used to produce a relative standard curve showing above 90% PCR efficiency in the area. The specificity of the PCR product was verified by sequencing showing the position of the product to span the last two exons of the gene. Relative quantification of the expression was determined as follows. Three replicates of the threshold cycle for *BRD1* (C_{T,X}) and *GAPDH* (C_{T,R}) were measured. Subtracting each C_{T,R} replicate from each C_{T,X} nine threshold differences (ΔC_{T}) were calculated. Under assumptions of normality and variance homogeneity the temporal change in expression was examined by analysis of variance contrasting to embryonic day 115 the mean threshold difference from each of the other days. P-values are adjusted for the number of contrasts tested using the Sidák method. Note this analysis is a variant of the 2-^{ΔΔC}T method (31) with ΔΔC_{T} equal to the contrasts and results are presented in terms of fold changes 2-^{ΔΔC}T (Fig. 5). Moreover, a polynomial regression model up to third order was determined using a forward inclusion stepwise procedure. The significance of the highest order term is indicated and the resulting regression curve with 95 % confidence bands is shown after back-transformation by 2-^{ΔC}T (Fig. 5).

### Results

### Association analysis

5 SNPs in *BRD1,* 9 SNPs in 4 neighboring genes and two microsatellite markers were selected and genotyped in a Scottish case-control sample, including 103 SZ cases, 162 BPD cases, and 200 controls (Table 1). Rs3752466 turned out to be constant and was consequently excluded from further analysis. No significant deviation from Hardy-Weinberg equilibrium was observed for any of the SNPs in the case or control groups. Single-marker and multi-marker haplotype analysis was performed comparing controls to SZ, BPD, and the two case groups combined. *(Table 1 around here).*

Significant single-marker associations were observed for two *BRD1* SNPs and D22S1169 (Table 2). The promoter SNP rs138880 showed association in SZ, BPD and the combined case group with p-values of 0.0061, 0.0274 and 0.0046, respectively. The 3' UTR SNP rs4468 and D22S1169 showed significant association with SZ (P = 0.0088 and 0.0214, respectively) but not with BPD. These allelic associations were also found in the analysis of the genotypic distribution with similar p-values (results not shown). *(Table 2 around here).*

The haplotype analysis considering the overall distribution of all haplotypes of 2-4 neighboring markers in a sliding window fashion showed comparable results in both disorders (Table 2). Highly significant overall p-values (as low as 0.00001) were observed in especially the 3- and 4-marker analysis involving the *BRD1* SNPs rs138855, rs2239848, rs138880 and rs138881. Haplotypes involving the microsatellite markers and the proximal *BRD1* SNPs showed primarily association in SZ.

Analysis of the individual haplotypes revealed that the overall association could be attributed to both "risk" and "protective" haplotypes (Table 3). A 3-marker core haplotype spanning *BRD1* SNPs rs138855, rs2239848 and rs138880 had a frequency of around 9% in cases against only 1 % in controls, producing a haplotype specific p-value of 10⁻⁰⁶. Removing the rare middle SNP rs2239848 from the analysis resulted in a reduced 2-marker "risk" haplotype (G-C) showing a frequency of ~10% in cases versus ~1% in controls (p-value of 2.8x10⁻⁰⁷ in the combined case group). *(Table 3 around here).*

"Protective" haplotypes over-represented among controls included both microsatellites, all the *BRD1* SNPs and extended into *MLC1* (megalencephalic leukoencephalopathy with subcortical cysts 1) (Table 3). These related and rather frequent haplotypes showed primarily significant results when compared to their frequencies in SZ.

### Linkage disequilibrium

A high degree of inter-marker linkage disequilibrium (LD) between the SNPs in *BRD1* was found in controls and cases (Table 4). Likewise a high LD between the SNPs in *MLC1* extending distally to *MOV10L1* (Moloney leukemia virus 10-like 1, homolog (mouse)) was seen in both cases and controls. No significant LD was seen between *BRD1* and *MLC1. (Table 4 around here).*

### In silico analysis

Using Matinspector, the two promoter SNPs in *BRD1* (Table 1) were analyzed for potential effects on binding sites for transcription factors. The C-allele of rs138880 introduced binding sites for two transcription factors: The zinc finger binding protein factor encoded by *ZNF202* which is thought to predominantly regulate genes participating in lipid metabolism (32), and hairy and enhancer of split homolog 1 (HES-1) (showing a high core and matrix similarity), which is a transcriptional repressor inhibiting neural differentiation (33). Rs138881 did not introduce any changes.

The synonymous *BRD1* SNP rs2239848 located in exon 1 was analyzed for effects on exon splicing enhancers (ESE) and exon splicing silencers (ESS). According to ESEfinder the presence of the rare A-allele eliminated a binding site for the SR protein SRp55. However, analysis of the splice site in exon 1 revealed a very strong donor site thus suggesting a very limited potential effect of exon 1 ESEs. Neither RESCUE-ESE nor FAS-ESS identified any effect of rs2239848 on splicing.

Analysis of intronic and 3'UTR SNPs did not suggest any differential effects of the alleles.

### Northern blotting

*A BRD1* transcript of the expected size (approximately 4.6 kb) was observed in most of the human brain regions tested, i.e. whole brain, cerebellum, cerebral cortex, medulla, spinal cord, occipital pole, frontal lobe, caudate nucleus, corpus callosum, hippocampus and thalamus (data not shown). A further faint band of a slightly larger size was present in whole brain, cerebellum and cerebral cortex, which suggests alternative splicing of the pre-mRNA that seems to be differently regulated across the human brain.

### Immunohistochemistry

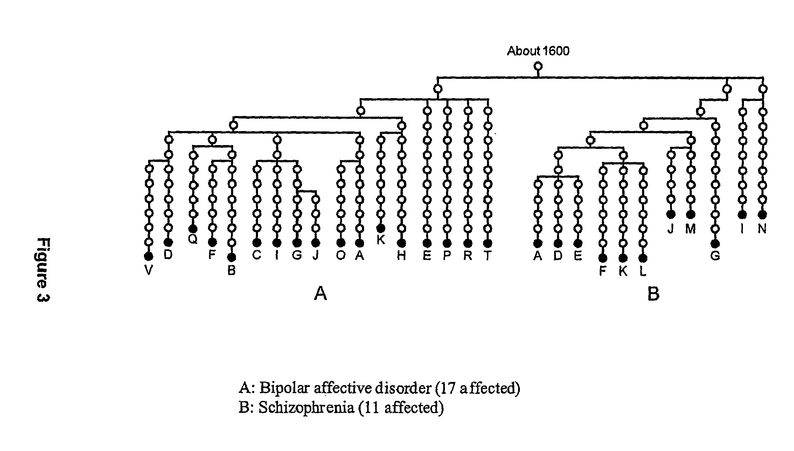

BRD1-immunostaining showed similar neuronal staining patterns in the adult rat, rabbit, and human cortex cerebri (Fig. 1). The neurons in cortex layers I-Vl displayed prominent BRD1 immunoreactivity in the perikaryal cytosol surrounding a weaker granular staining of the nucleus (Figs. 1-2). BRD1 immunoreactivity was likewise seen in the proximal part of the primary dendrites, whereas the distal dendrites and the axon seemed unstained (Figs. 1-2). Glial staining was not noted in the human cerebral cortex or any part of the rat and rabbit CNS. Consecutive sectioning and immunostaining of the rat and rabbit brain confirmed that neuronal BRD1 immunoreactivity was distributed throughout the adult nervous system e.g. the cerebrum, brainstem, cerebellum and spinal cord (Fig. 3). (*Figs. 1-3* *around here*).

Fetal pig brain tissue of embryonic day 40, 60 (Figs. 4A-C), 80, 100, and 115 (Fig. 4D-F) revealed dense nuclear staining in the neuroepithelial cell layer and the early differentiated neuroblasts (Figs. 4A-B). Medium differentiated neuroblasts displayed an intense nuclear and perikaryal cytosolar staining pattern (Fig. 4C), while fully differentiated neurons generally stained more weakly and in particular had a very weak nuclear staining compared to the staining intensity seen in the perikaryal cytosol (Figs. 4D-F). (*Fig. 4* *around here*).

### mRNA expression in fetal pig brain

The level of expression of *BRD1* mRNA was measured and normalized to the amount of *GAPDH* mRNA, which was constantly expressed. While the overall trend in the five examined areas of fetal pig brain was the same, the differences in expression levels were most pronounced in cortex cerebri in particular but also in the regions of the brainstem and basal ganglia (Fig. 5). The maximum amount of mRNA was measured at embryonic day 60 in all samples. The two areas that were possible to dissect in the 40-day-old embryo (cortex and cerebellum) showed a lower level of expression. For some areas the abundance of mRNA was also relatively increased at day 80 (brainstem, cortex and basal ganglia). Between embryonic day 80 and 115 *BRD1* mRNA expression leveled off. *(**Fig. 5* *around here).*

**Table 1 Genotyped polymorphisms and allele frequencies.**

| | | | | | | MAF | |
|---|---|---|---|---|---|---|---|
| Gene | SNP | Location (Mb)^{a} | Type of SNP | Alleles^{b} | Controls | BPD | SZ |
| *FAM19A5* | rs132234 | 47.424523 | Intron | C/T | 0.27 | 0.29 | 0.27 |
| *FAM19A5* | rs3752466 | 47.466709 | 3' UTR | C/T | 0.00 | 0,00 | 0.00 |
| | D22S922 | 47.491607 | Microsatellite | | | | |
| | D22S1169 | 47.722917 | Microsatellite | | | | |
| *BRD1* | rs4468 | 48.488513 | 3' UTR | T/C | 0.35 | 0.37 | 0.49 |
| *BRD1* | rs138855 | 48.519343 | Intron | G/C | 0.16 | 0.14 | 0.13 |
| *BRD1* | rs2239848 | 48.537615 | Syn. | G/A | 0.01 | 0.02 | 0.01 |
| *BRD1* | rs138880 | 48.539472 | Promoter | A/C | 0.16 | 0.22 | 0.25 |
| *BRD1* | rs138881 | 48.541343 | Promoter | G/A | 0.10 | 0.12 | 0.13 |
| *MLC1* | rs6010260 | 48.818300 | Nonsyn. | G/T | 0.13 | 0.14 | 0.16 |
| *MLC1* | rs137931 | 48.826710 | Promoter | C/- | 0.26 | 0.22 | 0.27 |
| *MLC1* | rs137932 | 48.826857 | Promoter | G/A | 0.25 | 0.22 | 0.27 |
| *MOV10L1* | rs3810971 | 48.849123 | Nonsyn. | C/T | 0.24 | 0.26 | 0.20 |
| *MOV10L1* | rs2272843 | 48.901923 | Nonsyn. | C/A | 0.15 | 0.13 | 0.12 |
| *MAPK8IP2* | rs715519 | 49.328513 | Promoter | C/G | 0.18 | 0.16 | 0.19 |
| *MAPKBLP2* | rs916005 | 49.334387 | Intron | G/A | 0.04 | 0.04 | 0.05 |

| | | | | | | | |
|---|---|---|---|---|---|---|---|
| ^{a} According to the UCSC Genome Browser, May 2004 assembly (http://www.genome.ucsc.edu) ^{b} Major allele/minor allele on the +strand (http://www.genome.ucsc.edu) Nonsyn=Nonsynonymous SNP, MAF= Minor allele frequency, Syn.=Synonymous SNP | | | | | | | |

**Table 2 Single marker and overall haplotype association analysis**

| | | Empirical overall p-values^{a} | | | |
|---|---|---|---|---|---|
| Gene | SNP | Single marker | 2-marker | 3-marker | 4-marker |
| BPD+SZ | | | | | |
| *FAM19A5* | rs132234 | 0,6116 | | | |
| | D22S922 | 0,5552 | 0,5738 | | |
| | D22S1169 | **0,0383** | 0,4270 | 0,4635 | |
| *BRD1* | rs4468 | **0,0215** | 0,3317 | 0,4030 | 0,1853 |
| *BRD1* | rs138855 | 0,4186 | 0.3182 | 0,1374 | 0,1149 |
| *BRD1* | rs2239848 | 0,3983 | 0.6658 | 0.2092 | 0,2630 |
| *BRD1* | rs138880 | **0,0046** | **0,0381** | **0,00005** | 0.0607 |
| *BRD1* | rs138881 | 0,1727 | 0.0681 | 0.1550 | **0.0003** |
| *MLc1* | rs6010260 | 0,1631 | 0.4043 | 0.2102 | 0,3086 |
| *MLC1* | rs137931 | 0,5796 | 0.1604 | 0.1926 | 0.2358 |
| *MLC1* | rs137932 | 0,6327 | 0.9210 | 0.4051 | 0.3061 |
| *MOV10L1* | rs3810971 | 0,9874 | 0.9882 | 0.8355 | 0.2924 |
| *MOV10L1* | rs2272843 | 0,3018 | 0.1966 | 0.4691 | 0,4295 |
| *MAPK81P2* | rs715519 | 0,7976 | 0.7516 | 0.4568 | 0.4123 |
| *MAPK81P2* | rs916005 | 1.0000 | 0.9552 | 0.9786 | 0,7152 |

| BPD | | | | | |
|---|---|---|---|---|---|
| *FAM19A5* | rs132234 | 0.6271 | | | |
| | D22S922 | 0,0885 | 0,8920 | | |
| | D22S1169 | 0,4042 | 0,6196 | 0,4032 | |
| *BRD1* | rs4468 | 0.7814 | 0,8945 | 0,8753 | 0,7872 |
| *BRD1* | rs138855 | 0.4377 | 0,0769 | 0,5369 | 0,5341 |
| *BRD1* | rs2239848 | 0,2501 | 0.5005 | 0.1742 | 0,5517 |
| *BRD1* | rs138880 | **0.0274** | 0.0797 | **0,00006** | 0.1877 |
| *BRD1* | rs138881 | 0.2473 | 0.1822 | 0.2096 | **0,0013** |
| *MLC1* | rs6010260 | 0.4011 | 0,6883 | 0.6108 | 0.5603 |
| *MLC1* | rs137931 | 0.2842 | 0.2795 | 0.1011 | 0.4430 |
| *MLC1* | rs137932 | 0.3229 | 0.8343 | 0.3976 | 0.2014 |
| *MOV10L1* | rs3810971 | 0.5368 | 0.8155 | 0.8016 | 0.7716 |
| *MOV10L1* | rs2272843 | 0.5064 | 0.1395 | 0.3280 | 0.3263 |
| *MAPK8IP2* | rs715519 | 0.4675 | 0.6392 | 0.3063 | 0.4475 |
| *MAPK8IP2* | rs916005 | 0.8427 | 0.8895 | 0.8328 | 0.3167 |

| SZ | | | | | |
|---|---|---|---|---|---|
| *FAM19A5* | rs132234 | 0.7013 | | | |
| | D22S922 | 0,3501 | 0,5950 | | |
| | D22S1169 | **0,0214** | **0,0194** | **0,0426** | |
| *BRD1* | rs4468 | **0,0088** | **0,0184** | 0,0657 | **0,0228** |
| *BRD1* | rs138855 | 0.5010 | 0.1138 | **0,0117** | 0,0839 |
| *BRD1* | rs2239848 | 1.0000 | 0.5944 | **0.0470** | **0,0095** |
| *BRD1* | rs138880 | **0.0061** | 0.1610 | **0,00001** | **0.0203** |
| *BRD1* | rs138881 | 0.2896 | 0.0521 | 0.1358 | **0,0002** |
| *MLC1* | rs6010260 | 0,0823 | 0.3103 | 0,0763 | 0.1593 |
| *MLC1* | rs137931 | 0.8440 | 0.1836 | 0.3326 | 0.1100 |
| *MLC1* | rs137932 | 0.7615 | 0,8969 | 0,2821 | 0.4183 |
| *MOV10L1* | rs3810971 | 0.3431 | 0,8971 | 0.9061 | 0.1839 |
| *MOV10L1* | rs2272843 | 0.2701 | 0.3417 | 0,7132 | 0.7849 |
| *MAPK8IP2* | rs715519 | 0.7254 | 0.6423 | 0.5626 | 0.5239 |
| *MAPK8IP2* | rs916005 | 0.8482 | 0.9765 | 0.9187 | 0.7463 |

**Table 3 Distribution of selected individual haplotypes.**

| Haplotype | | | | | | | | | | | Haplotype frequency | | | Empirical p-values^{a} | | |
|---|---|---|---|---|---|---|---|---|---|---|---|---|---|---|---|---|
| S1 | M1 | M2 | S2 | S3 | S4 | S5 | S6 | S7 | S8 | S9 | Controls | BPD | SZ | Combined | BPD | SZ |
| "Risk" haplotypes | | | | | | | | | | | | | | | | |
| | | | | G | | C | | | | | 0,0105 | 0.0937 | 0.0993 | 2.8x10⁻⁰⁷ | 1.6x10⁻⁰⁶ | 1x10⁻⁰⁶ |
| | | | | G | | C | A | | | | 0.0080 | 0.0591 | 0.0667 | 4x10⁻⁰⁵ | 1x10⁻⁰⁴ | 2x10⁻⁰⁴ |
| | | | | G | | C | G | | | | 0.0027 | 0.0258 | 0.0326 | 0,0022 | 0,0018 | 0.0015 |
| | | | | G | G | C | | | | | 0.0105 | 0.0895 | 0.0951 | 1x10⁻⁰⁶ | 3x10⁻⁰⁶, | 4x10⁻⁰⁶ |
| | | | | G | G C | | A | | | | 0.0081 | 0.0611 | 0.0663 | 2x10⁻⁵ | 8x10⁻⁰⁵ | 2x10⁻⁰⁴ |
| | 2 | 3 | C | | | | | | | | <0.0001 | <0.0001 | 0,0577 | 0,0248 | - | 0,0120 |

| "Protective" haplotypes | | | | | | | | | | | | | | | | |
|---|---|---|---|---|---|---|---|---|---|---|---|---|---|---|---|---|
| | | 2 | T | | | | | | | | 0,2743 | 0,0797 | 0,1253 | 1.5x10⁻⁴ | 0,0389 | 5x10⁻⁴ |
| | 1 | | 2 T | | | | | | | | 0,1740 | 0,0442 | 0,0300 | 1.8x10^{-0.4} | 0,0865 | 4.3x10" |
| | 1 | | 5 T | | | | | | | | 0,0585 | 0,0536 | <0.0001 | 0,0134 | 0,6006 | 0,0042 |
| | 1 | | 2 T | G | | | | | | | 0,2120 | 0,0675 | 0,0104 | 4x10⁻⁰⁵ | 0,0811 | 8.4x10⁻⁰⁴ |
| | 1 | | 5 T | G | | | | | | | 0,0458 | 0,0387 | <0.0001 | 0,0207 | 0,5625 | 0,0046 |
| | 1 | 2/5 | T | G | | | | | | | 0,2463 | 0,1672 | 0,0119 | 5.7x10⁻⁰⁶ | 0,0691 | 7.8x10⁻⁰⁶ |
| | | | 2 T | G | G | | | | | | 0,2930 | 0,1330 | 0,0843 | 9.2x10⁻⁰⁵ | 0,0461 | 2.5x10⁻⁰⁴ |
| | | | 5 T | G | G | | | | | | 0,0401 | <0.0001 | <0.0001 | 0,0044 | 0,0334 | 0,0090 |
| | | | | | | A | G | | | | 0.8378 | 0.7830 | 0.7640 | 0.0232 | 0,0658 | 0,0314 |
| | | | | | | A | G | G | | | 0.7369 | 0.6672 | 0.6185 | 0,0114 | 0,0785 | 0,0061 |
| | | | | | | A | G | G | C | | 0.5678 | 0.5072 | 0.3913 | 0,0254 | 0,2936 | 0,0019 |
| | | | | | | A | G | G | C | G | 0.5693 | 0.5038 | 0.3912 | 0,0211 | 0,2452 | 0,0014 |
| | | | T | G | G | A | G | G | C | G | 0.3898 | 0.2554 | 0.2402 | 0.0030 | 0,1486 | 0,0039 |
| | | | T | G | G | A | | | | | 0.6313 | 0.5795 | 0.4809 | 0,0189 | 0,4404 | 0,0100 |
| | | | | G | | A | | | | | 0,8315 | 0.7685 | 0.7666 | 0,0217 | 0,0414 | 0,0683 |

| | | | | | | | | | | | | | | | | |
|---|---|---|---|---|---|---|---|---|---|---|---|---|---|---|---|---|
| ^{a} Empirical haplotype specific p-values based on 100 000 000 permutations using the HTR program. P-values < 0.05 in bold S2 to S9 correpond to SNP rs4468, rs138855, rs2239848, rs138880, rs138881, rs6010260, rs137931, and rs137932 M1 to M2 correpond to D22S922 and D22S1169. | | | | | | | | | | | | | | | | |

**Table 4 Intermarker linkage disequilibrium measured by D'.**

| | Cases above and right of diagonal, controls below and left of diagonal. | | | | | | | | | | | | | | | | |
|---|---|---|---|---|---|---|---|---|---|---|---|---|---|---|---|---|---|
| Gene | SNP | | S1 | M1 | M2 | S2 | S3 | S4 | S5 | S6 | S7 | S8 | S9 | S10 | S11 | S12 | S13 |
| FAM19A5 | rs132234 | (S1) | | 0,05 | 0,06 | 0,12 | 0.53 | 1.00 | 0.39 | <0.01 | 0.09 | 0.08 | 0.10 | 0,04 | 0.03 | 0.14 | 0.27 |
| | D22S922 | (M1) | 0,08 | | 0,13 | 0,02 | 0,44 | 0,12 | 0,07 | 0,06 | 0,21 | 0,02 | 0,00 | 0,00 | 0,07 | 0,02 | 0,06 |
| | D22S1169 | (M2) | 0,13 | 0,22 | | 0,15 | 0,16 | 0,44 | 0,14 | 0,16 | 0,22 | 0,11 | 0,14 | 0,19 | 0,34 | 0,08 | 0,25 |
| BRD1 | rs4468 | (S2) | 0.14 | 0,25 | 0,28 | | 0.84 | 1.00 | 0.95 | 1.00 | 0.06 | 0.42 | 0,42 | 0.04 | 0.15 | 0.06 | 0.15 |
| BRD1 | rs138855 | (S3) | 0.04 | 0,06 | 0,17 | 0.90 | | 0.59 | 0.91 | 0.39 | 0.47 | 0.17 | 0.17 | 0,12 | 0.03 | 0.05 | <0.01 |
| BRD1 | rs2239841 | (S4) | 1.00 | <0.01 | <0.01 | 1.00 | 1.00 | | 1.00 | 0.69 | 1.00 | 0.11 | 0.11 | 0.1 | 0.85 | 0.20 | 1.00 |
| BRD1 | rs138880 | (S5) | 0.08 | 0,09 | 0,20 | 0.92 | 0.94 | 1.00 | | 0.98 | 0.31 | 0.07 | 0.07 | 0.05 | 0.09 | 0.01 | 0.09 |
| BRD1 | rs138881 | (S6) | 0.20 | 0,01 | 0,40 | 0.85 | 0.89 | 1.00 | 1.00 | | 0.04 | 0,19 | 0,19 | 0.07 | 0.07 | 0.12 | 1.00 |
| MLC1 | rs601026 | (S7) | <0.01 | 0,44 | 0,15 | 0.02 | 0.19 | 1.00 | 0.03 | 0.07 | | 1.00 | 1.00 | 0.53 | 1.00 | 0.29 | <0.01 |
| MLC1 | rs137931 | (S8) | 0.30 | 0,10 | 0,29 | 0.45 | 0.01 | 0,01 | 0.06 | 0.23 | 0.97 | | 0.99 | 0.93 | 1.00 | 0.06 | 0.03 |
| MLC1 | rs137932 | (S9) | 0,29 | 0,09 | 0,29 | 0.45 | 0.01 | 0,01 | 0.06 | 0.24 | 0.96 | 1.00 | | 0.93 | 1.00 | 0.06 | 0.04 |
| MOV10L1 | rs381097 | (S10) | 0.31 | 0,13 | 0,16 | 0.05 | 0.53 | 0.04 | 0.70 | 0.71 | 0.54 | 0.87 | 1.00 | | 0.95 | <0.01 | 0.16 |
| MOV10L1 | rs227284: | (S11) | 0.32 | 0,18 | 0,20 | 0.06 | 0.56 | 0,28 | 0.79 | 1.00 | 1.00 | 0.81 | 1.00 | 1.00 | | 0.03 | 0.19 |
| MAPK8IP2 | rs715519 | (S12) | 0.13 | 0,13 | 0,27 | 0.58 | <0.01 | 1.00 | 0.16 | 0.15 | 0.22 | 0.06 | 0.08 | <0.01 | 0.10 | | <0.01 |
| MAPK8IP2 | rs916005 | (S13) | 0,89 | 0,29 | 0,52 | 0.20 | 0.11 | 1.00 | 0.16 | 0.10 | 1.00 | 0.34 | 0.33 | 0.20 | 0.34 | 0.50 | |

| | | | | | | | | | | | | | | | | | |
|---|---|---|---|---|---|---|---|---|---|---|---|---|---|---|---|---|---|
| Significant (P<0.05) D' values >0.7 in bold | | | | | | | | | | | | | | | | | |

### Example 2. Analysis of distantly related patients and unrelated controls from the Faeroe Islands (with focus on GPR24)

### Subjects

Two samples were analyzed, one from the Faeroe Islands and one from Scotland. Informed consent was obtained from all patients prior to inclusion, and the study was approved by the local research ethical committees where the patients were recruited. The patients from the Faeroe Islands are well-documented cases of severe SZ or BPD treated at the Department of Psychiatry, National Hospital, Torshavn and thoroughly interviewed and diagnosed by experienced psychiatrists according to ICD10 diagnostic criteria for research and DSMIV (Jorgensen et al. 2002a). The genealogy of the distantly related 17 individuals with BPD and the 11 individuals with SZ was deduced from information on birth, marriages, and deaths in church and civic records of the Faeroese, and could be tracked back to a common ancestor bom around 1600 (Fig. 3). The average number of generations relating two patients in the genealogically shortest possibly way through one of the parents were six for patients with SZ and seven for patients with BPD. The sample included the cases previously analyzed for shared chromosome 22 segments using microsatellite markers (Jorgensen et al. 2002a) and four additional cases, one patient with SZ and three with BPD. The control group consisted of 44 unrelated persons (22 couples each with a single offspring) from the Faeroe Islands without a history of psychiatric disease. Haplotypes for chromosomal segments consisting of two to four neighboring markers were determined for cases on the basis of either available parental genotypes or genotypes of spouse and a child when available. All controls had their haplotype reconstructed from the genotypes of their offspring. This method will reconstruct the majority of relatively short haplotypes correctly

### (Fig. 3 about here)

The case-control sample from Scotland consisted of 103 patients with SZ, 162 patients with BPD and 200 ethnically matched controls. The cases were diagnosed using SADS-L interview and RDC and DSM-lV criteria (Borglum et al. 2001; Borglum et al. 2003). The controls were from the Blood Transfusion Service, Edinburgh, and were screened to exclude people with serious chronic illness. Genomic DNA was isolated from blood samples according to standard procedures.

### Sequencing and genotyping

Sequencing was carried out using 100 ng of DNA to perform PCR amplification, JETquick PCR Purification kit (Genomed GmbH, www.genomed-dna.com) for purification of the PCR product, and ABI BigDye kit for direct sequencing on an ABl310 Genetic Analyzer (Applied Biosystems, Foster City, CA). Sequences were analyzed in both directions. Genotyping of the selected SNPs was performed using 40 ng of DNA per multiplex PCR, Exonuclease1 and Shrimp Alkaline Phosphatase were used for purification steps and the SNPs were genotyped by multiplex single base extension technology using the ABI SNaP-shot kit and an ABI 310 Genetic Analyzer or a 3100 Avant Genetic Analyzer (Applied Biosystems, Foster City, CA) according to the manufacturer's recommendations. The data was analyzed using ABI 310 GeneScan 3.1.2 (Applied Biosystems, Foster City, CA). Standard PCR conditions were used for both sequencing and genotyping. Scoring of genotypes was performed by two investigators independently and in case of disagreement the sample was re-analyzed.

In order to further control for genotyping errors all SNPs were analyzed twice in at least 50 individuals, the GPR24 SNPs were analyzed twice in 100 individuals. No discordant genotypes were observed, indicating that the error rate was very low (for allele calls less than 0.01).

The microsatellite marker D22S279 was analyzed in the four newly ascertained Faeroese cases and the Scottish sample using fluorescent primers, standard conditions for PCR amplification, and separation of allelic fragments on an ABl310 Genetic Analyzer (Applied Biosystems, Foster City, CA).

### Statistical analysis

### The Faroese population

The data from the 6 polymorphic SNPs genotyped in the Faeroese sample and the new D22S279 genotypes were merged with the D22S279 genotypes produced by Jorgensen et al. (Jorgensen et al. 2002a), and a test of association was performed as implemented in CLUMP (Sham and Curtis 1995). The test is a modification of a Chi-squared test simulating the distribution of the test statistic using a Monte Carlo approach. The program evaluates all alleles or haplotypes in one test and is therefore sensitive to situations where more than one allele or haplotype are more frequent in either of the groups analyzed. In addition the Monte Carlo approach counteracts the invalidation of the asymptotic sampling distribution of the Chi-squared statistics potentially introduced by polymorphic markers. The p-values derived from CLUMP presented in this paper are from the subtests T1 and T4, which are the most reliable parameters when analyzing extended haplotypes. T1 is the standard Pearson χ² statistics of the 2xN contingency table and T4 is obtained by reshuffling alleles or haplotypes of a 2x2 table until χ² has reached a maximum, thereby comparing any combination of alleles or haplotypes with the rest. In the analysis of specific haplotypes, test of associations were performed using Fisher's Exact test.

Classical case-control analysis might detect differences between cases and controls owing to ignored population substructure or improperly accounted relatedness among individuals not necessarily owing to true association between a marker and a trait.

In the present dataset genealogical information is available for cases only (one of several genealogical routes is shown in Fig. 3), while the genealogy for controls remains unknown. In order to get an idea of how related controls are and whether they fall into the same genealogy as cases, we calculated pair-wise estimates of genetic relatedness (r) for all pairs and average relatedness estimates (r*) for pairs within the two groups (cases and controls), but also for case-control pairs. Relatedness or relationship coefficients are defined as the proportion of genes/loci in one individual with alleles identical to these of a reference individual. Estimates of genetic relatedness were calculated using the algorithms developed by Queller & Goodnight (Queller and Goodnight 1989) as implemented in SPAGeDi 1.2 (Hardy and Vekemans 2002). Average within and between group relatedness estimates were obtained using 60 randomly selected unlinked markers, standard errors were obtained by jack-knifing over loci. Parametric t-tests were used to test whether there were significant differences in average relatedness. Pair-wise relatedness coefficients for each pair of individuals were estimated using 660 markers more of less randomly distributed through out the genome (made available from an unpublished study). Using 660 markers of which some would be linked non-independent markers would overestimate the effective number of loci, resulting in underestimation of the variance (standard error). The actual value of relatedness coefficient based on all 660 markers should, however, be very accurate. Estimates of pair-wise genetic distances between individuals were obtained using the algorithm developed by Rousset (Rousset 2000) as implemented in SPAGeDi1.2 (Hardy and Vekemans 2002). Pair-wise genetic distances were used in a multidimensional scaling algorithm (Alscal procedure - as implemented in SPSS 11.5) to map similarity between individuals relative to each other. The population structure including the genetic differentiation within the case-control sample was evaluated by Wright's F-statistics. The 60 unlinked markers were used to calculate the genetic distance between the case and control group using Wright's F_{ST}. Under the hypothesis of no differentiation between the individuals and populations a null distribution of F_{IT}, F_{IS} and F_{ST} values was obtained by performing 3,000 permutations of individual genotypes among all individuals (F_{IT}), among individuals within populations (F_{IS}) and among populations (F_{ST}) as implemented in SPAGEDi 1.2 (Hardy and Vekemans 2002).

### The Scottish population

For each of the seven polymorphic SNPs genotyped in the Scottish sample Chi-square and Fisher's Exact test were used to assess allele and genotype distribution and the program Haplotype Trend Regression (HTR) was used to estimate the frequency and analyze the distribution of haplotypes (Zaykin et al. 2002). When comparing two groups HTR produces an overall p-value for the observed distribution of all the haplotypes of a given segment and in addition a haplotype-specific p-value describing the likelihood of the observed distribution of each of the specific haplotypes. The p-values from HTR presented in this study are empirical values based on 100,000 permutations. In both samples analyzed the controls were compared to individuals with BPD, to individuals with SZ and to the two groups combined. P-values less than 0.05 are referred to as significant. No correction for multiple testing was performed.

Pair-wise linkage disequilibrium was tested using the Slatkin and Excoffier expectation-maximization algorithm (Excoffier and Slatkin 1995) as implemented in Ldmax from the GOLD software package
(http://www.sph.umich.edu/csg/abecasis/GOLD/index.html).

### In silico analyses

The impact of a promoter SNP on potential binding sites for transcription factors was analyzed using the program Matinspector (www.genomatix.de) (Quandt et al. 1995; Wemer 2000). This program utilizes a library of matrix descriptions for transcription factor binding sites to identify potential sites in a sequence analyzed and assign a quality rating of matches (core and matrix similarity) estimating the influence of a SNP on the binding of transcription factors. The possible effect of an intragenic SNP on splicing was investigated using the programs ESEfinder release 2.0 (http://rulai.cshl.edu/tools/ESE) (Cartegni et al. 2003; Quandt et al. 1995; Werner 2000), RESCUE-ESE Web Server (hftp://genes.mit.edu/burgelab/rescue-ese) (Fairbrother et al. 2002) and NNSPLICE (hftp://www.fruitfly.org/seq_tools/splice) (Reese et al. 1997). ESEfinder identifies putative exon splicing enhancers (ESE) responsive to the human SR proteins SF2/ASF, SC35, SRp40 and SRp55. RESCUE-ESE Web Server predicts which sequences have ESE activity by statistical analysis of exon-intron and splice site compositions. NNSPLlCE analyze the structure of the donor and the acceptor sites using a neural network recognizer.

### Results

### SNPs

In search for potential susceptibility variants the coding region (1269 bp in 2 exons), intron-exon boundaries, and the promoter region (500bp upstream to the transcription initiation site) of *GPR24* were sequenced in five individuals with SZ, four with BPD and one control person from the Faeroese sample. Two SNPs (rs133070 and rs133073) were identified. In addition the dbSNP database (hftp://www.ncbi.nlm.nih.gov/SNP) and the genome browser of University of California Santa Cruz (http://www.genome.ucsc.edu/) were used for selection of two additional SNPs in *GPR24,* two SNPs in *ADSL* and two SNPs in *ST13* (Table 1). Eight markers (including D22S279) were genotyped in the sample from the Faeroe Islands and all 10 markers in the Scottish sample. Rs5757921 and rs133071 turned out to be monomorphic and were excluded from further analysis. All SNPs were found to be in Hardy-Weinberg equilibrium in both samples (results not shown).

### (Table 1 about here)

### Association analysis of the Faeroese sample

The Faeroese sample was analyzed using CLUMP comparing the controls to individuals with BPD, to individuals with SZ and to the two groups combined, and significant associations in all 3 groups were observed (Table 2).

Several single markers showed significant association. The three *GPR24* SNPs rs133068, rs133069 and rs133073 showed association with SZ yielding p-values of 0.008-0.02. In BPD and BPD/SZ combined, single marker association was observed for rs909669 *(ADSL)* and rs133070 *(GPR24)* with p-values between 0.0036 and 0.037.

Significantly skewed overall distribution of 2-, 3-, and 4-marker haplotypes involving all four *GPR24* SNPs were found when comparing controls to BPD with T1 p-values as low as 0.0009. The strongest signal was centered on 2-marker haplotypes from rs133069 to rs133073 in *GPR24.* When comparing controls to SZ unequal haplotype distribution was observed for 2-, 3-, 4- and 5-marker haplotypes spanning all four SNPs in *GPR24* in addition to rs909669 in *ADSL* with a 5-marker haplotype showing a T4 p-value of 0.0054. Similarly, comparing controls to the combined group of cases revealed significant associations with 2-, 3-, 4- and 5-marker haplotypes spanning the SNPs in *GPR24* as well as rs909669 in *ADSL.* The strongest signal was observed for the same 2-marker haplotypes as in BPD (minimal p-value of 0.004) and the 4-/5-marker haplotypes yielding maximum signals in SZ (p-values as low as 0.002).

### (Table 2 about here)

The distribution of the specific haplotypes contributing to the overall signal is summarized in Table 3. The overall signal in BPD was strongest for 2- and 3-marker haplotypes involving rs133069 to rs133073. The specific 2-marker haplotype containing the G and C alleles of the *GPR24* SNPs rs133070 and rs133073, respectively, was present in 9% of the chromosomes in bipolar cases and 39% in controls yielding a p-value of 0.0165. Another 2-marker haplotype A-C involving the same SNPs had a frequency of 23% in BPD and 0% in controls (p-value of 0.0048). In SZ the overall signal appeared strongest for 5-marker haplotypes spanning the segment from rs909669 to rs133070. This signal was mainly due to the 5-marker haplotype C7CCA, which was overrepresented in cases. It was present in 90% of the chromosomes in SZ against only 9% in the controls giving a p-value of 5x10⁻⁶. A more modest overall signal appearing in 2-marker haplotypes covering rs909669 to rs133069 was due to part of the same specific 5-marker haplotype described above. The overall signal in the combined group reflected the strongest of the signals in BPD and SZ and correlated to the same specific haplotypes. The specific 5-marker haplotype straddling rs909660 to rs133070 as seen in SZ (C7CCA) showed a p-value of 0.0006 in the combined sample, and together with a variant of this haplotype (C6CCA) the haplotypes C-6/7-CCA were overrepresented in cases with a frequency of 75% against only 22% in controls (p-value of 7x10⁻⁵). Finally, the same specific 2-and 3-marker haplotypes covering rs133069 to rs133073 as seen in BPD showed p-values as low as 0.0183 in the combined sample (Table 3).

### (Table 3 about here)

The within group estimates of relatedness among cases with bipolar affective disorder did not differ significantly from the between group relatedness estimates (r_{bp} = -0.0272 ± 0.0107 vs. r_{bp<>sz+con} = -0.0203 ± 0.0040, t_{17,72} = 0.7102, p = 0.4795), nor did the average relatedness among cases with schizophrenia differ significantly from the between group relatedness estimate (r_{sz} = -0.0223 ± 0.0126 vs. r_{sz<>bp+con} = - 0.0152 ± 0.0045, t_{11,72} = 0.5675, p = 0.5719). Considering the combined dataset of cases; the within group relatedness estimate of cases for both disorder did not differ significantly from the estimated relatedness between the case and control group (r_{bp+sz} = -0,0208 ± 0.0063 vs. r_{bp+sz<>con} = -0.0189 ± 0.0023, t₂₈,₇₂ = 0.3536, p = 0.7244). Individuals within the two case groups (considered separately and together) are therefore not significantly more related to each other than they are to individuals outside the group. However, the within group estimate did differ significantly from the between group estimate for controls (r_{con} = -0.0059 ± 0.0041 vs. r_{con-b<>+sz} = -0.0189 ± 0.0023, t₄₄,₇₂ = 2.9902, p = 0.0034), indicating that controls are in fact on average more related to each other than they are to individuals outside the group.

Overall within group estimated relatedness did not differ significantly from the average between group relatedness estimate (r_{within group} = -0.0143 ± 0.0005 vs. r_{between} groups = -0.0185 ± 0.0024, t_{72,72} = 1.7132, p = 0.0889).

Multidimensional scaling of pair-wise genetic distances (Rousset 2000) between individuals did not reveal an overall clustering of cases in relation to controls (results not shown). Based on the pair-wise estimates of relatedness or genetic distance between each pair of individuals, some individuals appeared to be more related than the population average, thus sharing more alleles than expected based on the population allele frequencies. This was, however, not consistently within groups. The amount of genetic differentiation (F_{ST}) between cases and controls was not statistically significant (F_{ST} = 0.0014, P_{two-talled} = 0.4595, 3,000 permutations). Likewise there was no evidence for inbreeding within individuals neither relative to the total sample nor relative to subgroups (F_{IT} = -0.0008, P_{two-talled} = 0.9180; F_{IS} = - 0.0022; P_{two-talled} = 0.8121, 3,000 permutations, when cases and controls are considered as two subpopulations). Combining the two case groups did, however, reveal genetic differentiation among the case groups and the control group (F_{ST} = 0.0034, P_{two-talled} = 0.0330; F_{TT} = 0.0004, P_{two-talled} = 0.9540; F_{IS} = -0.0030, P_{two-tailed} = 0.7414, 3,000 permutations), increasing the risk for false-positive findings when combining the two disorders.

### Association analysis of the Scottish case-control sample

In the Scottish sample only D22S279 showed significant single marker association with SZ, while haplotype analysis revealed significant associations in both disorders (Table 4). In BPD a minimal overall p-value of 0.0003 was observed for haplotypes including all four *GPR24* SNPs. In SZ the maximal signal was slightly more proximal, including 2 *GPR24* SNPs and D22S279 (p=0.0005). In the combined group of cases similar but less significant associations were observed.

### (Table 4 about here)

Some of the "risk" haplotypes identified in the Faeroese sample were also found over-represented among the Scottish patients (Table 5). These (C)7CCA(T) haplotypes were predominantly over-represented in Faeroese SZ and in the Scottish BP, while the related (C)2CCA haplotypes, which were not present in the Faeroese population, were over-represented among Scottish SZ patients. The rest of the individual haplotypes found associated in the Scottish sample differed from those identified in the Faeroese sample. For example the 2-marker haplotypes containing either the 2, 4 or 8 allele of D22S279 in conjunction with the *GPR24* rs133068 G-allele, which had a combined frequency of 9.3% in SZ versus 1.5% in controls (p=9.8x10⁻⁵).

### (Table 5 about here)

### Linkage disequilibrium

A high degree of intermarker linkage disequilibrium (LD) was observed especially between the closely located SNPs in *GPR24,* extending to some degree to the more distal *ST13* SNP rs710193 in the Scottish sample, and in the Faeroese sample centromeric to *ADSL* (Table 6).

### (Table 6 about here)

### In silico analysis

Using the Matinspector (www.genomatix.de) the different alleles of the four promoter SNPs (Table 1) were analyzed for potential effects on binding sites for transcription factors. The C allele of the *GPR24* SNP rs133068 introduced a binding site for the transcription factor ZBP-89 whereas the G allele introduced a binding site for X-box binding protein RFX1. In both cases a high core- and matrix similarity were seen. The C allele of rs133069 introduced binding sites for SP1, TGFbeta, RREB1, ZBP-89 and ZIC2 of which especially ZBP-89 and ZIC2 showed high core and matrix similarity. Particularly the transcription factor ZIC2 is interesting since the ZIC genes play an important role in neural development (Aruga 2004). For rs133070 and rs909669 alternative alleles did not cause any changes.

The synonymous *GPR24* SNP rs133073 was analyzed for effects on exon splicing enhancers (ESE) and donor and acceptor sites. ESEfinder identified a single potential exon splicing enhancer that only appeared when the T allele was present, creating a binding site for the splicing factor SRp40 with the score 3.3, which is just above the threshold of 2.67 indicating a significant score.

**Table 1 Genotyped polymorphisms and allele frequencies in samples from the Faeroe Islands and Scotland.**

| | | | | | MAF, Faeroe Islands | | | MAF, Scotland | | |
|---|---|---|---|---|---|---|---|---|---|---|
| Gene | Marker | Location (Mb)^{a} | Type of marke | Alleles^{b} | Controls | BPD | SZ | Controls | BPD | SZ |
| *ADSL* | rs909669 | 39.066917 | Promoter | C/T | 0.15 | 0.00 | 0.00 | 0.10 | 0.11 | 0.15 |
| *ADSL* | rs5757921 | 39.067154 | Nonsyn | G/A | NG | NG | NG | 0.00 | 0.00 | 0.00 |
| | D22S279 | 39.347314 | Microsattelite | | - | - | - | - | - | - |
| *GPR24* | rs133068 | 39.398907 | Promoter | C/G | 0.45 | 0.42 | 0.15 | 0.48 | 0.47 | 0.49 |
| *GPR24* | rs133069 | 39.398962 | Promoter | C/A | 0.48 | 0.59 | 0.15 | 0.49 | 0.47 | 0.48 |
| *GPR24* | rs133070 | 39.399273 | Promoter | A/G | 0.44 | 0.16 | 0.22 | 0.42 | 0.42 | 0.39 |
| *GPR24* | rs133071 | 39.399732 | Promoter | C/T | 0.00 | 0.00 | 0.00 | 0.00 | 0.00 | 0.00 |
| *GPR24* | rs133073 | 39.400195 | Synonymous | T/C | 0.50 | 0.41 | 0.19 | 0.41 | 0.45 | 0.41 |
| *ST13* | rs710193 | 39.547690 | Nonsyn | C/T | 0.20 | 0.16 | 0.22 | 0.10 | 0.12 | 0.13 |
| *ST13* | rs1573745 | 39.565335 | Intron | G/A | NG | NG | NG | 0.07 | 0.05 | 0.10 |

| | | | | | | | | | | |
|---|---|---|---|---|---|---|---|---|---|---|
| ^{a} According to the UCSC Genome Browser, May 2004 assembly (http://www.genome.ucsc.edu) ^{b} Major allele/minor allele on the +strand (http://www.genome.ucsc.edu) Nonsyn=Nonsynonymous SNP, MAF= Minor allele frequency, NG=Not genotyped | | | | | | | | | | |

**Table 2 T1 and T4 p-values from CLUMP association analysis of the Faeroese sample.**

| | | Single marker | | 2-marker | | 3-marker | | 4-marker | | 5-marker | |
|---|---|---|---|---|---|---|---|---|---|---|---|
| Gene | Marker | T1 | T4 | T1 | T4 | T1 | T4 | T1 | T4 | T1 | T4 |
| BPD+SZ | | | | | | | | | | | |
| *ADSL* | rs909669 | **0.0036** | **0.0036** | | | | | | | | |
| | D22S279 | 0.0705 | **0.0268** | 0.0805 | 0.0501 | | | | | | |
| *GPR24* | rs133068 | 0.1461 | 0.1461 | 0.1818 | 0.1270 | **0.4823** | 0.3996 | | | | |
| *GPR24* | rs133069 | 0.0740 | 0.0740 | 0.0716 | 0.1107 | 0.2017 | 0.2023 | 0.2672 | 0.2064 | | |
| *GPR24* | rs133070 | **0.0116** | **0.0116** | **0.0145** | 0.0828 | 0.0525 | 0.1831 | **0.0064** | **0.0023** | **0.0059** | **0.0031** |
| *GPR24* | rs133073 | 0.0546 | 0.0546 | **0.0040** | **0.0188** | **0.0268** | 0.0626 | **0.0479** | 0.1278 | **0.0063** | **0.0023** |
| *ST13* | rs710193 | 0.7631 | 0.7631 | 0.4522 | 0.4361 | 0.1158 | 0.1697 | 0.1755 | 0.2417 | 0.1722 | 0.239 |

| SZ | | | | | | | | | | | |
|---|---|---|---|---|---|---|---|---|---|---|---|
| *ADSL* | rs909669 | 0.1178 | 0.1178 | | | | | | | | |
| | D22S279 | **0.0322** | **0.0174** | 0.0447 | **0.008** | | | | | | |
| *GPR24* | rs133068 | **0.0197** | **0.0197** | 0.0542 | 0.0476 | 0.0990 | **0.0353** | | | | |
| *GPR24* | rs133069 | **0.0100** | **0.0100** | 0.0469 | 0.0353 | 0.1870 | 0.1727 | 0.1481 | **0.0495** | | |
| *GPR24* | rs133070 | 0.2107 | 0.2107 | 0.3299 | 0.3299 | 0.4456 | 0.4456 | **0.0426** | **0.0367** | **0.0156** | **0.0054** |
| *GPR24* | rs133073 | **0.0083** | **0.0083** | 0.5104 | 0.5104 | 0.3269 | 0.3269 | 0.4486 | 0.4486 | **0.0432** | **0.0375** |
| *ST13* | rs710193 | 0.7065 | 0.7065 | 0.3844 | 0.3368 | 0.3917 | 0.5842 | 0.3957 | 0.5862 | 0.3932 | 0.5852 |

| BPD | | | | | | | | | | | |
|---|---|---|---|---|---|---|---|---|---|---|---|
| *ADSL* | rs909669 | **0.0373** | **0.0373** | | | | | | | | |
| | D22S279 | 0.3548 | 0.2390 | 0.4799 | 0.3873 | | | | | | |
| *GPR24* | rs133068 | 0.8365 | 0.8365 | 0.4893 | 0.5473 | 0.8745 | 0.7353 | | | | |
| *GPR24* | rs133069 | 0.6815 | 0.6815 | 0.6484 | 0.5936 | 0.5063 | 0.4663 | 0.7976 | 0.5424 | | |
| *GPR24* | rs133070 | **0.0195** | **0.0195** | **0.0030** | **0.0159** | **0.0316** | **0.0972** | 0.1531 | 0.1076 | 0.2957 | 0.1557 |
| *GPR24* | rs133073 | 0.4217 | 0.4217 | **0.0009** | **0.0093** | **0.0042** | **0.0303** | **0.0433** | 0.0997 | 0.1521 | 0.1056 |
| *ST13* | rs710193 | 1.0000 | 1.0000 | 0.8264 | 0.8600 | 0.0850 | 0.1940 | 0.1753 | 0.2821 | 0.1745 | 0.2831 |

| | | | | | | | | | | | |
|---|---|---|---|---|---|---|---|---|---|---|---|
| P-values < 0.05 in bold | | | | | | | | | | | |

**Table 3 Distribution of selected individual haplotypes from CLUMP analysis of the sample from the Faeroe Islands.**

| Haplotype | | | | | | Haplotype frequency | | | | P-values | | |
|---|---|---|---|---|---|---|---|---|---|---|---|---|
| S1 | M | S2 | S3 | S4 | S5 | Controls | BPD | SZ | BPD+SZ | BPD | SZ | BPD+SZ |
| "Risk" haplotypes | | | | | | | | | | | | |
| C | 7 | C | C | A | | 0.09 | 0.28 | 0.90 | 0.50 | 0.1179 | **5x10⁻⁶** | **0.0006** |
| C | 6 | C | C | A | | 0.13 | 0.39 | 0.00 | 0.25 | **0.0406** | 0.5569 | 0.3176 |
| C | 6/7 C | | C | A | | 0.22 | 0.67 | 0.90 | 0.75 | **0.0026** | **0.0002** | **7x10⁻⁵** |
| | 7 | C | C | A | T | 0.15 | 0.39 | 0.64 | 0.44 | 0.2868 | **0.0013** | **0.0139** |
| | 6 | C | C | A | T | 0.12 | 0.28 | 0.14 | 0.28 | **0.0340** | 1.0000 | 0.2166 |
| | 6/7 | C | C | A | T | 0.26 | 0.67 | 0.78 | 0.72 | **0.0076** | **0.0013** | **0.0005** |
| | | | | A | C | 0.00 | 0.23 | 0.00 | 0.14 | **0.0048** | 1.0000 | **0.0234** |

| "Protective" haplotypes | | | | | | | | | | | | |
|---|---|---|---|---|---|---|---|---|---|---|---|---|
| | | | A | G | | 0.38 | 0.08 | 0.22 | 0.16 | **0.0393** | 0.3279 | **0.0364** |
| | | | A | G | C | 0.38 | 0.10 | 0.21 | 0.15 | **0.0307** | 0.3279 | 0.0526 |
| | | | | G | C | 0.39 | 0.09 | 0.21 | 0.14 | **0.0165** | 0.3286 | **0.0183** |

**Table 4 Single marker and haplotype association analysis in the case-control sample from Scotland**

| | | | Empirical overall p-values ^{a} | | | |
|---|---|---|---|---|---|---|
| Gene | SNP | Single marker | 2-marker | 3-marker | 4-marker | 5-marker |
| BPD+SZ | | | | | | |
| *ADSL* | rs909660 | 0,2189 | | | | |
| *D22S279* | | 0,4644 | **0,0490** | | | |
| *GPR24* | rs133068 | 0,8538 | 0,1821 | **0,0319** | | |
| *GPR24* | rs133069 | 0,7126 | 0,1791 | 0,3066 | 0,0591 | |
| *GPR24* | rs133070 | 0,6721 | 0,4592 | 0,5945 | 0,3363 | 0,3390 |
| *GPR24* | rs133073 | **0,5632** | **0,0262** | **0,0155** | **0,0184** | 0,1747 |
| *ST13* | rs710193 | 0,2317 | 0,1247 | 0,0715 | 0.0790 | 0,1895 |
| *ST13* | rs1573745 | 0,7989 | 0,4429 | 0,0632 | 0,1346 | 0,2604 |

| SZ | | | | | | |
|---|---|---|---|---|---|---|
| *ADSL* | rs909660 | **0,0655** | | | | |
| *D22S279* | | **0,0277** | **0,0012** | | | |
| *GPR24* | rs133068 | 0,8145 | **0,0054** | **0,0015** | | |
| *GPR24* | rs133069 | 0,8734 | 0,1238 | **0,0005** | **0,0012** | |
| *GPR24* | rs133070 | 0,3638 | 0,0618 | 0,0691 | **0,0053** | **0,0497** |
| *GPR24* | rs133073 | 0,8469 | 0,1879 | **0,0148** | 0,0135 | **0,0189** |
| *ST13* | rs710193 | 0,2758 | 0,3269 | 0.2240 | 0,1069 | 0.1460 |
| *ST13* | rs1573745 | 0,2879 | 0,1394 | 0,2105 | 0,2465 | 0,1871 |

| BPD | | | | | | |
|---|---|---|---|---|---|---|
| *ADSL* | rs909660 | 0,6833 | | | | |
| *D22S279* | | 0,5453 | 0,0580 | | | |
| *GPR24* | rs133068 | 0,6757 | 0,6295 | 0,3278 | | |
| *GPR24* | rs133069 | 0,6748 | 0.3582 | 0,7233 | 0,5948 | |
| *GPR24* | rs133070 | 0,9373 | 0.1855 | 0.0779 | 0,3612 | 0,4471 |
| *GPR24* | rs133073 | 0.3470 | 0.1471 | **0,0053** | **0.0003** | **0,0410** |
| *ST13* | rs710193 | 0,3292 | 0.1197 | 0.2251 | **0.0252** | **0,0018** |
| *ST13* | rs1573745 | 0,1588 | 0.5903 | 0.1230 | 0.3998 | 0.1587 |

| | | | | | | |
|---|---|---|---|---|---|---|
| ^{a} Empirical overall p-values based on 100,000 permutations using the HTR program. P-values < 0.05 in bold. | | | | | | |

**Table 5**

| Distribution of selected individual haplotypes in the case-control sample from Scotland. | | | | | | | | | | | | |
|---|---|---|---|---|---|---|---|---|---|---|---|---|
| Haplotype | | | | | | | Haplotype frequency | | | Empirical p-values ^{a} | | |
| S1 | M | S2 | S3 | S4 | S5 | S6 | Controls | BPD | SZ | BPD | SZ | BPD+SZ |
| "Risk" haplotypes | | | | | | | | | | | | |
| C | 7 | C | C | A | | | 0.1564 | 0.2459 | 0.1783 | 0.0520 | 0.8847 | 0.1120 |
| | 7 | C | C | A | | | 0.1560 | 0.2518 | 0.1660 | **0.0221** | 0.8869 | 0.1040 |
| | 7 | C | C | A | T | | 0.1663 | 0.2470 | 0.1659 | **0.0499** | 0.9698 | 0.1966 |
| C | 2 | | | | | | 0.0115 | <0.0001 | 0.0909 | 0.2349 | **0.0005** | 0.1118 |
| | 2 | C | C | A | | | 0.0039 | <0.0001 | 0.0604 | 0.4531 | **0.0022** | 0.1005 |
| | 8 | G | | | | | 0.0037 | 0.0173 | 0.0219 | 0.0691 | **0.0229** | 0.0559 |
| | 2 | G | | | | | 0.0074 | <0.0001 | 0.0434 | 0.1632 | **6.90x10-4** | 0.1366 |
| | 4 | G | | | | | 0.0037 | 0.0038 | 0.0287 | 1.0000 | **0.0247** | 0.3235 |
| | 2/4/8 | G | | | | | 0.0148 | 0.0208 | 0.0934 | 0.6185 | **9.78×10-5** | **0.0152** |
| | 2/4/8 | G | A | | | | 0.0147 | 0.0208 | 0.0942 | 0.6200 | **9.40x10-5** | **0.0150** |
| T | 5 | | | | | | <0.0001 | 0.0355 | 0.0405 | **0.0041** | **0.0025** | **0.0058** |
| T | 5 | G | | | | | <0.0001 | 0.0180 | 0.0246 | **0.0164** | **0.0134** | 0.0181 |
| T | 5 | G | A | | | | <0.0001 | 0.0179 | 0.0250 | **0.0191** | **0.0136** | **0.0179** |
| T | | G | A | G | | | 0.0169 | 0.0621 | 0.0380 | **0.0474** | 0.3964 | **0.0330** |
| T | | G | A | G | C | | 0.0129 | 0.0619 | 0.0236 | **0.0296** | 0.4308 | **0.0263** |

| "Protective" haplotypes | | | | | | | | | | | | |
|---|---|---|---|---|---|---|---|---|---|---|---|---|
| | | G | A | G | T | | 0.0202 | <0.0001 | <0.0001 | **0.0257** | 0.0675 | **0.0006** |
| | | | A | G | T | | 0.0202 | <0.0001 | <0.0001 | **0.0264** | 0.0683 | **0.0006** |
| | | | | G | T | | 0.0254 | 0.0041 | <0.0001 | **0.0252** | **0.0279** | **0.0050** |
| | | G | A | A | T | | 0.0537 | 0.0082 | 0.0897 | **0.0021** | 0.0841 | 0.4945 |
| | | G | A | A | | | 0.0522 | 0.0162 | 0.0977 | **0.0256** | **0.0442** | 0.9692 |
| | | | A | A | T | | 0.0537 | 0.0165 | 0.0949 | **0.0247** | 0.0534 | 0.8646 |
| C | 3 | | | | | | 0.2634 | 0.2675 | 0.1704 | 0.8912 | **0.0314** | 0.2608 |
| C | 5 | | | | | | 0.1718 | 0.1531 | 0.0958 | 0.7527 | **0.0534** | 0.2421 |
| C | 3/5 | | | | | | 0.4353 | 0.4203 | 0.2662 | 0.7444 | **0.0018** | 0.0750 |
| C | 5 | G | A | | | | 0.1614 | 0.1307 | 0.0719 | 0.5996 | **0.0247** | 0.1250 |
| C | 3/5 | G | | | | | 0.3199 | 0.3208 | 0.2080 | 0.9939 | **0.0204** | 0.2432 |
| | 3/5 | G | | | | | 0.3339 | 0.3454 | 0.2256 | 0.8470 | **0.0351** | 0.3597 |

| | | | | | | | | | | | | |
|---|---|---|---|---|---|---|---|---|---|---|---|---|
| ^{a} Empirical overall p-values based on 100,000 permutations using the HTR program. P-values < 0.05 in bold. S1 to S6 correpond to SNP: rs909669, rs133068, rs133069, rs133070, rs133073, rs710193. M= D22S279. | | | | | | | | | | | | |

**Table 6 Intermarker linkage disequilibrium measured by D'.**

| Cases above and right of diagonal, controls below and left of diagonal. | | | | | | | | | | |
|---|---|---|---|---|---|---|---|---|---|---|
| Gene | SNP | | S1 | M | S2 | S3 | S4 | S5 | S6 | S7 |
| Faeroese sample | | | | | | | | | | |
| *ADSL* | rs909669 | (S1) | | 0.00 | 0.00 | 0.00 | 0.00 | 0.00 | 0.00 | |
| | D22S279 | **(M)** | 0.30 | | **0.73** | **0.73** | **0.82** | **0.84** | 0.54 | |
| *GPR24* | rs133068 | (S2) | 0.68 | 0.43 | | **1.00** | **1.00** | **0.92** | 0.41 | |
| *GPR24* | rs133069 | (S3) | **0.72** | 0.52 | **1.00** | | **1.00** | **0.91** | 0.41 | |
| *GPR24* | rs133070 | (S4) | **1.00** | 0.46 | **1.00** | **1.00** | | **1.00** | 0.36 | |
| *GPR24* | rs133073 | (S5) | **0.75** | 0.58 | **0.94** | **1.00** | **1.00** | | 0.39 | |
| *ST13* | rs710193 | (S6) | **1.00** | 0.43 | 0.61 | 0,61 | **1.00** | 0.64 | | |

| Scottish sample | | | | | | | | | | |
|---|---|---|---|---|---|---|---|---|---|---|
| *ADSL* | rs909669 | (S1) | | 0,32 | 0.10 | 0.11 | 0.14 | 0.01 | 0.05 | 0.20 |
| | D22S279 | **(M)** | 0,46 | | 0,38 | 0,37 | 0,48 | 0,46 | 0.23 | 0,23 |
| *GPR24* | rs133068 | (S2) | 0,19 | 0,46 | | **0.95** | **0.93** | **0.92** | 0.58 | 0.60 |
| *GPR24* | rs133069 | (S3) | 0.20 | 0,46 | **0,99** | | **0.89** | **0.89** | 0.56 | 0.60 |
| *GPR24* | rs133070 | (S4) | 0,58 | 0,55 | **0,94** | **0,95** | | **0.99** | 0.51 | **0.85** |
| *GPR24* | rs133073 | (S5) | 0,55 | 0,61 | **0,91** | **0,92** | **0.95** | | 0.48 | **0.86** |
| *ST13* | rs710193 | (S6) | 1.00 | 0,70 | **0.90** | **0,89** | **0.90** | **0.90** | | 0.63 |
| *ST13* | rs1573745 | (S7) | 0,28 | 0,55 | 0,42 | 0,43 | 0,49 | 0,61 | **1.00** | |

| | | | | | | | | | | |
|---|---|---|---|---|---|---|---|---|---|---|
| Significant (P<0.05) D' values >0.7 in bold | | | | | | | | | | |

### Example 3

### Association in three different case-control samples

### Subjects

Three Caucasian case-control samples from Scotland, UK and Denmark respectively were analyzed. All patients gave informed consent prior to inclusion, and study approval was obtained from the local research ethical committees where the patients were recruited.

The case-control sample from Scotland consisted of 103 patients with SZ, 162 patients with BPD and 200 ethnically matched controls. The subjects suffering from SZ or BPD were interviewed using the Schizophrenia and Affective Disorder Schedule-Lifetime Schedule (SADS-L)⁴⁰ (SADS-L) interview and diagnosed according to RDC and DSM-IV criteria.^{41,42} The controls were from the Blood Transfusion Service, Edinburgh, and were screened to exclude people with serious chronic illness.^{43,44}

The UK sample consisted of 300 individuals with BPD, 265 individuals with SZ and 314 screened normal controls. The BPD cases and controls were included if both parents and all four grandparents were of Irish, Welsh, Scottish or English ancestry as defined by an ancestry checklist. In the selection of the SZ cases one of the grand parents was allowed to be of Caucasian European origin but not of Jewish or non-European Union ancestry (EU countries before the 1994 enlargement). All subjects, were interviewed with the SADS-L by a psychiatrist and diagnosed according to RDC criteria. Cases with bipolar disorder were all bipolar 1 disorder. In the SZ group patients with schizoaffective bipolar disorder or schizo-mania were not included. The controls were selected on the basis of not having a family history of schizophrenia, alcoholism or bipolar disorder and for not having a past or present personal history of any defined mental disorder.⁴⁵

The Danish sample consisted of 124 bipolar patients, 115 individuals suffering from schizophrenia and 96 ancestrally matched unscreened controls. Only patients with an age of onset below 35 were included. Cases were interviewed using the semi-structured interview SCAN version 2.1.⁴⁶ and best estimate diagnoses according to ICD-10-DCR⁴⁷ and DSM-IV⁴⁸ were made by two psychiatrists.. All bipolar cases met the ICD-10 -DCR diagnostic criteria for bipolar affective disorder and bipolar I disorder (DSM-IV).

The 115 individuals suffering from schizophrenia were first ever admitted cases fulfilling DSM-IV and ICD-10-DCR criteria for schizophrenia.

### Genotyping and sequencing

Genomic DNA was isolated from blood samples according to standard procedures. Sequencing was carried out using 100 ng of DNA to perform PCR amplification, JETquick PCR Purification kit (Genomed GmbH, www.genomed-dna.com) for purification of the PCR product, and ABI BigDye kit for direct sequencing on an ABI310 Genetic Analyzer (Applied Biosystems, Foster City, CA). Sequences were analyzed in both directions.

Genotyping was carried out using the ABI SNaP-shot kit (Applied Biosystems, Foster City, CA), an ABI 310 Genetic Analyzer or a 3100 Avant Genetic Analyzer (Applied Biosystems, Foster City, CA), and the program ABI 310 GeneScan 3.1.2 (Applied Biosystems, Foster City, CA). Primer sequences were obtained from dbSNP (www.ncbi.nim.nih.-gov/SNP). PCR was performed with up to 5 primer sets simultaneously using 40 ng of DNA and standard PCR conditions (primer sequences and PCR conditions are available on request). The PCR products were treated with Exonuclease1 and Shrimp Alkaline Phosphatase, and the multiplex single base extension was carried out according to the manufacturer's recommendations (Applied Biosystems, Foster City, CA).

To control for genotyping errors all SNPs were scored independently by two investigators. Any discordances lead to re-analysis of the sample. Furthermore a number of SNPs (including *SERHL* rs881542) were analyzed twice in at least 85 individuals. No divergent genotypes were observed, indicating a very low error rate.

### Statistical analysis

The data from the polymorphic SNPs genotyped in the samples from Scotland, Denmark, and the UK were analyzed using the program Haplotype Trend Regression (HTR), which estimate the frequency and the distribution of single markers and haplotypes.⁴⁹ When comparing two groups HTR produce an overall p-value for the observed distribution of all the different haplotypes. In addition HTR produce a haplotype-specific p-value describing the likelihood of the observed distribution of each of the specific haplotypes. In this study the empirical p-values presented are based on up to 100,000,000 permutations. In the analyses performed the controls were compared to individuals with BPD, to individuals with SZ as well as to these groups combined. P-values less than 0.05 are referred to as significant. No correction for multiple testing was performed. Hardy Weinberg equilibrium for the individual SNPs was analyzed using χ².

Analyzes of linkage disequilibrium (LD) was performed using the program Ldmax from the GOLD software package (http://www.sph.umich.edu/csg/abecasis/GOLD/index.html).^{50,51}

### In silico analyses

MatInspector (www.genomatix.de)^{52,53} was used to assess and analyze the impact of promoter SNPs on potential binding sites for transcription factors. The possible effect of intragenic SNPs on splicing was investigated using the programs ESEfinder release 2.0 (hftp://rulai.cshl.edu/tools/ESE),⁵²⁻⁵⁴ RESCUE-ESE Web Server,
(http://genes.mit.edu/burgelab/rescue-ese),⁵⁵ FAS-ESS web server
(http://genes.mit.edul/fas-ess/),⁵⁶ ExonScan Web Server
(hftp://genes.mit.edu/exonscan/),⁵⁶
and NNSPLICE (http://www.
fruitfly.org/seq_tools/splice).⁵⁷
The effect of 3' UTR SNPs on microRNA binding sites were analyzed using the miRBase Targets Pre-release Version 1.0 (http://microma.sanger.ac.uk/targets/v1/), a web resource provided by the Wellcome Trust Sanger Institute containing computationally predicted targets for microRNAs across a number of species.⁵⁸

### Results

### SNPs

In search for susceptibility variants sequencing of the coding region, intron-exon boundaries, and the promoter region (500bp upstream to the transcription initiation site) of the gene *PIPPIN* was carried out in five individuals with SZ, four with BPD and one control person from the Faeroese sample. We identified a single polymorphism in the promoter region (rs6002408). In addition the dbSNP database (hftp://www.ncbi.nim.nih.gov/SNP) and the genome browser of University of California Santa Cruz (http://www.genome.ucsc.edu/) were used for selection of 11 SNPs in *EP300, PIPPIN, NHP2L1,* and *SERHL* (table 1). A total of 12 SNPs were analyzed. All SNPs were found to be in Hardy-Weinberg equilibrium in all samples. Rs5758252 and rs1802521 were monomorphic and excluded from statistical analyses. Method problems, and uncertain genotyping caused rs2294976, rs60002408, rs132806, and rs5758405 to be excluded from the statistical analyses of the Scottish sample, and rs60002408 to be excluded from statistical analysis in the replication samples from Denmark and the UK.

### Association analysis.

In the Scottish sample three SNPs showed a significant single marker association; rs20551 (*EP300*) was associated with BPD (p-value=0.0132), rs926333 (*SERHL*) was associated with SZ (p-value=0.0131), and rs1006407 (*PIPPIN*) was associated with SZ and BPD combined (p-value=0.049) (table 2). In the overall haplotype analysis the strongest association was observed when comparing controls to cases combined. 2-, 3-, 4-, and 5-marker haplotypes was associated with both SZ and BPD with the most significant signal centered on rs8779 (*NHP2L1*). Especially the specific 2-marker haplotype rs1006407-T / rs8779-A, was underrepresented in cases (0.7%) compared to controls (5%) yielding a p-value of 4.17x10⁻⁶ (table 3).

In the UK sample rs8779 showed significant association with BPD, SZ, and cases combined (p-values of 0.0044, 0.0149, and 0.0024 respectively). Rs926333 was found to be associated with BPD and cases combined (table 4). In the overall haplotype analysis the association was almost entirely found in BPD centered on the 2-marker haplotype rs1006407-rs8779 and 3-, 4-, and 5-marker centromeric extensions of this haplotype (table 4). Analysis of the individual haplotypes revealed that rs1006407-T / rs8779-A (the same haplotype as original was identified in the Scottish sample), had a frequency of 2.8% in the controls, and a frequency of 13.6% in cases suffering from BPD (table 5) (which is skewed in the opposite direction compared to the Scottish sample) yielding a haplotype specific p-value of 1.36x10⁻⁹. The haplotype rs1006407-T/rs8779-G in contrary was underrepresented in cases suffering from BPD (68.7%) compared to controls (80.2%) producing a haplotype specific p-value of 1.93x10⁻⁵.

Additionally two modest overall significant results were found involving the SNPs in *NHP2L1* and in *SERHL* (table 4), with several individual haplotypes generating the association.

In the Danish sample rs881542 (*SERHL*) showed single marker association with SZ, BPD and cases combined whereas rs5758405 (*NHP2L1*) and rs1006407 was found to be associated with BPD and cases combined. Rs8779 showed a significant association with BPD (table 6).

The haplotype analysis produced the most significant associations when comparing BPD to controls and less though still significant association when comparing SZ to controls. The strongest signal was centered on 2-, to 5-marker haplotypes associated with BPD, SZ, as well as theses disorders combined, involving primarily the three SNPs rs5758405 (*NHP2L1*), rs881542 and rs926333 (both *SERHL*). The specific haplotypes causing this association were rs5758405-A /rs881542-C/rs926333-G (and derivates of this haplotype), which was overrepresented in cases (72%) compared to controls (56%), and rs881542-G/rs926333-G (and derivates of this haplotype), which was overrepresented in controls (23%) compared to cases (13%), yielding haplotype specific p-values of 1.49x10⁻⁴ and 1.2.x10⁻³ respectively.

As in the samples from Scotland and UK the overall 2-marker haplotype rs1006407/rs8779 showed association with BPD and SZ. The specific haplotype rs1006407-T/rs8779-A was in this sample significantly overrepresented in SZ (12.8%) compared to controls (2.12%)(p-value=7.82x10-4) whereas the haplotype was not found in BPD at all (table 7).

Combining the original case-control sample from Scotland with the replication samples from Denmark and UK produced overall and haplotype specific associations similar to the signals found in the samples separately. Furthermore a number of haplotypes were found to have a consistently skewed frequency across all three samples (table 8).

### In silico analysis

Using Matinspector (www.genomatix.de) the *SERHL* promoter SNP rs881542 (table 1) was analyzed for effects on potential binding sites for transcription factors. The program suggested that the rare G-allele introduced new binding sites for the transcription factors Collagen krox protein (zink finger protein zfp67) shown to be involved in regulation of type 1-, and 2 collagen gene transcription ^{59,60} and Myc associated zing finger protein (MAZ), a transcription factor closely related to SP1 and involved in regulation of numerous genes.⁶¹

The non-synonymous *EP300* SNP rs20551 located in exon 15 and the *SERHL* SNP rs926333 located in exon 2 were analyzed for effects on exon splicing enhancers (ESE), exon splice silencers (ESS) and donor and acceptor sites. FAS-ESS identified a site for a potential exon splice silencer introduced by the G-allele of rs20551 but analysis of the splice sites revealed strong donor and acceptor site suggesting a very limited potential effect on splicing of this site. The ExonScan analysis of rs20551 confirmed this by finding no other potential splicing variation of exon 15 and hardly any difference in the summed splicing score introduced by the G-allele of rs20551. According to NNSPLICE the *NHP2L1* intron SNP rs5758405 had no effect on potential donor or acceptor sites in intron 1. Likewise the *EP300* intron SNPs rs2294976 (*EP300*) and rs2076578 (*EP300*) had no effect on splicing.

MiRBase Targets Pre-release Version 1.0 identified no changes in miRNA binding sites in the 3'UTR sequences of *PIPPIN* or *NHP2L1.*

### Linkage Disequilibrium

In general the same LD pattern was observed in cases and in controls in the three different samples (table 9). A high degree of LD was observed between SNPs located in *EP300, PIPPIN,* and *NHP2L1.* A limited level of LD was observed between SNPs located in *SERHL* and SNPs in *EP300, PIPPIN* and *NHP2L1* indicating that *SERHL* could be located in another haplotype block. The results was confirmed by data retrieved from The International HapMap Project (http://www.hapmap.org/) in which *EP300, PIPPIN* and *NHP2L1* in the CEU population were located in a 780 kb loosely defined haplotype block, while *SERHL* was located in a clearly distinctive more telomeric block.

### References:

1. 22, S.C.L.G.f.C. A transmission disequilibrium and linkage analysis of D22S278 marker alleles in 574 families: further support for a susceptibility locus for schizophrenia at 22q12. Schizophrenia Collaborative Linkage Group for Chromosome 22. Schizophr Res 32, 115-21 (1998).
2. Aamantidis A, Thomas E, Foidart A, Tyhon A, Coumans B, Minet A, Tirelli E, Seutin V, Grisar T, Lakaye B. 2005. Disrupting the melanin-concentrating hormone receptor 1 in mice leads to cognitive deficits and alterations of NMDA receptor function. Eur J Neurosci 21(10):2837-44.
3. Abecasis, G.R. & Cookson, W.O. GOLD-graphical overview of linkage disequilibrium. Bioinformatics 16, 182-3 (2000).
4. Als TD, Dahl HA, Flint TJ, Wang AG, Vang M, Mors O, Kruse TA and Ewald H (2004) Possible evidence for a common risk locus for bipolar affective disorder and schizophrenia on chromosome 4p16 in patients from the Faroe Islands. Mol Psychiatry, 9, 93-8.
5. An, W.; Kim, J.; Roeder, R. G. Ordered cooperative functions of PRMT1, p300, and CARM1 in transcriptional activation by p53. Cell117: 735-748, 2004.
6. Arany, Z.; Newsome, D.; Oldread, E.; Livingston, D. M.; Eckner, R. A family of transcriptional adaptor proteins targeted by the E1A oncoprotein. Nature 374: 81-84, 1995.
7. Arany, Z.; Sellers, W. R.; Livingston, D. M.; Eckner, R. E1A-associated p300 and CREB-associated CBP belong to a conserved family of coactivators. (Letter) Cell77: 799-800, 1994.
8. Asherson P, Mant R, Williams N, Cardno A, Jones L, Murphy K, Collier DA, Nanko S, Craddock N, Morris S et al. (1998) A study of chromosome 4p markers and dopamine D5 receptor gene in schizophrenia and bipolar disorder. Mol Psychiatry, 3, 310-20.
9. Association, A.P. Diagnostic and statistical manual of mental disorders., (American Psychiatric Association, Washington, 1994).
10Auga J. 2004. The role of Zic genes in neural development. Mol Cell Neurosci 26(2):205-21.
11 Badner J. A., and E. S. Gershon, 2002, Meta-analysis of whole-genome linkage scans of bipolar disorder and schizophrenia: Mol Psychiatry, v. 7, p. 405-11.
12 Berrettini W (2003) Evidence for shared susceptibility in bipolar disorder and schizophrenia. Am J Med Genet C Semin Med Genet, 123, 59-64.
13 Berrettini W. H., 2000, Are schizophrenic and bipolar disorders related? A review of family and molecular studies: Biol Psychiatry, v. 48, p. 531-8.
14 Bjarkam CR, Pedersen M and Sorensen JC (2001) New strategies for embedding, orientation and sectioning of small brain specimens enable direct correlation to MR-images, brain atlases, or use of unbiased stereology. J Neurosci Methods, 108, 153-9.
15 Blackwood DH, He L, Morris SW, McLean A, Whitton C, Thomson M, Walker MT, Woodburn K, Sharp CM, Wright AF et al. (1996) A locus for bipolar affective disorder on chromosome 4p. Nat Genet, 12, 427-30.
16 Blouin, J.L. et al. Schizophrenia susceptibility loci on chromosomes 13q32 and 8p21. Nat Genet20, 70-3 (1998).
17 Borglum AD, Hampson M, Kjeldsen TE, Muir W, Murray V, Ewald H, Mors O, Blackwood D and Kruse TA (2001) Dopa decarboxylase genotypes may influence age at onset of schizophrenia. Mol Psychiatry, 6, 712-7.
18 Borglum AD, Kirov G, Craddock N, Mors O, Muir W, Murray V, McKee I, Collier DA, Ewald H, Owen MJ et al. (2003) Possible parent-of-origin effect of Dopa decarboxylase in susceptibility to bipolar affective disorder. Am J Med Genet B Neuropsychiatr Genet, 117, 18-22.
19 Borowsky, B.; Durkin, M. M.; Ogozalek, K.; Marzabadi, M. R.; DeLeon, J.; Heurich, R.; Lichtblau, H.; Shaposhnik, Z.; Daniewska, I.; Blackburn, T. P.; Branchek, T. A.; Gerald, C.; Vaysse, P. J.; Forray, C. Antidepressant, anxiolytic and anorectic effects of a melanin-concentrating hormone-1 receptor antagonist. Nature Med. 8: 825-830, 2002.
20 Bttencourt JC, Frigo L, Rissman RA, Casatti CA, Nahon JL, Bauer JA. 1998. The distribution of melanin-concentrating hormone in the monkey brain (Cebus apella). Brain Res 804(1):140-3.
21 Bttencourt JC, Presse F, Arias C, Peto C, Vaughan J, Nahon JL, Vale W, Sawchenko PE. 1992. The melanin-concentrating hormone system of the rat brain: an immuno- and hybridization histochemical characterization. J Comp Neurol 319(2):218-45.
22 Cartegni L, Wang J, Zhu Z, Zhang MQ and Krainer AR (2003) ESEfinder: A web resource to identify exonic splicing enhancers. Nucleic Acids Res, 31, 3568-71.
23 Castella P, Wagner JA and Caudy M (1999) Regulation of hippocampal neuronal differentiation by the basic helix-loop-helix transcription factors HES-1 and MASH-1. J Neurosci Res, 56, 229-40.
24 Castiglia D, Scaturro M, Nastasi T, Cestelli A, Di Liegro I. PIPPin, a putative RNA-binding protein specifically expressed in the rat brain. Biochem Biophys Res Commun. 1996 Jan 5;218(1):390-4.
25 Chaki S, Funakoshi T, Hirota-Okuno S, Nishiguchi M, Shimazaki T, lijima M, Grottick AJ, Kanuma K, Omodera K, Sekiguchi Y and others. 2005. Anxiolytic- and antidepressant-like profile of ATC0065 and ATC0175: nonpeptidic and orally active melanin-concentrating hormone receptor 1 antagonists. J Pharmacol Exp Ther 313(2):831-9.
26 Chambers, J.; Ames, R. S.; Bergsma, D.; Muir, A.; Fitzgerald, L. R.; Hervieu, G.; Dytko, G. M.; Foley, J. J.; Martin, J.; Liu, W.-S.; Park, J.; Ellis, C.; Ganguly, S.; Konchar, S.; Cluderay, J.; Leslie, R.; Wilson, S.; Sarau, H. M. Melanin-concentrating hormone is the cognate-ligand for the orphan G-protein-coupled receptor SLC-1. Nature400: 261-265, 1999.
27 Chang, M.S. et al. HRad17 colocalizes with NHP2L1 in the nucleolus and redistributes after UV irradiation. J Biol Chem274, 36544-9 (1999).
28 Chen S, Smith DF. 1998. Hop as an adaptor in the heat shock protein 70 (Hsp70) and hsp90 chaperone machinery. J Biol Chem 273(52):35194-200.
29 Cojocaru, V., Nottrott, S., Klement, R. & Jovin, T.M. The snRNP 15.5K protein folds its cognate K-turn RNA: a combined theoretical and biochemical study. Rna 11, 197-209 (2005).
30 Coon H, Jensen S, Holik J, Hoff M, Myles-Worsley M, Reimherr F, Wender P, Waldo M, Freedman R, Leppert M et al. (1994) Genomic scan for genes predisposing to schizophrenia. Am J Med Genet, 54, 59-71.
31 Coon, H. et al. Analysis of chromosome 22 markers in nine schizophrenia pedigrees. Am J Med Genet 54, 72-9 (1994).
32 Corfas G, Roy K and Buxbaum JD (2004) Neuregulin 1-erbB signaling and the molecular/cellular basis of schizophrenia. Nat Neurosci, 7, 575-80.
33 Craddock N, O'Donovan MC and Owen MJ (2005) The genetics of schizophrenia and bipolar disorder: dissecting psychosis. J Med Genet, 42, 193-204.
34 Craddock N, O'Donovan MC and Owen MJ (2006) Genes for schizophrenia and bipolar disorder? Implications for psychiatric nosology. Schizophr Bull, 32, 9-16.
35 DeLisi, L.E. et al. A genome-wide scan for linkage to chromosomal regions in 382 sibling pairs with schizophrenia or schizoaffective disorder. Am J Psychiatry 159, 803-12 (2002).
36 Detera-Wadleigh, S.D. et al. A high-density genome scan detects evidence for a bipolar-disorder susceptibility locus on 13q32 and other potential loci on 1q32 and 18p11.2. Proc Natl Acad Sci U S A96, 5604-9 (1999).
37 Devaney JM, Donarum EA, Brown KM, Meyer J, Stober G, Lesch KP, Nestadt G, Stephan DA and Pulver AE (2002) No missense mutation of WKL1 in a subgroup of probands with schizophrenia. Mol Psychiatry, 7, 419-23.
38 Devlin B, Roeder K. 1999. Genomic control for association studies. Biometrics 55(4):997-1004.
39 Eckner, R.; Ewen, M. E.; Newsome, D.; Gerdes, M.; DeCaprio, J. A.; Lawrence, J. B.; Livingston, D. M. Molecular cloning and functional analysis of the adenovirus E1A-associated 300-kD protein (p300) reveals a protein with properties of a transcriptional adaptor. Genes Dev. 15: 869-884, 1994.
40 Edenberg, H.J. et al. Initial genomic scan of the NIMH genetics initiative bipolar pedigrees: chromosomes 3, 5, 15, 16, 17, and 22. Am J Med Genet 74, 238-46 (1997).
41 Enright, A.J. et al. MicroRNA targets in Drosophila. Genome Biol 5, R1 (2003).
42 Etchegaray, J.-P.; Lee, C.; Wade, P. A.; Reppert, S. M. Rhythmic histone acetylation underlies transcription in the mammalian circadian clock. Nature421: 177-182, 2003.
43 Excoffier L and Slatkin M (1995) Maximum-likelihood estimation of molecular haplotype frequencies in a diploid population. Mol Biol Evol, 12, 921-7.
44 Fairbrother WG, Yeh RF, Sharp PA and Burge CB (2002) Predictive identification of exonic splicing enhancers in human genes. Science, 297, 1007-13.
45 Galera, P., Musso, M., Ducy, P. & Karsenty, G. c-Krox, a transcriptional regulator of type I collagen gene expression, is preferentially expressed in skin. Proc Natl Acad Sci U S A91, 9372-6 (1994).
46 Gayther, S. A.; Batley, S. J.; Linger, L.; Bannister, A.; Thorpe, K.; Chin, S.-F.; Daigo, Y.; Russell, P.; Wilson, A.; Sowter, H. M.; Delhanty, J. D. A.; Ponder, B. A. J.; Kouzarides, T.; Caldas, C. Mutations truncating the EP300 acetylase in human cancers. Nature Genet.24: 300-303, 2000.
47 Ghayor, C. et al. Regulation of human COL2A1 gene expression in chondrocytes. Identification of C-Krox-responsive elements and modulation by phenotype alteration. J Biol Chem275, 27421-38 (2000).
48 Gill M., H. Vallada, D. Collier, P. Sham, P. Holmans, R. Murray, P. McGuffin, S. Nanko, M. Owen, S. Antonarakis, D. Housman, H. Kazazian, G. Nestadt, A. E. Pulver, R. E. Straub, C. J. MacLean, D. Walsh, K. S. Kendler, L. DeLisi, M. Polymeropoulos, H. Coon, W. Byerley, R. Lofthouse, E. Gershon, C. M. Read, and et al., 1996, A combined analysis of D22S278 marker alleles in affected sib-pairs: support for a susceptibility locus for schizophrenia at chromosome 22q12. Schizophrenia Collaborative Linkage Group (Chromosome 22): Am J Med Genet, v. 67, p. 40-5.
49 Grossman, S. R.; Deato, M. E.; Brignone, C.; Chan, H. M.; Kung, A. L.; Tagami, H.; Nakatani, Y.; Livingston, D. M. Polyubiquitination of p53 by a ubiquitin ligase activity of p300. Science300: 342-344, 2003.
50 Hamshere, M.L. et al. Genomewide linkage scan in schizoaffective disorder: significant evidence for linkage at 1q42 close to DISC1, and suggestive evidence at 22q11 and 19p13.Arch Gen Psychiatry62, 1081-8 (2005).
51 Hardy OJ, Vekemans X. 2002. SPAGEDi: a versatile computer program to analyse spatial genetic structure at the individual or population levels. Molecular Ecology Notes2:618-620
52 Harrison PJ and Weinberger DR (2005) Schizophrenia genes, gene expression, and neuropathology: on the matter of their convergence. Mol Psychiatry, 10, 40-68.
53 Hasan, S.; Hassa, P. O.; Imhof, R.; Hottiger, M. O. Transcription coactivator p300 binds PCNA and may have a role in DNA repair synthesis. Nature 410: 387-391, 2001.
54 Hasan, S.; Stucki, M.; Hassa, P. O.; Imhof, R.; Gehrig, P.; Hunziker, P.; Hubscher, U.; Hottiger, M. O. Regulation of human flap endonuclease-1 activity by acetylation through the transcriptional coactivator p300. Molec. Cell7: 1221-1231, 2001.
55 Ida, K.; Kitabayashi, I.; Taki, T.; Taniwaki, M.; Noro, K.; Yamamoto, M.; Ohki, M.; Hayashi, Y. Adenoviral E1A-associated protein p300 is involved in acute myeloid leukemia with t(11;22)(q23;q13). Blood 90: 4699-4704, 1997.
56 Ishibashi M (2004) Molecular mechanisms for morphogenesis of the central nervous system in mammals. Anat Sci Int, 79, 226-34.
57 Jensen TG, Andresen BS, Bross P, Jensen UB, Holme E, Kolvraa S, Gregersen N and Bolund L (1992) Expression of wild-type and mutant medium-chain acyl-CoA dehydrogenase (MCAD) cDNA in eucaryotic cells. Biochim Biophys Acta, 1180, 65-72.
58 Jorgensen TH, Borglum AD, Mors O, Wang AG, Pinaud M, Flint TJ, Dahl HA, Vang M, Kruse TA and Ewald H (2002) Search for common haplotypes on chromosome 22q in patients with schizophrenia or bipolar disorder from the Faroe Islands. Am J Med Genet, 114, 245-52.
59 Jorgensen TH, Degn B, Wang AG, Vang M, Gurling H, Kalsi G, McQuillin A, Kruse TA, Mors O, Ewald H. 2002b. Linkage disequilibrium and demographic history of the isolated population of the Faroe Islands. Eur J Hum Genet 10(6):381-7.
60 Kaganovich M, Peretz A, Ritsner M, Bening Abu-Shach U, Attali B and Navon R (2004) Is the WKL1 gene associated with schizophrenia? Am J Med Genet B Neuropsychiatr Genet, 125, 31-7.
61 Kasper, L. H.; Boussouar, F.; Ney, P. A.; Jackson, C. W.; Rehg, J.; van Deursen, J. M.; Brindle, P. K. A transcription-factor-binding surface of coactivator p300 is required for haematopoiesis. Nature 419: 738-743, 2002.
62 Kelsoe J. R., M. A. Spence, E. Loetscher, M. Foguet, A. D. Sadovnick, R. A. Remick, P. Flodman, J. Khristich, Z. Mroczkowski-Parker, J. L. Brown, D. Masser, S. Ungerleider, M. H. Rapaport, W. L. Wishart, and H. Luebbert, 2001, A genome survey indicates a possible susceptibility locus for bipolar disorder on chromosome 22: Proc Natl Acad Sci U S A, v. 98, p. 585-90.
63 Kmoch S, Hartmannova H, Stiburkova B, Krijt J, Zikanova M, Sebesta I. 2000. Human adenylosuccinate lyase (ADSL), cloning and characterization of full-length cDNA and its isoform, gene structure and molecular basis for ADSL deficiency in six patients. Hum Mol Genet 9(10):1501-13.
64 Kolakowski, L. F., Jr.; Jung, B. P.; Nguyen, T.; Johnson, M. P.; Lynch, K. R.; Cheng, R.; Heng, H. H. Q.; George, S. R.; O'Dowd, B. F. Characterization of a human gene related to genes encoding somatostatin receptors. FEBS Lett. 398: 253-258, 1996.
65 Kolodrubetz, D.; Burgum, A. Sequence and genetic analysis of NHP2: a moderately abundant high mobility group-like nuclear protein with an essential function in Saccharomyces cerevisiae. Yeast 7: 79-90, 1991.
66 Lachman, H.M. et al. Linkage studies suggest a possible locus for bipolar disorder near the velo-cardio-facial syndrome region on chromosome 22. Am J Med Genet 74, 121-8 (1997).
67 Ladumer AG, Inouye C, Jain R and Tjian R (2003) Bromodomains mediate an acetyl-histone encoded antisilencing function at heterochromatin boundaries. Mol Cell, 11, 365-76.
68 Lakaye, B.; Minet, A.; Zorzi, W.; Grisar, T. Cloning of the rat brain cDNA encoding for the SLC-1 G protein-coupled receptor reveals the presence of an intron in the gene. Biochim. Biophys. Acta 1401: 216-220, 1998.
69 Lewis CM, Levinson DF, Wise LH, DeLisi LE, Straub RE, Hovatta I, Williams NM, Schwab SG, Pulver AE, Faraone SV et al. (2003) Genome scan meta-analysis of schizophrenia and bipolar disorder, part II: Schizophrenia. Am J Hum Genet, 73, 34-48.
70 Li T, Ma X, Sham PC, Sun X, Hu X, Wang Q, Meng H, Deng W, Liu X, Murray RM, Collier DA. Evidence for association between novel polymorphisms in the PRODH gene and schizophrenia in a Chinese population. Am J Med Genet B Neuropsychiatr Genet. 129B:13-5, 2004
71 Liang, S.G. et al. A linkage disequilibrium study of bipolar disorder and microsatellite markers on 22q13. Psychiatr Genet 12, 231-5 (2002).
72 Lin, C. H.; Hare, B. J.; Wagner, G.; Harrison, S. C.; Maniatis, T.; Fraenkel, E. : A small domain of CBP/p300 binds diverse proteins: solution structure and functional studies. Molec. Cell 8: 581-590, 2001.
73 Livak KJ and Schmittgen TD (2001) Analysis of relative gene expression data using real-time quantitative PCR and the 2(-Delta Delta C(T)) Method. Methods, 25, 402-8.
74 Lukasik SM, Cierpicki T, Borloz M, Grembecka J, Everett A and Bushweller JH (2006) High resolution structure of the HDGF PWWP domain: a potential DNA binding domain. Protein Sci, 15, 314-23.
75 Manning, E.T., Ikehara, T., Ito, T., Kadonaga, J.T. & Kraus, W.L. p300 forms a stable, template-committed complex with chromatin: role for the bromodomain. Mol Cell Biol 21, 3876-87 (2001).
76 Marie S, Cuppens H, Heuterspreute M, Jaspers M, Tola EZ, Gu XX, Legius E, Vincent MF, Jaeken J, Cassiman JJ and others. 1999. Mutation analysis in adenylosuccinate lyase deficiency: eight novel mutations in the re-evaluated full ADSL coding sequence. Hum Mutat 13(3):197-202.
77 Marsh, D. J.; Weingarth, D. T.; Novi, D. E.; Chen, H. Y.; Trumbauer, M. E.; Chen, A. S.; Guan, X.-M.; Jiang, M. M.; Feng, Y.; Camacho, R. E.; Shen, Z.; Frazier, E. G.; et al. Melanin-concentrating hormone 1 receptor-deficient mice are lean, hyperactive, and hyperphagic and have altered metabolism. Proc. Nat. Acad. Sci.99: 3240-3245, 2002.
78 McCullagh, P.; Chaplin, T.; Meerabux, J.; Grenzelias, D.; Lillington, D.; Poulsom, R.; Gregorini, A.; Saha, V.; Young, B. D. The cloning, mapping and expression of a novel gene, BRL, related to the AF1 0 leukaemia gene. Oncogene 18: 7442-7452, 1999.
79 McGuffin P., M. J. Owen, and A. E. Farmer, 1995, Genetic basis of schizophrenia: Lancet, v. 346, p. 678-82.
80 Meyer J, Huberth A, Ortega G, Syagailo YV, Jatzke S, Mossner R, Strom TM, Ulzheimer-Teuber I, Stober G, Schmitt A et al. (2001) A missense mutation in a novel gene encoding a putative cation channel is associated with catatonic schizophrenia in a large pedigree. Mol Psychiatry, 6, 302-6.
81 Moises, H.W. et al. Potential linkage disequilibrium between schizophrenia and locus D22S278 on the long arm of chromosome 22. Am J Med Genet 60, 465-7 (1995).
82 Mowry BJ, Holmans PA, Pulver AE, Gejman PV, Riley B, Williams NM, Laurent C, Schwab SG, Wildenauer DB, Bauche S et al. (2004) Multicenter linkage study of schizophrenia loci on chromosome 22q. Mol Psychiatry, 9, 784-95.
83 Muraoka, M.; Konishi, M.; Kikuchi-Yanoshita, R.; Tanaka, K.; Shitara, N.; Chong, J.-M.; Iwama, T.; Miyaki, M. p300 gene alterations in colorectal and gastric carcinomas. Oncogene 12: 1565-1569, 1996.
84 Myles-Worsley, M. et al. Linkage of a composite inhibitory phenotype to a chromosome 22q locus in eight Utah families. Am J Med Genet 88, 544-50 (1999).
85 Nakashima, K.; Yanagisawa, M.; Arakawa, H.; Kimura, N.; Hisatsune, T.; Kawabata, M.; Miyazono, K.; Taga, T. Synergistic signaling in fetal brain by STAT3-Smad1 complex bridged by p300. Science 284: 479-482, 1999.
86 Nastasi T, Muzi P, Beccari S, Bellafiore M, Dolo V, Bologna M, Cestelli A, Di Liegro I. Specific neurons of brain cortex and cerebellum are PIPPin positive. Neuroreport. 2000 Jul 14;11 (10):2233-6.
87 Nastasi T, Scaturro M, Bellafiore M, Raimondi L, Beccari S, Cestelli A, di Liegro I. PIPPin is a brain-specific protein that contains a cold-shock domain and binds specifically to H1 degrees and H3.3 mRNAs. J Biol Chem. 1999 Aug 20;274(34):24087-93
88 Pissios P, Trombly DJ, Tzameli I, Maratos-Flier E. 2003. Melanin-concentrating hormone receptor 1 activates extracellular signal-regulated kinase and synergizes with G(s)-coupled pathways. Endocrinology 144(8):3514-23.
89 Plomann, M.; Lange, R.; Vopper, G.; Cremer, H.; Heinlein, U. A. O.; Scheff, S.; Baldwin, S. A.; Leitges, M.; Cramer, M.; Paulsson, M.; Barthels, D. PACSIN, a brain protein that is upregulated upon differentiation into neuronal cells. Europ. J. Biochem. 256: 201-211, 1998.
90 Polymeropoulos MH, Coon H, Byerley W, Gershon ES, Goldin L, Crow TJ, Rubenstein J, Hoff M, Holik J, Smith AM and others. 1994. Search for a schizophrenia susceptibility locus on human chromosome 22. Am J Med Genet 54(2):93-9.
91 Potash J. B., and J. R. DePaulo, Jr., 2000, Searching high and low: a review of the genetics of bipolar disorder: Bipolar Disord, v. 2, p. 8-26.
92 Potash J. B., P. P. Zandi, V. L. Willour, T. H. Lan, Y. Huo, D. Avramopoulos, Y. Y. Shugart, D. F. MacKinnon, S. G. Simpson, F. J. McMahon, J. R. DePaulo, Jr., and M. G. McInnis, 2003b, Suggestive linkage to chromosomal regions 13q31 and 22q12 in families with psychotic bipolar disorder: Am J Psychiatry, v. 160, p. 680-6.
93 Potash J. B., Y. F. Chiu, D. F. MacKinnon, E. B. Miller, S. G. Simpson, F. J. McMahon, M. G. McInnis, and J. R. DePaulo, Jr., 2003a, Familial aggregation of psychotic symptoms in a replication set of 69 bipolar disorder pedigrees: Am J Med Genet, v. 116B, p. 90-7.
94 Potash JB, Willour VL, Chiu YF, Simpson SG, MacKinnon DF, Pearlson GD, DePaulo JR, Jr., McInnis MG. 2001. The familial aggregation of psychotic symptoms in bipolar disorder pedigrees. Am J Psychiatry 158(8):1258-64.
95 Prapapanich V, Chen S, Nair SC, Rimerman RA, Smith DF. 1996. Molecular cloning of human p48, a transient component of progesterone receptor complexes and an Hsp70-binding protein. Mol Endocrinol 10(4):420-31.
96 Pulver A. E., M. Karayiorgou, P. S. Wolyniec, V. K. Lasseter, L. Kasch, G. Nestadt, S. Antonarakis, D. Housman, H. H. Kazazian, D. Meyers, and et al., 1994, Sequential strategy to identify a susceptibility gene for schizophrenia: report of potential linkage on chromosome 22q12-q13.1: Part 1: Am J Med Genet, v. 54, p. 36-43.
97. Puri, V. et al. Failure to Confirm Allelic Association Between Markers at the CAPON Gene Locus and Schizophrenia in a British Sample. Biol Psychiatry (2005).
98. Qu D, Ludwig DS, Gammeltoft S, Piper M, Pelleymounter MA, Cullen MJ, Mathes WF, Przypek R, Kanarek R, Maratos-Flier E. 1996. A role for melanin-concentrating hormone in the central regulation of feeding behaviour. Nature 380(6571):243-7.
99. Quandt K, Frech K, Karas H, Wingender E and Wemer T (1995) MatInd and MatInspector: new fast and versatile tools for detection of consensus matches in nucleotide sequence data. Nucleic Acids Res, 23, 4878-84.
100. Queller DC, Goodnight KF. 1989. Estimating Relatedness Using Genetic-Markers. Evolution 43(2):258-275.
101. Ragvin A, Valvatne H, Erdal S, Arskog V, Tufteland KR, Breen K, AM OY, Eberharter A, Gibson TJ, Becker PB et al. (2004) Nucleosome binding by the bromodomain and PHD finger of the transcriptional cofactor p300. J Mol Biol, 337, 773-88.
102. Rapoport JL, Addington AM, Frangou S and Psych MR (2005) The neurodevelopmental model of schizophrenia: update 2005. Mol Psychiatry, 10, 434-49.
103. Reese MG, Eeckman FH, Kulp D and Haussler D (1997) Improved splice site detection in Genie. J Comput Biol, 4, 311-23.
104. Ritter, B.; Modregger, J.; Paulsson, M.; Plomann, M. PACSIN 2, a novel member of the PACSIN family of cytoplasmic adapter proteins. FEBS Lett. 454: 356-362, 1999.
105. Roelfsema, J.H. et al. Genetic heterogeneity in Rubinstein-Taybi syndrome: mutations in both the CBP and EP300 genes cause disease. Am J Hum Genet 76, 572-80 (2005).
106. Rousset F. 2000. Genetic differentiation between individuals. Journal of Evolutionary Biology 13(1):58-62.
107. Rubie C, Lichtner P, Gartner J, Siekiera M, Uziel G, Kohlmann B, Kohlschutter A, Meitinger T, Stober G and Bettecken T (2003) Sequence diversity of KIAA0027/MLC1: are megalencephalic leukoencephalopathy and schizophrenia allelic disorders? Hum Mutat, 21, 45-52.
108. Sadusky, T. J.; Kemp, T. J.; Simon, M.; Carey, N.; Coulton, G. R. : Identification of Serhl, a new member of the serine hydrolase family induced by passive stretch of skeletal muscle in vivo. Genomics 73: 38-49, 2001. Note: Erratum: Genomics 74: 251 only, 2001.
109. Sadusky, T.J., Kemp, T.J., Simon, M., Carey, N. & Coulton, G.R. Identification of Serhl, a new member of the serine hydrolase family induced by passive stretch of skeletal muscle in vivo. Genomics 73, 38-49 (2001).
110. Saito, H.; Fujiwara, T.; Shin, S.; Okui, K.; Nakamura, Y. Cloning and mapping of a human novel cDNA (NHP2L1) that encodes a protein highly homologous to yeast nuclear protein NHP2. Cytogenet. Cell Genet. 72: 191-193, 1996.
111. Saito, Y.; Nothacker, H.-P.; Wang, Z.; Lin, S. H. S.; Leslie, F.; Civelli, O. Molecular characterization of the melanin-concentrating-hormone receptor. Nature 400: 265-269, 1999.
112. Saleem Q, Dash D, Gandhi C, Kishore A, Benegal V, Sherrin T, Mukherjee O, Jain S, Brahmachari SK. 2001. Association of CAG repeat loci on chromosome 22 with schizophrenia and bipolar disorder. Mol Psychiatry 6(6):694-700.
113. Schmitz G, Heimerl S and Langmann T (2004) Zinc finger protein ZNF202 structure and function in transcriptional control of HDL metabolism. Curr Opin Lipidol, 15, 199-208.
114. Schwab SG and Wildenauer DB (1999) Chromosome 22 workshop report. Am J Med Genet, 88, 276-8.
115. Segurado, R. et al. Genome scan meta-analysis of schizophrenia and bipolar disorder, part III: Bipolar disorder. Am J Hum Genet 73, 49-62 (2003).
116. Sham PC, Curtis D. 1995. Monte Carlo tests for associations between disease and alleles at highly polymorphic loci. Ann Hum Genet 59 ( Pt 1):97-105.
117. Sinibaldi L, De Luca A, Bellacchio E, Conti E, Pasini A, Paloscia C, Spalletta G, Caltagirone C, Pizzuti A, Dallapiccola B. Mutations of the Nogo-66 receptor (RTN4R) gene in schizophrenia.Hum Mutat. 24:534-5, 2004.
118. Skibinska M, Hauser J, Czerski PM, Leszczynska-Rodziewicz A, Kosmowska M, Kapelski P, Slopien A, Zakrzewska M, Rybakowski JK. Association analysis of brain-derived neurotrophic factor (BDNF) gene Val66Met polymorphism in schizophrenia and bipolar affective disorder. World J Biol Psychiatry 5:215-20, 2004
119. Smith DG, Tzavara ET, Shaw J, Luecke S, Wade M, Davis R, Salhoff C, Nomikos GG, Gehlert DR. 2005. Mesolimbic dopamine super-sensitivity in melanin-concentrating hormone-1 receptor-deficient mice. J Neurosci 25(4):914-22.
120. Song, J. et al. Transcriptional regulation by zinc-finger proteins Sp1 and MAZ involves interactions with the same cis-elements. Int J Mol Med 11, 547-53 (2003).
121. Spitzer R & J, E. The Schedule for Affective Disorders and Schizophrenia, Lifetime Version, 3rd edition., (The Schedule for Affective Disorders and Schizophrenia, Lifetime Version, 3rd edition, New York, 1977).
122. Spitzer R, J, E. & E, R. Research Diagnostic Criteria for a selected group of functional disorders, 3rd edition, (New York, New York State Psychiatric Institute, New York, 1978).
123. Stober G, Saar K, Ruschendorf F, Meyer J, Nurnberg G, Jatzke S, Franzek E, Reis A, Lesch KP, Wienker TF et al. (2000) Splitting schizophrenia: periodic catatonia-susceptibility locus on chromosome 15q15. Am J Hum Genet, 67, 1201-7.
124. Strakowski SM, Delbello MP and Adler CM (2005) The functional neuroanatomy of bipolar disorder: a review of neuroimaging findings. Mol Psychiatry, 10, 105-16.
125. Sumoy, L.; Pluvinet, R.; Andreu, N.; Estivill, X.; Escarceller, M. PACSIN 3 is a novel SH3 domain cytoplasmic adapter protein of the pacsin-syndapin-FAP52 gene family. Gene 262:199-205, 2001.
126. Takahashi S, Faraone SV, Lasky-Su J and Tsuang MT (2005) Genome-wide scan of homogeneous subtypes of NIMH genetics initiative schizophrenia families. Psychiatry Res, 133, 111-22.
127. Takahashi, K.; Totsune, K.; Murakami, O.; Sone, M.; Satoh, F.; Kitamuro, T.; Noshiro, T.; Hayashi, Y.; Sasano, H.; Shibahara, S. Expression of melanin-concentrating hormone receptor messenger ribonucleic acid in tumor tissues of pheochromocytoma, ganglioneuroblastoma, and neuroblastoma. J. Clin. Endocr. Metab. 86: 369-374, 2001.
128. Tini, M.; Benecke, A.; Um, S.-J.; Torchia, J.; Evans, R. M.; Chambon, P. Association of CBP/p300 acetylase and thymine DNA glycosylase links DNA repair and transcription. Molec. Cell 9: 265-277, 2002.
129. Vallada H, Curtis D, Sham PC, Murray RM, McGuffin P, Nanko S, Gill M, Owen M, Collier DA. 1995. Chromosome 22 markers demonstrate transmission disequilibrium with schizophrenia. Psychiatr Genet 5(3):127-30.
130. Verma R, Mukerji M, Grover D, C BR, Das SK, Kubendran S, Jain S and Brahmachari SK (2005) MLC1 gene is associated with schizophrenia and bipolar disorder in Southern India. Biol Psychiatry, 58, 16-22.
131. Wang Z, Rolish ME, Yeo G, Tung V, Mawson M and Burge CB (2004) Systematic identification and analysis of exonic splicing silencers. Cell, 119, 831-45.
132. Weaver, B. K.; Kumar, K. P.; Reich, N. C. Interferon regulatory factor 3 and CREB-binding protein/p300 are subunits of double-stranded RNA-activated transcription factor DRAF1. Molec. Cell. Biol. 18: 1359-1368, 1998.
133. Wemer T (2000) Computer-assisted analysis of transcription control regions. Matinspector and other programs. Methods Mol Biol, 132, 337-49.
134. WHO. Schedules for Clinical Assessment in Neuropsychiatry, (World Health Organization, Geneva., 1996).
135. WHO. The ICD-10 Classification of Mental and Behavioural Disorders. Diagnostic Criteria for Research., (World Health Organization, Geneva., 1993).
136. Wildenauer DB, Schwab SG, Maier W and Detera-Wadleigh SD (1999) Do schizophrenia and affective disorder share susceptibility genes? Schizophr Res, 39, 107-11.
137. Wing, J.K., Sartorius, N. & and Üstün, T.B.E. Diagnosis and clinical measurement in psychiatry. A reference manual for SCAN, (New York, 1998).
138. Xi ZR, Qin W, Yang YF, He G, Gao SH, Ren MS, Peng YW, Zhang Z, He L. Transmission disequilibrium analysis of the GSN gene in a cohort of family trios with schizophrenia Neurosci Lett.. 372:200-3, 2004
139. Yao, T. P.; Oh, S. P.; Fuchs, M.; Zhou, N.-D.; Ch'ng, L.-E.; Newsome, D.; Bronson, R. T.; Li, E.; Livingston, D. M.; Eckner, R. Gene dosage-dependent embryonic development and proliferation defects in mice lacking the transcriptional integrator p300. Cell 93: 361-372, 1998.
140. Yeo G and Burge CB (2004) Maximum entropy modeling of short sequence motifs with applications to RNA splicing signals. J Comput Biol, 11, 377-94.
141. Zaykin DV, Westfall PH, Young SS, Kamoub MA, Wagner MJ and Ehm MG (2002) Testing association of statistically inferred haplotypes with discrete and continuous traits in samples of unrelated individuals. Hum Hered, 53, 79-91.

**Table 1-9 Table 1 Genotyped polymorphisms and allele frequencies.**

| | | | | | MAF SCOTLAND | | | MAF UK | | | MAF DK | | |
|---|---|---|---|---|---|---|---|---|---|---|---|---|---|
| Gene | SNP | Location (Mb)^{a} | Type of SNP | Alleles^{b} | Controls | BPD | SZ | Controls | BPD | SZ | Controls | BPD | SZ |
| *EP300* | rs20551 | 39.872508 | Nonsyn | A/G | 0.38 | 0.30 | 0.38 | 0.30 | 0.32 | 0.29 | 0.28 | 0.23 | 0.27 |
| *EP300* | rs2294976 | 39.889208 | Intron | C/A | - | - | - | 0.08 | 0.07 | 0.11 | 0.10 | 0.11 | 0.06 |
| *EP300* | rs2076578 | 39.894109 | Intron | C/T | 0.34 | 0.33 | 0.31 | 0.34 | 0.32 | 0.33 | 0.36 | 0.35 | 0.37 |
| *EP300* | rs5758252 | 39.898735 | Nonsyn | A/T | 0.00 | 0.00 | 0.00 | - | - | - | - | - | - |
| *PIPPIN* | rs6002408 | 40.210909 | Promoter | G/A | - | - | - | - | - | - | - | - | - |
| *PIPPIN* | rs1006407 | 40.296532 | 3' UTR | T/C | 0.15 | 0.20 | 0.21 | 0.17 | 0.18 | 0.18 | 0.20 | 0.12 | 0.16 |
| *NHP2L1* | rs8779 | 40.394776 | 3' UTR | G/A | 0.21 | 0.18 | 0.19 | 0.19 | 0.26 | 0.26 | 0.22 | 0.12 | 0.34 |
| *NHP2L1* | rs132806 | 40.395293 | 3' UTR | C/T | - | - | - | 0.38 | 0.34 | 0.39 | 0.34 | 0.41 | 0.36 |
| *NHP2L1* | rs1802521 | 40.400816 | Nonsyn | T/G | 0.00 | 0.00 | 0.00 | - | - | - | - | - | - |
| *NHP2L1* | rs5758405 | 40.400881 | Intron | A/C | - | - | - | 0.18 | 0.19 | 0.20 | 0.21 | 0.13 | 0.16 |
| *SERHL* | rs881542 | 41.220498 | Promoter | C/G | 0.20 | 0.16 | 0.23 | 0.23 | 0.17 | 0.17 | 0.23 | 0.12 | 0.14 |
| *SERHL* | rs926333 | 41.222243 | Nonsyn | G/A | 0.11 | 0.14 | 0.20 | 0.01 | 0.02 | 0.03 | 0.05 | 0.09 | 0.03 |

| | | | | | | | | | | | | | |
|---|---|---|---|---|---|---|---|---|---|---|---|---|---|
| ^{a} According to the UCSC Genome Browser, May 2004 assembly (http://www.genome.ucsc.edu) ^{b} Major allele/minor allele on the +strand (http://www.genome.ucsc.edu) Nonsyn=Nonsynonymous SNP, MAF= Minor allele frequency, Syn.=Synonymous SNI =no data available for the SNP in this sample. | | | | | | | | | | | | | |

**Table 2**

| Single marker and overall haplotype association analysis in the case-control sample from Scotland. | | | | | | |
|---|---|---|---|---|---|---|
| Empirical overall p-values ^{a} | | | | | | |
| _{Gene} | _{SNP} | Single marker | 2-marker | 3-marker | 4-marker | 5-marker |
| BPD+SZ | | | | | | |
| *EP300* | rs20551 | 0.1072 | | | | |
| *EP300* | rs2076578 | 0.7960 | **0.0158** | | | |
| *PIPPIN* | rs1006407 | **0.0487** | 0.2046 | **0.0047** | | |
| *NHP2L1* | rs8779 | 0.3639 | **4.0x10⁻⁵** | **0.0016** | **7.5x10⁻⁵** | |
| *SERHL* | rs881542 | 0.7099 | 0.5484 | **0.0006** | **0.0020** | **5.9x10⁻⁵** |
| *SERHL* | rs926333 | 0.0565 | 0.1627 | 0.2433 | **0.0007** | **0.0109** |
| SZ | | | | | | |
| *EP300* | rs20551 | 0.7312 | | | | |
| *EP300* | rs2076578 | 0.7049 | 0.6775 | | | |
| *PIPPIN* | rs1006407 | 0.0899 | 0.3323 | **0.0285** | | |
| *NHP2L1* | rs8779 | 0.6759 | 0.0315 | 0.2056 | 0.0957 | |
| *SERHL* | rs881542 | 0.2902 | 0.7437 | 0.1035 | 0.0922 | **0.0164** |
| *SERHL* | rs926333 | **0.0131** | **0.0406** | 0.1300 | **0.0122** | **0.0426** |
| BPD | | | | | | |
| *EP300* | rs20551 | **0.0132** | | | | |
| *EP300* | rs2076578 | 0.9154 | **0.0011** | | | |
| *PIPPIN* | rs1006407 | 0.0963 | 0.3200 | **0.0016** | | |
| *NHP2L1* | rs8779 | 0.3100 | **0.0007** | **0.0176** | **0.0001** | |
| *SERHL* | rs881542 | 0.1884 | 0.3666 | **0.0063** | **0.0308** | **0.0008** |
| *SERHL* | rs926333 | 0.3943 | 0.5573 | 0.6804 | **0.0468** | 0.2464 |

| | | | | | | |
|---|---|---|---|---|---|---|
| ^{a} Empirical overall p-values based on 100,000,000 permutations using the HTR program. P-values < 0.05 in bold. | | | | | | |

**Table 3 Distribution selected individual haplotypes in the case-control sample from Scotland.**

| Haplotype | | | | | | Haplotype frequency | | | | Empirical p-values^{a} | | |
|---|---|---|---|---|---|---|---|---|---|---|---|---|
| S1 | S3 | S4 | S5 | S8 | S9 | Controls | Cases | BPD | SZ | Combined | BPD | SZ |
| | | T | A | | | 0.0639 | 0.0069 | 0.0038 | 0.0117 | **4.2x10-6** | **7.7x10-5** | **0.0065** |
| | | T | A | C | | 0.0588 | 0.0032 | <0.001 | 0.0087 | **2.2x10-6** | **5.4x10-5** | **0.0053** |
| G | T | | | | | <0.001 | 0.0435 | 0.0625 | <0.001 | **0.0024** | **0.0002** | - |
| G | T | T | | | | <0.001 | 0.0382 | 0.0589 | <0.001 | **0.0096** | **0.0005** | - |
| G | C | C | | | | 0.0874 | 0.1606 | 0.1607 | 0.1649 | **0.0039** | 0.0069 | **0.0234** |
| G | C | C | A | | | 0.0927 | 0.1478 | 0.1454 | 0.1555 | **0.0244** | **0.0483** | 0.0519 |
| | | C | A | C | A | 0.0161 | 0.0582 | 0.0475 | 0.0684 | **0.0036** | **0.0444** | **0.0012** |
| | | | | G | A | 0.0073 | 0.0306 | 0.0142 | 0.0549 | 0.0799 | 0.8565 | **0.0071** |

| | | | | | | | | | | | | |
|---|---|---|---|---|---|---|---|---|---|---|---|---|
| ^{a} Empirical overall p-values based on 100,000,000 permutations using the HTR program. S1 to S9 correspond to SNP: rs20551, rs2294976, rs2076578, rs1006407, rs8779, rs132806, rs5758405, rs881542, rs926333. - =no p-value value calculated by HTR. | | | | | | | | | | | | |

**Table 4 Single marker and overall haplotype association analysis in the case-control sample from UK.**

| | | | Empirical overall p-values^{a} | | | |
|---|---|---|---|---|---|---|
| Gene | SNP | Single marker | 2-marker | 3-marker | 4-marker | 5-marker |
| BPD+SZ | | | | | | |
| *EP300* | rs20551 | 0.9165 | | | | |
| *EP300* | rs2294976 | 0.8545 | 0.9540 | | | |
| *EP300* | rs2076578 | 0.6831 | 0.9145 | 0.6174 | | |
| *PIPPIN* | rs1006407 | 0.7295 | 0.9554 | 0.7203 | 0.1967 | |
| *NHP2L1* | rs8779 | **0.0024** | **1.0x10⁻⁶** | **4.6x10⁻⁵** | **0.0002** | **7.1x10⁻⁵** |
| *NHP2L1* | rs132806 | 0.7380 | **0.0411** | 0.2390 | 0.5064 | 0.4782 |
| *NHP2L1* | rs5758405 | 0.5405 | 0.5010 | **0.0218** | 0.0536 | 0.1800 |
| *SERHL* | rs881542 | 0.1777 | 0.5431 | 0.7254 | 0.1253 | 0.3031 |
| *SERHL* | rs926333 | **0.0378** | **0.0106** | 0.0825 | 1.0000 | **0.0497** |

| SZ | | | | | | |
|---|---|---|---|---|---|---|
| *EP300* | rs20551 | 0.5960 | | | | |
| *EP300* | rs2294976 | 0.2095 | 0.4662 | | | |
| *EP300* | rs2076578 | 0.8892 | 0.5282 | 0.5771 | | |
| *PIPPIN* | rs1006407 | 0.7103 | 0.8425 | 0.5255 | 0.3510 | |
| *NHP2L1* | rs8779 | **0.0149** | 0.0827 | 0.2246 | 0.1940 | 0.1058 |
| *NHP2L1* | rs132806 | 0.7226 | 0.0546 | 0.1928 | 0.3120 | 0.4166 |
| *NHP2L1* | rs5758405 | 0.4549 | 0.3086 | **0.0459** | 0.0926 | 0.1729 |
| *SERHL* | rs881542 | 0.3350 | 0.8203 | 0.5076 | 0.1430 | 0.2796 |
| *SERHL* | rs926333 | 0.3211 | 0.1065 | 0.3183 | 1.0000 | 1.0000 |

| BPD | | | | | | |
|---|---|---|---|---|---|---|
| *EP300* | rs20551 | 0.5202 | | | | |
| *EP300* | rs2294976 | 0.3364 | 0.4251 | | | |
| *EP300* | rs2076578 | 0.5903 | 0.4775 | 0.3769 | | |
| *PIPPIN* | rs1006407 | 0.8198 | 0.9425 | 0.9041 | 0.2412 | |
| *NHP2L1* | rs8779 | **0.0044** | **2.4x10⁻¹¹** | **4.7x10⁻⁹** | **1.3x10⁻⁸** | **1.1x10⁻⁸** |
| *NHP2L1* | rs132806 | 0.2669 | 0.0038 | 0.0501 | 0.2802 | 0.3318 |
| *NHP2L1* | rs5758405 | 0.7431 | 0.4534 | **0.0081** | **0.0144** | 0.1489 |
| *SERHL* | rs881542 | 0.1882 | 0.3103 | 0.7406 | 0.0852 | 0.1502 |
| *SERHL* | rs926333 | **0.0155** | **0.0157** | 0.0677 | 0.4050 | 0.0714 |

| | | | | | | |
|---|---|---|---|---|---|---|
| ^{a} Empirical overall p-values based on 100,000,000 permutations using the HTR program. P-values < 0.05 in bold. | | | | | | |

**Table 5 Distribution of selected Individual haplotypes in the case-control sample from UK.**

| Haplotype | | | | | | | | | Haplotype frequency | | | | Empirical p-values^{a} | | |
|---|---|---|---|---|---|---|---|---|---|---|---|---|---|---|---|
| S1 | S2 | S3 | S4 | S5 | S6 | S7 | S8 | S9 | Controls | Cases | BPD | SZ | Combined | BPD | SZ |
| A | C | | T | A | | | | | 0.0127 | 0.0718 | 0.0890 | 0.0493 | **7.0x10⁻⁶** | **6.5x10⁻⁸** | **0.0061** |
| A | | | T | A | | | | | 0.0133 | 0.0860 | 0.1080 | 0.0614 | **6.2x10⁻⁷** | **2.1x10⁻⁹** | **0.0016** |
| | C | | T | A | | | | | 0.0280 | 0.0882 | 0.1153 | 0.0530 | **1.3x10⁻⁵** | **2.9x10⁻⁸** | **0.0402** |
| | | C | T | A | | | | | 0.0178 | 0.0601 | 0.0835 | 0.0251 | **6.3x10⁻⁵** | **2.3x10⁻⁷** | 0.0650 |
| | | | T | A | | | | | 0.0283 | 0.1026 | 0.1362 | 0.0645 | **1.6x10⁻⁶** | **1.4x10⁻⁹** | **0.0137** |
| | | | T | G | | | | | 0.8021 | 0.7197 | 0.6874 | 0.7562 | **0.0005** | **1.9x10⁻⁵** | 0.0933 |
| | | | | A | T | | | | <0.001 | 0.0267 | 0.0432 | 0.0071 | **0.0088** | **0.0028** | **0.0295** |
| | | | | | | A | C | A | 0.0345 | 0.0108 | 0.0097 | 0.0256 | **0.0068** | **0.0091** | 0.1274 |
| | | | | | | | C | A | 0.0371 | 0.0138 | 0.0106 | 0.0290 | **0.0126** | **0.0077** | 0.2231 |
| | | | | | | | C | G | 0.7687 | 0.8162 | 0.8221 | 0.7862 | **0.0209** | **0.0167** | 0.1399 |

| | | | | | | | | | | | | | | | |
|---|---|---|---|---|---|---|---|---|---|---|---|---|---|---|---|
| ^{a} Empirical overall p-values based on 100,000,000 permutations using the HTR program. S1 to S9 correspond to SNP: rs20551, rs2294976, rs2076578, rs1006407, rs8779, rs132806, rs5758405, rs881542, rs926333. - =no value calculated by HTR. | | | | | | | | | | | | | | | |

**Table 6 Single marker and overall haplotype association analysis in the case-control sample from Denmark.**

| | | | Empirical overall p-values ^{a} | | | |
|---|---|---|---|---|---|---|
| Gene | SNP | Single marker | 2-marker | 3-marker | 4-marker | 5-marker |
| BPD+SZ | | | | | | |
| *EP300* | rs20551 | 0.4570 | | | | |
| *EP300* | rs2294976 | 0.6981 | 0.8096 | | | |
| *EP300* | rs2076578 | 0.8966 | 0.0599 | 0.8173 | | |
| *PIPPIN* | rs1006407 | **0.0488** | 0.2144 | **0.0462** | 0.3234 | |
| *NHP2L1* | rs8779 | 0.3981 | 0.0797 | 0.2749 | **0.0335** | 0.6045 |
| *NHP2L1* | rs132806 | 0.2982 | 0.5614 | 0.1823 | 0.2319 | **0.0437** |
| *NHP2L1* | rs5758405 | **0.0453** | 0.1958 | 0.2549 | 0.3628 | 0.2453 |
| *SERHL* | rs881542 | **0.0005** | **0.0015** | **0.0113** | **0.0461** | **0.0357** |
| *SERHL* | rs926333 | 0.4982 | **0.0010** | **0.0015** | **0.0217** | **0.0492** |

| SZ | | | | | | |
|---|---|---|---|---|---|---|
| *EP300* | rs20551 | 0.8907 | | | | |
| *EP300* | rs2294976 | 0.1734 | 0.6296 | | | |
| *EP300* | rs2076578 | 0.7536 | 0.0582 | 0.6455 | | |
| *PIPPIN* | rs1006407 | 0.2771 | 0.7759 | 0.4973 | 0.7063 | |
| *NHP2L1* | rs8779 | 0.2156 | **0.0065** | 0.0557 | 0.0693 | 0.1549 |
| *NHP2L1* | rs132806 | 0.7415 | 0.3238 | **0.0269** | 0.0872 | 0.0905 |
| *NNP2L1* | rs5758405 | 0.1829 | 0.4890 | **0.0221** | 0.0621 | 0.1038 |
| *SERHL* | rs881542 | **0.0086** | **0.0237** | 0.1012 | **0.0322** | **0.0190** |
| *SERHL* | rs926333 | 0.3166 | **0.0148** | **0.0391** | 0.1481 | **0.0367** |

| BPD | | | | | | |
|---|---|---|---|---|---|---|
| *EP300* | rs20551 | 0.2597 | | | | |
| *EP300* | rs2294976 | 0.6625 | 0.6913 | | | |
| *EP300* | rs2076578 | 0.9466 | 0.2034 | 0.6708 | | |
| *PIPPIN* | rs1006407 | **0.0219** | **0.0463** | **0.0182** | 0.2915 | |
| *NHP2L1* | rs8779 | **0.0034** | **0.0078** | **0.0236** | 0.0928 | 0.1696 |
| *NHP2L1* | rs132806 | 0.1508 | **0.0251** | **0.0347** | **0.0247** | **0.0123** |
| *NHP2L1* | rs5758405 | **0.0302** | 0.0972 | **0.0390** | 0.1322 | **0.0389** |
| *SERHL* | rs881542 | **0.0008** | **0.0016** | **0.0082** | **0.0248** | **0.0152** |
| *SERHL* | rs926333 | 0.8064 | **0.0011** | **0.0031** | **0.0144** | **0.0214** |

| | | | | | | |
|---|---|---|---|---|---|---|
| ^{a} Empirical overall p-values based on 100,000,000 permutations using the HTR program. P-values < 0.05 in bold. | | | | | | |

**Table 7 Distribution of selected individual haplotypes in the case-control sample from Denmark**

| Haplotype | | | | | | | | | Haplotype frequency | | | | Empirical p-values ^{a} | | |
|---|---|---|---|---|---|---|---|---|---|---|---|---|---|---|---|
| S1 | S2 | S3 | S4 | S5 | S6 | S7 | S8 | S9 | Controls | Cases | BPD | SZ | Combined | BPD | SZ |
| | | C | C | | | | | | 0.1735 | 0.1046 | 0.0700 | 0.1333 | **0.0423** | **0.0093** | 0.3027 |
| | | | T | G | | | | | 0.7736 | 0.8093 | 0.8780 | 0.7195 | 0.3215 | **0.0036** | 0.2499 |
| | | C | T | G | T | | | | 0.1112 | 0.2130 | 0.2532 | 0.1676 | 0.0583 | **0.0041** | 0.4447 |
| | | | T | G | T | A | C | | 0.2151 | 0.3232 | 0.3451 | 0.2961 | **0.0303** | **0.0046** | 0.2928 |
| | | | T | A | | | | | 0.0212 | 0.0554 | <0.001 | 0.1279 | 0.1129 | **0.0214** | **0.0008** |
| | | | | G | C | A | G | | 0.0950 | 0.0382 | 0.0444 | 0.0304 | **0.0058** | **0.0296** | **0.0078** |
| | | | | A | C | A | | | <0.001 | 0.0442 | <0.001 | 0.0950 | 0.0530 | - | **0.0006** |
| | | | | | | A | C | | 0.5902 | 0.7504 | 0.7643 | 0.7345 | **0.0002** | **0.0001** | **0.0045** |
| | | | | | | A | C | G | 0.5563 | 0.7119 | 0.7227 | 0.6984 | **0.0005** | **0.0008** | **0.0058** |
| | | | | | | | C | G | 0.7120 | 0.8298 | 0.8349 | 0.8235 | **0.0007** | **0.0030** | **0.0042** |
| | | | | | | A | G | G | 0.2003 | 0.1017 | 0.0955 | 0.1096 | **0.0037** | **0.0046** | **0.0284** |
| | | | | | | | G | G | 0.2337 | 0.1318 | 0.1159 | 0.1512 | **0.0012** | **0.0013** | **0.0252** |

| | | | | | | | | | | | | | | | |
|---|---|---|---|---|---|---|---|---|---|---|---|---|---|---|---|
| ^{a} Empirical overall p-values based on 100 000 000 permutations using the HTR program. S1 to S9 correspond to SNP: rs20551, rs2294976, rs2076578, rs1006407, rs8779, rs132806, rs5758405, rs881542, rs926333. - =no value calculated by HTR. | | | | | | | | | | | | | | | |

**Table 8 Selected Individual haplotypes and SNPs having a consistent distribution inbetween samples.**

| | Haplotype | | | | | | | | | Haplotype frequency | | | | Empirical p-values ^{a} | | |
|---|---|---|---|---|---|---|---|---|---|---|---|---|---|---|---|---|
| Sample | S1 | S2 | S3 | S4 | S5 | S6 | S7 | S8 | S9 | Controls | CASES | BPD | SZ | Combined | BPD | SZ |
| DK | | | | | | | | 2 | | 0.2300 | 0.1330 | 0.1200 | 0.1400 | **0.0005** | **0.0008** | **0.0086** |
| UK | | | | | | | | 2 | | 0.2300 | 0.1700 | 0.1700 | 0.1700 | 0.1777 | 0.1882 | 0.3350 |
| Scottish | | | | | | | | 2 | | 0.2000 | 0.1907 | 0.1650 | 0.2300 | 0.7099 | 0.1884 | 0.2902 |
| Combined | | | | | | | | 2 | | 0.2000 | 0.1700 | 0.1600 | 0.1800 | **0.0050** | **0.0017** | 0.1295 |
| DK | | | | 1 | 2 | | | | | 0.0054 | 0.0072 | 0.0041 | 0.0121 | 0.8689 | 0.8511 | 0.6778 |
| UK | | | | 1 | 2 | | | | | 0.0144 | 0.0374 | 0.0557 | 0.0181 | **0.0254** | **0.0015** | 0.7145 |
| Scottish | | | | 1 | 2 | | | | | 0.0184 | 0.0271 | 0.0298 | 0.0230 | 0.3366 | 0.2798 | 0.6337 |
| Combined | | | | 1 | 2 | | | | | 0.0140 | 0.0279 | 0.0355 | 0.0179 | **0.0291** | **0.0028** | 0.5858 |
| DK | | | | | 1 | 1 | 1 | | | 0.0000 | 0.0442 | 0.0000 | 0.0950 | 0.0530 | - | **0.0006** |
| UK | | | | | 1 | 1 | 1 | | | 0.0118 | 0.0196 | 0.0078 | 0.0296 | 0.0324 | 0.3299 | **0.0166** |
| DK | | | | | | | | 1 | 2 | 0.2808 | 0.1700 | 0.1650 | 0.1760 | **0.0007** | **0.0030** | **0.0042** |
| UK | | | | | | | | 1 | 2 | 0.2310 | 0.1840 | 0.1780 | 0.2140 | **0.0209** | **0.0167** | 0.1399 |
| Scottish | | | | | | | | 1 | 2 | 0.3150 | 0.3180 | 0.2890 | 0.3680 | 0.5508 | 0.5385 | 0.6798 |
| Combined | | | | | | | | 1 | 2 | 0.2660 | 0.2120 | 0.2040 | 0.2220 | **0.0011** | **0.0005** | 0.0716 |
| DK | | | | | | | | 2 | 2 | 0.2337 | 0.1318 | 0.1159 | 0.1512 | **0.0012** | **0.0013** | **0.0252** |
| UK | | | | | | | | 2 | 2 | 0.1941 | 0.1636 | 0.1642 | 0.1629 | 0.1437 | 0.1745 | 0.2537 |
| Scottish | | | | | | | | 2 | 2 | 0.1970 | 0.1624 | 0.1558 | 0.1760 | 0.3795 | 0.1802 | 0.7788 |
| Combined | | | | | | | | 2 | 2 | 0.2011 | 0.1551 | 0.1509 | 0.1605 | **0.0020** | **0.0016** | 0.0670 |

| | | | | | | | | | | | | | | | | |
|---|---|---|---|---|---|---|---|---|---|---|---|---|---|---|---|---|
| ^{a} Empirical overall p-values based on 100,000,000 permutations using the HTR program. S1 to S9 correspond to SNP: rs20551, rs2294976, rs2076578, rs1006407, rs8779, rs132806, rs5758405, rs881542, rs926333. - =no p-value calculated by HTR. | | | | | | | | | | | | | | | | |

**Table 9 Intermarker linkage disequilibrium measured by D'.**

| Cases above and right of diagonal, controls below and left of diagonal | | | | | | | | | | | |
|---|---|---|---|---|---|---|---|---|---|---|---|
| DK sample | | | | | | | | | | | |
| Gene | SNP | | S1 | S2 | S3 | S4 | S5 | S6 | S7 | S8 | S9 |
| *EP300* | rs20551 | (S1) | | 0.60 | 0.62 | ***0.70*** | ***0.76*** | 0.01 | ***0.67*** | 0.17 | 0.12 |
| *EP300* | rs2294976 | (S2) | 0.52 | | ***0.74*** | ***1.00*** | 0.57 | ***0.84*** | ***1.00*** | 0.01 | 0.10 |
| *EP300* | rs2076578 | (S3) | ***1.00*** | ***1.00*** | | 0.42 | 0.62 | 0.14 | 0.39 | 0.16 | 0.10 |
| *PIPPIN* | rs1006407 | (S4) | 0.43 | 1.00 | 0.57 | | 0.95 | 0.63 | ***0.85*** | 0.14 | 0.23 |
| *NHP2L1* | rs8779 | (S5) | 0.52 | 0.91 | *0.74* | ***0.85*** | | 0.55 | ***0.87*** | 0.34 | 0.20 |
| *NHP2L1* | rs132806 | (S6) | 0.01 | ***0.75*** | 0.12 | ***0.75*** | ***0.76*** | | 0.45 | 0.25 | 0.17 |
| *NHP2L1* | rs5758405 | (S7) | ***0.68*** | ***1.00*** | ***0.87*** | ***0.87*** | ***0.94*** | ***0.89*** | | 0.68 | 0.16 |
| *SERHL* | rs881542 | (S8) | 0.19 | 0.19 | 0.14 | 0.11 | 0.06 | 0.09 | 0.29 | | 0.00 |
| *SERHL* | rs926333 | (S9) | 0.35 | 0.24 | 0.37 | 0.00 | 0.06 | 0.29 | 0.02 | ***0.71*** | |

| UK sample | | | | | | | | | | | |
|---|---|---|---|---|---|---|---|---|---|---|---|
| Gene | SNP | | S1 | S2 | S3 | S4 | S5 | S6 | S7 | S8 | S9 |
| *EP300* | rs20551 | (S1) | | ***1.00*** | ***0.99*** | ***0.67*** | 0.28 | 0.21 | ***0.69*** | 0.02 | 0.02 |
| *EP300* | rs2294976 | (S2) | ***1.00*** | | ***1.00*** | ***1.00*** | 0.27 | ***0.84*** | ***0.73*** | 0.20 | 0.04 |
| *EP300* | rs2076578 | (S3) | ***0.98*** | ***1.00*** | | 0.47 | 0.09 | 0.14 | 0.53 | 0.16 | 0.14 |
| *PIPPIN* | rs1006407 | (S4) | ***0.73*** | 0.63 | 0.53 | | ***0.72*** | ***0.97*** | ***0.95*** | 0.00 | 0.29 |
| *NHP2L1* | rs8779 | (S5) | ***0.69*** | ***0.78*** | 0.51 | ***0.90*** | | ***0.66*** | ***0.66*** | 0.02 | 0.65 |
| *NHP2L1* | rs132806 | (S6) | 0.05 | 0.70 | 0.10 | ***0.94*** | ***1.00*** | | ***0.92*** | 0.10 | 0.40 |
| *NHP2L1* | rs5758405 | (S7) | ***0.75*** | ***0.81*** | 0.60 | ***0.93*** | ***0.90*** | ***1.00*** | | 0.00 | 0.13 |
| *SERHL* | rs881542 | (S8) | 0.02 | 0.05 | 0.14 | 0.03 | 0.04 | 0.13 | 0.07 | | 0.18 |
| *SERHL* | rs926333 | (S9) | 0.01 | 0.01 | 0.03 | 0.25 | 0.30 | 0.07 | 0.57 | 1.00 | |

| Scottish sample | | | | | | | | | | | |
|---|---|---|---|---|---|---|---|---|---|---|---|
| Gene | SNP | | S1 | S3 | S4 | S5 | S8 | S9 | | | |
| *EP300* | rs20551 | (S1) | | ***1.00*** | ***0.67*** | 0.46 | 0.06 | 1.00 | | | |
| *EP300* | rs2076578 | (S3) | ***1.00*** | | 0.31 | 0.07 | 0.09 | 0.04 | | | |
| *PIPPIN* | rs1006407 | (S4) | ***0.70*** | 0.63 | | ***0.93*** | 0.09 | 1.00 | | | |
| *NHP2L1* | rs8779 | (S5) | ***0.69*** | 0.61 | ***0.97*** | | 0.11 | ***1.00*** | | | |
| *SERHL* | rs881542 | (S8) | 0.01 | 0.32 | 0.24 | 0.17 | | 0.03 | | | |
| *SERHL* | rs926333 | (S9) | 0.21 | 0.47 | 0.12 | 0.07 | 1.00 | | | | |

| | | | | | | | | | | | |
|---|---|---|---|---|---|---|---|---|---|---|---|
| Significant (P<0.05) D' values >0.65 in bold and italic | | | | | | | | | | | |

### FEATURES OF THE INVENTION

The present invention is also described with reference to the following numbered paragraphs:
1. A method for determining the predisposition for a mental disease, such as schizophrenia (SCH) and/or bipolar disorder (BPD) in a subject comprising determining in a biological sample isolated from said subject one or more polymorphisms in the DNA sequence of chromosome 22q13 containing the NHP2L1, PACSIN2, SERHL, PIPPIN, BRD1, EP300, FAM19A5 and/or GPR24 genes or in a translational or transcriptional product of said genes.
2. The method according to paragraph 1, wherein the polymorphism is a single nucleotide polymorphism (SNP).
3. The method according to paragraph 2, wherein the predisposition is determined by determining the presence of one or more SNPs in the DNA sequence of one individual gene selected from any of the genes of the group consisting of the NHP2L1, PACSIN2, SERHL, PIPPIN, BRD1, EP300 and GPR24 genes.
4. The method according to paragraph 1-3, wherein at least one of the polymorphisms is located in a non-coding region of the gene DNA sequence.
5. The method according to paragraph 5, wherein the non-coding region is an intron, or a region controlling the gene expression, such as a promoter region.
6. The method according to paragraph 1-4, wherein the at least one of the polymorphisms is determined in a coding region of the gene DNA sequence.
7. The method according to any of the preceding paragraphs wherein two or more polymorphisms are determined.
8. The method according to paragraph 7, wherein the two or more polymorphisms are SNPs.
9. The method according to paragraph 8, wherein the two or more SNPs are determined in the DNA sequence of same gene, said gene is selected from any of the genes as defined in paragraph 1.
10. The method according to paragraph 8, wherein the two or more SNPs are determined in the DNA sequences of two or more different genes selected from any of the genes as defined in paragraph 1.
11. The method according to any of the preceding claims, wherein the SNP is selected from the group consisting of SNPs having refSNP IDs: rs11561, rs5758405 rs8779, rs132806, rs2068943, rs2267487, rs881542, rs926333, rs1060387, rs1006407, rs6002408, rs4468, rs138855, rs2239848, rs138880, rs138881, rs20551, rs2294976, rs2076578, rs1046088, rs133068, rs133069, rs133070, rs133073, rs6002408.
12. The method according to paragraphs 1 to 11, wherein the predisposition to SCH and/or BPD is determined by determining of the presence of the risky allele of an SNP selected from the SNPs as defined in paragraph 11
13. The method according to paragraphs 1 to 11, wherein the predisposition to SCH and/or BPD is determined by determining the presence of a specific haplotype comprising two or more of the SNPs selected from the SNPs as defined in paragraph 11.
14. The method according to paragraph 13, wherein the specific haplotype comprises at least one of the SNPs as determined in claim 11 and a polymorphism of the DNA. sequence in the region comprising 100 to 10000 base pairs upstream or down stream from said SNP.
15. The method according to paragraph 14, wherein the polymorphism is SNP.
16. The method according to paragraph 15, wherein the SNP is selected from the group consisting of SNPs having refSNP ID No: rs132234, rs3752466, rs6010260, rs137931, rs137932, rs3810971, rs2272843, rs1063900, rs715519, rs916005.
17. The method according to paragraph 16, wherein the SNP is a part of a haplotype comprising at least one of the SNPs selected from the group consisting of SNPs having refSNP ID NOs: rs4468, rs138855, rs2239848, rs138880, rs138881.
18. The method according to paragraph 15, the SNP is selected from the group consisting of SNPs having refSNP ID No: rs909660, rs710193, rs1573745.
19. The method according to paragraph 18, wherein the SNP is a part of a haplotype comprising at least one of the SNPs selected from the group consisting of SNPs having refSNP ID NOs: rs133068, rs133069, rs133070, rs133073.
20. The method according to paragraph 13, wherein the specific haplotype comprises a polymorphism in a chromosome region adjacent to the region containing a gene selected from the group consisting the NHP2L1, PACSIN2, SERHL, PIPPIN, BRD1, EP300 and GPR24 genes.
21. The method according to any of the preceding paragraphs 14-20, wherein the polymorphism is in linkage disequilibrium with at least one of the SNPs identified in any of the paragraphs 11, 16, 17, 18 or 19.
22. The method of paragraph 21, wherein the polymorphism is SNP.
23. The method according to any of the preceding paragraphs, wherein a SNP(s) is(are) determined in
   i) a nucleotide sequence selected from SEQ ID NOs: 1-7 and 94,
   ii) a sequence having at least 90% sequence identity with SEQ ID NOs: 1-7 and 94, or a fragment thereof, and
   iii) a sequence being complementary to one of these sequences or a fragment thereof.
24. The method according to any of the preceding paragraphs, wherein the SNP(s) is (are) determined in the transcriptional or translational products of the genes as defined in paragraph 1.
25. The method according to paragraph 24, wherein the transcriptional products of the genes being selected from
   (i) nucleic acid sequences identified as SEQ ID NO: 8-14, or fragments thereof,
   (ii) nucleic acid sequences having at least 90% identity with SEQ ID NO: 8-14 or fragments thereof,
   (iii) nucleic acid sequences being complementary to any of the sequences of (i) or (ii),
   said nucleic acid sequences comprising the polymorphism(s) of the corresponding genomic sequences identified as SEQ ID NO: 1-7 and 94.
26. The method according to paragraph 24, wherein the translational products of the genes being selected from
   ii) polypeptide sequences identified as SEQ ID NOs: 87-93 , or fragments thereof,
   iii) polypeptide sequences having at least 90% identity with the polypeptide sequences of (i), or fragments thereof,
   said polypeptide sequences comprising a polymorphism(s) corresponding to the polymorphism(s) of the corresponding nucleic acid sequence(s), said nucleic acid sequence(s) being identified as SEQ ID NO: 1-7 and 94 or SEQ ID NO: 8-14.
27. The method according to any of the preceding paragraphs, wherein the presence or absence of a polymorphism is detected in a target nucleic acid sequence isolated from a biological sample.
28. The method according to paragraph 27, said method comprising amplification of the target nucleotide sequence.
29. The method according to paragraph 27 or 28, wherein the nucleotide sequence is a genomic DNA sequence, a mRNA sequence, or a cDNA sequence.
30. The method according to paragraph 27, wherein amplification comprises use of a primer pair selected from the oligonucleotide sequences identified as SEQ ID Nos: 15-86.
31. The method according to any of the preceding paragraphs, wherein the presence or absence of the polymorphism is determined in a variant protein, said variant protein being a translational product of a gene selected from the genes as defined in claim 1, wherein said variant protein comprising a polymorphism corresponding to the polymorphism of the nucleic acid sequence encoding said variant protein.
32. The method according to paragraph 31, wherein the variant protein is NHP2L1 protein (SEQ ID NO: 87) having the sequence wherein amino acid residue Thr at position 43 is substituted for amino acid residue Ala.
33. The method according to paragraph 31, wherein the variant protein is SERHL protein (SEQ ID NO: 89) having the sequence wherein amino acid residue Ala at position 2 is substituted for amino acid residue Val.
34. The method according to paragraph 31, wherein the variant protein is SERHL protein (SEQ ID NO: 89) having the sequence wherein amino acid residue Ser at position 46 is substituted for amino acid residue Ala.
35. The method according to paragraph 31, wherein the variant protein is EP300 protein (SEQ ID NO: 92) having the sequence wherein amino acid residue Ile at position 997 is substituted for amino acid residue Val.
36. The method according to paragraph 31, the variant protein is EP300 protein (SEQ ID NO: 92) having the sequence wherein amino acid residue Gln at position 2223 is substituted for amino acid residue Pro.
37. The method according to paragraph 1, wherein the predisposition to a mental disease is determined by determining the presence of a haplotype comprising the SNPs having refSNP No: rs133068, rs133069, rs133070, rs133073 and one or more SNPs as defined in paragraph 18
38. The method according to paragraph 1, wherein the predisposition to a mental disease is determined by determining the presence of a haplotype comprising the SNPs having refSNP No: rs4468, rs138855, rs2239848, rs138880, rs138881and one or more SNPs as defined in paragraph 16.
39. The method according to any of the preceding paragraphs, wherein the predisposition to a mental disease is determined by determining in a biological sample isolated from said subject the risky allele(s) of an SNP(s) as defined in paragraph 16.
40. A method for determining the absence of predisposition to a mental disease, such as SCH and/or BPD, in a subject comprising determining in a biological sample isolated from said subject a protective allele of an SNP(s) selected form the SNP(s) as defined in paragraph 11.
41. A method for determining a protection against a mental disease, such as SCH and/or BPD, in a subject comprising determining in a biological sample isolated from said subject a protective allele of an SNP(s) selected form the SNP(s) as defined in paragraph 11.
42. An isolated oligonucleotide comprising at least 10 contiguous nucleotides being 100% identical to a subsequence of a gene selected from of the NHP2L1, PACSIN2, SERHL, PIPPIN, BRD1, EP300 and GPR24 genes comprising or adjacent to a polymorphism or mutation being correlated to an mental disease.
43. The isolated oligonucleotide according to paragraph 42, wherein the polymorphism is located in the centre of the nucleic acid sequence.
44. The isolated oligonucleotide according to paragraph 42, wherein the polymorphism is located in the 5' end of the nucleic acid sequence.
45. The isolated oligonucleotide according to paragraph 42, wherein the mutation/polymorphism is located in the 3' end of the nucleic acid sequence.
46. The isolated oligonucleotide according to paragraph 42, wherein the sequence is adjacent to the mutation/polymorphism, either in the 3' or 5' direction.
47. The isolated oligonucleotide according to any of the paragraphs 42-46, said oligonucleotide being selected from the oligonucleotides identified as SEQ ID NO: 15-86.
48. A diagnostic kit comprising at least two oligonucleotides as defined by any of the preceding paragraphs 42-47.
49. The diagnostic kit according to paragraph 48, wherein the at least two oligonucleotides are the amplification primers or probes for determining a polymorphism associated with a predisposition to a mental disease, such as SCH and/or BPD, as defined in any of the preceding paragraphs.
50. The diagnostic kit according to paragraph 49, wherein the probe is linked to a detectable label.
51. The diagnostic kit of paragraph 49, wherein the primers or probes are selected from the nucleic acid sequences identified as SEQ ID NOs: 15-86.
52. A variant protein as defined in any of the paragraphs 32-36, said protein being indicative of a predisposition to a mental disease, such as SCH and/or BPD.
53. An antibody capable of selectively binding to a variant protein of paragraph 52 to an epitope comprising a polymorphism of the variant protein as defined in any of the paragraphs 32-36.
54. A diagnostic kit comprising an antibody selected from the antibody(s) according to paragraph 53.
55. The diagnostic kit according to paragraph 54, said kit further comprising the diagnostic kit according to any of the paragraphs 48-51.
56. A gene therapy vector comprising
   (i) a DNA sequence selected from the sequences identified as SEQ ID NO 1-7 and 94, or a fragment thereof, or
   (ii) a DNA sequence selected from the sequences identified as SEQ ID NOs: 8-14, or a fragment of said DNA sequence.
57. The gene therapy vector according to paragraph 56, wherein the DNA sequence or a fragment thereof comprises the protective allele of an SNP selected from the SNPs as defined in any of the paragraphs 11, 16, 17, 18 or 19
58. A method of treatment of a subject having the predisposition to a mental disease such as SCH and/or BPD, said method comprising administering to said subject a therapeutically effective amount of a gene therapy vector as defined in paragraphs 56 or 57.
59. A vector comprising a nucleic acid sequence selected from the nucleic acid sequences identified as SEQ ID NOs: 1-14, or a fragment thereof, said sequence, or said fragment comprising a polymorphism associated with a predisposition to an mental disease according to any of the paragraphs 1 to 39, said sequence being operably linked to a promoter sequence capable of directing the expression of a variant protein encoded by said sequence.
60. A compound capable of
   i) inhibiting expression of a gene selected from the genes according to paragraph 1 said compound being selected from an isolated antisense nucleotide sequence or an nucleotide sequence complementary to the regulatory region of said gene, said nucleotide sequence being capable of forming triple helix structures that prevent transcription of said gene, and/or
   ii) inhibiting activity of a transcriptional product of a gene selected from the genes according to paragraph 1, said transcriptional product being as defined in paragraph 25, said compound is selected from an isolated antisense sequence or a ribozyme molecule, and/or
   iii) inhibiting activity of a translational product of a gene selected from the genes according to paragraph 1, said transcriptional product being as defined in paragraph 26, said compound is selected from an antibody molecule against said transcriptional product, or a molecule capable of interfering with biological activity of said transcriptional product.
61. Use of a compound according to paragraph 60 for the manufacture of a medicament for treatment of a mental disease.
62. The use according to paragraph 61, wherein the disease is SCH and/or BPD.
63. A pharmaceutical composition for the treatment of mental disease, such as SCH and/or BPD, comprising a compound according to paragraph 59.
64. A method of treatment of a mental disease, such as SCH and/or BPD, comprising administering a compound according to 60 or a pharmaceutical composition according to paragraph 63.
65. A method of screening for a candidate compound for therapeutic treatment of a mental disease, such as SCH and/or BPD, said meth comprising an in vitro or in vivo model system comprising a gene according to paragraph 1 or a product of said gene, said product being a transcriptional product of the gene as defined in paragraph 25 or a translational product of the gene as defined in paragraph 26.
66. The method according to paragraph 65, said method further comprising a cell expressing a gene according to paragraph 1, a transcriptional product of said gene as defined in paragraph 25 or a translational product of said gene as defined in paragraph 26.
67. A method for prognosis of the likelihood of development of a mental disease comprising determining a polymorphism of a gene selected from the genes according to paragraph 1, said polymorphism being as defined in any of the paragraph 2-39.
68. The method according to paragraph 67, wherein the mental disease is SCH and/or BPD.
69. A method of predicting the likelihood of a subject to respond to a therapeutic treatment of a mental disease, such as SCH and/or BPD, said method comprising determining the genotype of said subject in the chromosome areas comprising the NHP2L1, PACSIN2, SERHL, PIPPIN, BRD1, EP300, FAM19A5 and/or GPR24 gene.
70. The method according to paragraph 69, wherein the determining comprises assessing a polymorphism in the DNA sequence of the NHP2L1, PACSIN2, SERHL, PIPPIN, BRD1, EP300, FAM19A5 and/or GPR24 gene, or the corresponding polymorphism in a transcriptional or translational product of said gene, and/or assessing a polymorphism in the DNA sequence of chromosome 22q13, said polymorphism being in linkage disequilibrium with a SNP of the NHP2L1, PACSIN2, SERHL, PIPPIN, BRD1, EP300, FAM19A5 and/or GPR24 gene, said SNP being associated with a predisposition of a subject to a mental disease, such as SCH and/or BPD..
71. The method according to paragraph 70, wherein the polymorphism comprises or corresponds to an SNP selected from the SNPs having refSNP IDs: rs11561, rs5758405 rs8779, rs132806, rs2068943, rs2267487, rs881542, rs926333, rs1060387, rs1006407, rs6002408, rs4468, rs138855, rs2239848, rs138880, rs138881, rs20551, rs2294976, rs2076578, rs1046088, rs133068, rs133069, rs133070, rs133073, rs6002408.
72. A method for assessing a therapeutic treatment for a mental disease, such as SCH and/or BPD comprising using genotype data.

## Claims

1. A method for determining the predisposition for a mental disease, such as schizophrenia (SCH) and/or bipolar disorder (BPD) in a subject comprising determining in a biological sample isolated from said subject one or more polymorphisms in the DNA sequence of chromosome 22q13 consisting of coding and/or non-coding regions of the BRD1 gene or in a translational or transcriptional product of said gene.

2. The method according to claim 1, wherein the predisposition is determined by determining the presence of one or more SNPs in the DNA sequence of the BRD1 gene.

3. The method according to any of the preceding claims, wherein the SNP is selected from the group consisting of SNPs having refSNP IDs: rs4468, rs138855, rs2239848, rs138880, rs138881.

4. The method according to claims 1 to 3, wherein the predisposition to SCH and/or BPD is determined by determining of the presence of the risky allele of an SNP selected from the SNPs as defined in claim 3.

5. The method according to claims 1 to 3, wherein the predisposition to SCH and/or BPD is determined by determining the presence of a specific haplotype comprising two or more of the SNPs selected from the SNPs as defined in claim 3, or at least one of the SNPs as determined in claim 3 and a polymorphism of the DNA sequence in the region comprising 50 to 10000 base pairs upstream or down stream from said SNP.

6. The method according to any of the preceding claims 3 to 5, wherein the polymorphism is in linkage disequilibrium with at least one of the SNPs identified in any one of claims 3 to 5.

7. The method according to any of the preceding claims, wherein a SNP(s) is(are) determined in
i) a nucleotide sequence selected from SEQ ID NO: 5,
ii) a sequence having at least 90% sequence identity with SEQ ID NO: 5, or a fragment consisting of at least 20 consecutive nucleotides thereof, and
iii) a sequence being complementary to this sequence or a fragment consisting of at least 20 consecutive nucleotides thereof.

8. The method according to any of the preceding claims, wherein the SNP(s) is (are) determined in the transcriptional or translational products of the BRD1 gene as defined in claim 1.

9. The method according to claim 8, wherein the transcriptional product of the gene being selected from
(i) nucleic acid sequence identified as SEQ ID NO: 5, or fragments thereof,
(ii) nucleic acid sequences having at least 90% identity with SEQ ID NO: 5 or fragments thereof,
(iii) nucleic acid sequences being complementary to any of the sequences of (i) or (ii),
said nucleic acid sequences comprising the polymorphism(s) of the corresponding genomic sequences identified as SEQ ID NO: 5.

10. The method according to claim 8, wherein the translational product of the gene being selected from
i) polypeptide sequence identified as SEQ ID NO: 91 , or fragments thereof,
ii) polypeptide sequences having at least 90% identity with the polypeptide sequence of (i), or fragments thereof,
said polypeptide sequences comprising a polymorphism(s) corresponding to the polymorphism(s) of the corresponding nucleic acid sequence(s), said nucleic acid sequence(s) being identified as SEQ ID NO: 5 and 91 or SEQ ID NO: 12.

11. The method according to any preceding claim, wherein the nucleotide sequence is a genomic DNA sequence, a mRNA sequence, or a cDNA sequence.

12. The method according to any preceding claim, wherein amplification comprises use of a primer pair selected from the oligonucleotide sequences identified as SEQ ID NOs: 51-65.

13. The method according to claim 1, wherein the predisposition to a mental disease is determined by determining the presence of a haplotype comprising the SNPs having refSNP No: rs4468, rs138855, rs2239848, rs138880, rs138881 and/or one or more SNPs as defined in claims 3 to 7.

14. The method according to any of the preceding claims, wherein the predisposition to a mental disease is determined by determining in a biological sample isolated from said subject the risky allele(s) of an SNP(s) as defined in claim 3.

15. A method for determining the absence of predisposition to a mental disease or a protection against a mental disease, such as SCH and/or BPD, in a subject comprising determining in a biological sample isolated from said subject a protective allele of an SNP(s) selected form the SNP(s) as defined in claim 3.

16. An isolated oligonucleotide comprising at least 10 contiguous nucleotides being 100% identical to a subsequence of the BRD1 gene comprising or adjacent to a polymorphism or mutation being correlated to an mental disease, said oligonucleotide being selected from the oligonucleotides identified as SEQ ID NO: 51-65.

17. A diagnostic kit comprising at least two oligonucleotides as defined by the preceding claim 16.

18. The diagnostic kit according to claim 17, wherein the at least two oligonucleotides are the amplification primers or probes for determining a polymorphism associated with a predisposition to a mental disease, such as SCH and/or BPD, as defined in any of the preceding claims.

19. The diagnostic kit of claim 18, wherein the primers or probes are selected from the nucleic acid sequences identified as SEQ ID NOs: 51-65.

20. A gene therapy vector comprising
(i) a DNA sequence selected from the sequences identified as SEQ ID NO 5, or a fragment consisting of at least 20 consecutive nucleotides thereof, or
(ii) a DNA sequence selected from the sequences identified as SEQ ID NO: 12, or a fragment consisting of at least 20 consecutive nucleotides of said DNA sequence.

21. The gene therapy vector according to claim 20, wherein the DNA sequence or a fragment consisting of at least 20 consecutive nucleotides thereof comprises the protective allele of an SNP selected from the SNPs as defined in any of claims 3 to 7.

22. A vector comprising a nucleic acid sequence selected from the nucleic acid sequences identified as SEQ ID NOs: 5 or 12, or a fragment consisting of at least 20 consecutive nucleotides thereof, said sequence, or said fragment comprising a polymorphism associated with a predisposition to an mental disease according to any of the claims 1 to 14, said sequence being operably linked to a promoter sequence capable of directing the expression of a variant protein encoded by said sequence.

23. A compound capable of
i) modulating expression of the BRD1 gene, said compound being selected from an isolated antisense nucleotide sequence or an nucleotide sequence complementary to the regulatory regions of said gene, said nucleotide sequence being capable of forming triple helix structures that modulate transcription of said gene, and/or
ii) modulating activity of a transcriptional product of the BRD1 gene, said transcriptional product being as defined in claim 9, said compound is selected from an isolated sense or antisense sequence or a ribozyme molecule, and/or
iii) modulating activity of a translational product of the BRD1 gene, said translational product being as defined in claim 10, said compound is an antibody molecule against said translational product or binding partner for said translational product.

24. Use of a compound according to claim 23 for the manufacture of a medicament for treatment of a mental disease.

25. The use according to claim 24, wherein the disease is SCH and/or BPD.

26. A pharmaceutical composition for the treatment of mental disease, such as SCH and/or BPD, comprising a compound according to claim 23.

27. A method of screening for a candidate compound for therapeutic treatment of a mental disease, such as SCH and/or BPD, said method comprising an in vitro or in vivo model system comprising a gene according to claim 1 or a product of said gene, said product being a transcriptional product of the gene as defined in claim 9 or a translational product of the gene as defined in claim 10.

28. The method according to claim 27, said method further comprising a cell expressing a BRD1 gene, a transcriptional product of said gene as defined in claim 9 or a translational product of said gene as defined in claim 10.

29. A method for prognosis of the likelihood of development of a mental disease , such as SCH and/or BPD, said method comprising determining a polymorphism of a gene selected from the BRD1 gene, said polymorphism being as defined in any of the claims 1-14.

30. A method of predicting the likelihood of a subject to respond to a therapeutic treatment of a mental disease, such as SCH and/or BPD, said method comprising determining the genotype of said subject in the chromosome area comprising the BRD1 gene.

31. The method according to claim 30, wherein the determining of genotypes comprises assessing a polymorphism in the DNA sequence of the BRD1 gene, or the corresponding polymorphism in a transcriptional or translational product of said gene, and/or assessing a polymorphism in the DNA sequence of chromosome 22q13, said polymorphism being in linkage disequilibrium with a SNP of the BRD1 gene.

32. The method according to claim 31, wherein the polymorphism comprises or corresponds to an SNP selected from the SNPs having refSNP IDs: rs4468, rs138855, rs2239848, rs138880, rs138881.

33. A method for assessing a therapeutic treatment for a mental disease, such as SCH and/or BPD comprising using BRD1 genotype data.
